(19) Europäisches Patentamt / European Patent Office / Office européen des brevets

(11) **EP 4 524 134 A1**

(12) **EUROPEAN PATENT APPLICATION**
published in accordance with Art. 153(4) EPC

(43) Date of publication:
**19.03.2025 Bulletin 2025/12**

(21) Application number: **23802925.0**

(22) Date of filing: **09.05.2023**

(51) International Patent Classification (IPC):
***C07D 401/04*** (2006.01) ***C07D 401/14*** (2006.01)
***A61P 35/00*** (2006.01) ***A61K 31/44*** (2006.01)

(52) Cooperative Patent Classification (CPC):
**A61K 31/44; A61K 31/4427; A61K 31/4439; A61K 31/444; A61K 31/4545; A61K 31/4709; A61K 31/4725; A61K 31/497; A61K 31/501; A61K 31/506; A61K 45/06; A61P 35/00; C07D 213/75; C07D 401/04; C07D 401/14;** (Cont.)

(86) International application number:
**PCT/CN2023/093095**

(87) International publication number:
**WO 2023/217156 (16.11.2023 Gazette 2023/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.05.2022 CN 202210501749**

(71) Applicant: **TYK Medicines Inc.**
**Huzhou, Zhejiang 313100 (CN)**

(72) Inventors:
- **LIANG, Apeng**
  **Huzhou, Zhejiang 313100 (CN)**
- **LIU, Guangbin**
  **Huzhou, Zhejiang 313100 (CN)**
- **CHEN, Shaoqing**
  **Huzhou, Zhejiang 313100 (CN)**
- **LI, Jun**
  **Huzhou, Zhejiang 313100 (CN)**
- **WU, Yusheng**
  **Huzhou, Zhejiang 313100 (CN)**
- **YIN, Zhou**
  **Huzhou, Zhejiang 313100 (CN)**
- **DONG, Shengli**
  **Huzhou, Zhejiang 313100 (CN)**
- **NIU, Chengshan**
  **Huzhou, Zhejiang 313100 (CN)**
- **LI, Meihua**
  **Huzhou, Zhejiang 313100 (CN)**

(74) Representative: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(54) **POLYCYCLIC COMPOUND AND USE THEREOF**

(57) A polycyclic compound, a preparation method therefor, and the use thereof in the prevention and/or treatment of proliferative diseases. The compound has a structure as represented by formula (I).

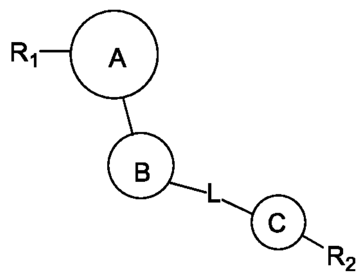

Formula I

EP 4 524 134 A1

(52) Cooperative Patent Classification (CPC): (Cont.)
**C07D 403/04; C07D 409/14; C07D 413/14;**
**C07D 417/14; C07D 487/10**

## Description

### THCHNICAL FIFLD

The present invention relates to the field of pharmaceutical technology, and specifically to polycyclic compounds used as modulators of the Hippo pathway, and preparation method and use thereof, primarily for use in the treatment or prevention of proliferative disorders (e.g., cancers), and in particular for the modulation and treatment of disorders associated with aberrant activity of YAP/TEAD.

### BACKGROUND ART

The Hippo pathway essentially consists of a core kinase cascade that contains the Ste-20 family of protein kinases MST1-2, the scaffolding protein Salvador, and the large tumor suppressor kinase LATS1-2, as well as the repressor transcriptional co-activators YAP (Yes1-associated protein) and TAZ (transcriptional co-activator with a PDZ-binding motif). YAP and TAZ are major effectors of the Hippo signaling pathway, and together with TEAD (Transcriptionally Enhanced Architecture Domain) in the nucleus, they act as transcription factors that increase the expression of target genes such as CTGF (Connective Tissue Growth Factor), CYR61, etc. The Hippo pathway is an important regulator of cell growth, proliferation and migration. TEAD transcription factors lie at the core of the Hippo pathway and are critical for regulating organ growth and wound repair. Dysregulation of TEAD and its regulatory cofactor Yes-associated protein (YAP) have been implicated in a number of human cancers and hyperproliferative pathological processes, and dysregulation of this pathway is frequently detected in human cancers. Like TEAD proteins, YAP and TAZ activation have been identified in many human tumors and are critical for tumor initiation, progression, and metastasis, e.g., elevated YAP expression is present in patients with breast, ovarian, colon, liver, and pancreatic cancers and is associated with reduced survival. Consistent with this, activation or overexpression of YAP or TAZ enhances TEAD-dependent gene expression (e.g., CCN1, CTGF, ITGB2, and Birc5/survival proteins) and promotes cell proliferation and migration in many cell types. In contrast, blocking the signaling for YAP/TAZ-TEAD complex formation or intervening to prevent the expression of many mitogenic TEAD target genes significantly reduces cell proliferation and oncogenic transformation activity. In addition, the Hippo pathway cross-talks with other signaling pathways such as Wnt, Notch, Hedgehog, and MAPK, thereby affecting a variety of biological functions, and its dysfunction may be involved in many human diseases in addition to cancer. Thus, the YAP-TEAD complex is a promising therapeutic target.

In addition, a number of compounds structurally similar to the present invention are disclosed in patent WO2009081259A1 with the formula

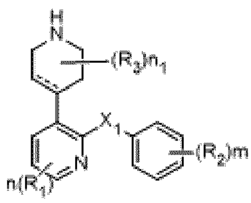,

from them, the present inventors selected some compounds with representative structures and conducted activity tests, which showed that none of these compounds had Y AP/TEAD-related activity, and the detailed results are shown in Table 2.

### SUMMARY OF THE INVENTION

The purpose of the present invention is to provide a compound shown in Formula I and its preparation method and its use for the prevention or treatment of proliferative diseases.

In the first aspect of the present invention, it provides a compound shown in Formula I or a pharmaceutically acceptable salt, solvate or prodrug thereof:

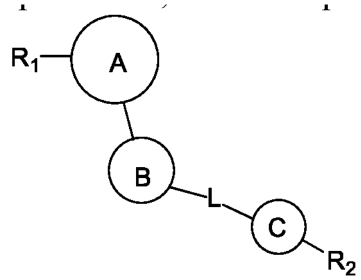

Formula I

wherein,

A is selected from the group consisting of

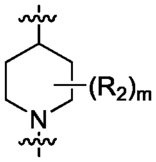 , 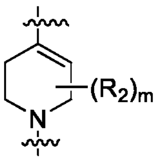 , 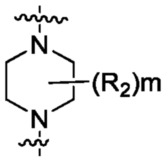 ,

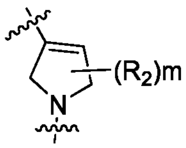 , 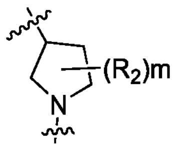 , 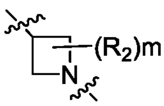 , and 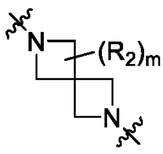 ;

B is selected from the group consisting of

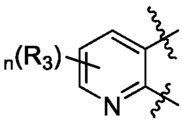 , 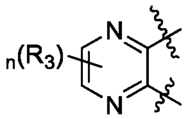 ,

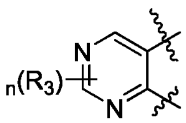 , 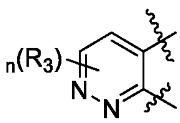 , 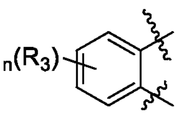 , 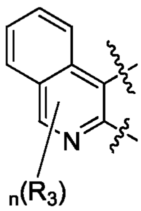 , 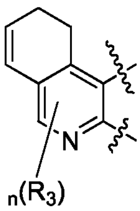 , and 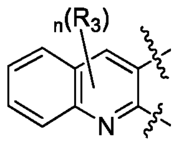 ;

C is selected from the group consisting of

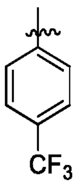 , 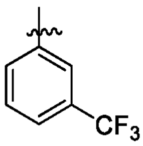 , 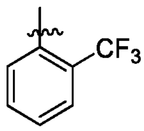 , 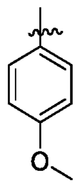 ,

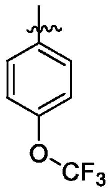, 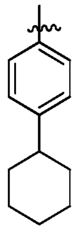, 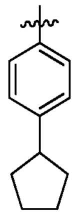, 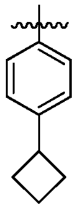, 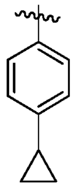, 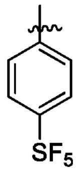, 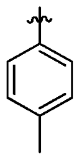, 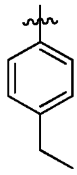, 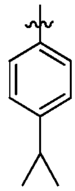, 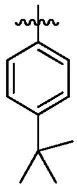, 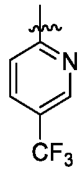,

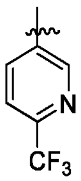, 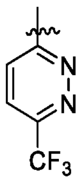, 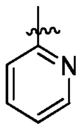, 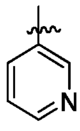, 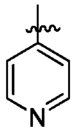, 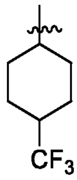, 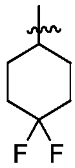, 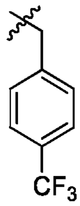, and 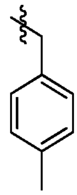;

L is selected from the group consisting of $NR_4$, O, S, Se, sulfoxide, sulfone, and $CR_2R_3$;

$R_1$ is selected from the group consisting of

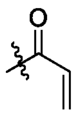, 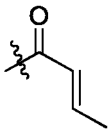, 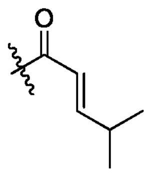, 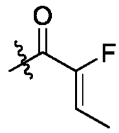,

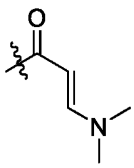, 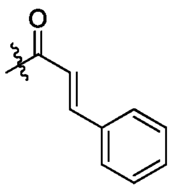, 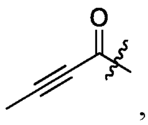, 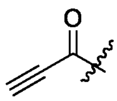, 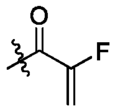, 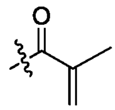, 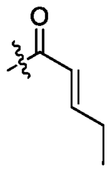, 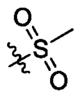,

and

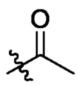,

which is connected to N on A;

$R_2$ is each independently selected from the group consisting of H, halogen, CN, $NH_2$, hydroxyl, ester group, ureido, urethane, amido, $C_{1-6}$ alkyl, $C_{1-6}$ alkoxy, $C_{3-6}$ cycloalkyl, $C_{3-6}$ cycloalkoxy, aryl, heteroaryl, wherein the hydrogen in $NH_2$, ester group, ureido, urethane, amido, $C_{1-6}$ alkyl, $C_{1-6}$ alkoxy, $C_{3-6}$ cycloalkyl, $C_{3-6}$ cycloalkoxy, aryl, heteroaryl can be optionally substituted, and the substituents are H, halogen, CN, $NH_2$, hydroxyl, ester group, ureido, urethane, amido, $C_{1-6}$ alkyl, $C_{1-6}$ alkoxy, $C_{3-6}$ cycloalkyl, $C_{3-6}$ cycloalkoxy, aryl, heteroaryl, or $R_2$ forms a 3-7-membered ring with the attached structure, or a 3-7-membered ring is formed between the different $R_2$;

$R_3$ is each independently selected from the group consisting of H, halogen, CN, $NH_2$, hydroxyl, ester group, ureido, urethane, amido, $C_{1-6}$ alkyl, $C_{1-6}$ alkoxy, $C_{3-6}$ cycloalkyl, $C_{3-6}$ cycloalkoxy, aryl, heteroaryl, wherein the hydrogen in $NH_2$, ester group, ureido, urethane, amido, $C_{1-6}$ alkyl, $C_{1-6}$ alkoxy, $C_{3-6}$ cycloalkyl, $C_{3-6}$ cycloalkoxy, aryl, heteroaryl can be optionally substituted, and the substituents are H, halogen, CN, $NH_2$, hydroxyl, ester group, ureido, urethane, amido, $C_{1-6}$ alkyl, $C_{1-6}$ alkoxy, $C_{3-6}$ cycloalkyl, $C_{3-6}$ cycloalkoxy, aryl, heteroaryl, or a 5-7-membered ring is formed between the different $R_3$ and the attached B;

$R_4$ is selected from the group consisting of H, ester group, $-C(O)R_5$, ureido, $C_{1-6}$ alkyl, $C_{1-6}$ alkoxy, $C_{3-6}$ cycloalkyl, aryl, heteroaryl, wherein the hydrogen in the ester group, ureido, $C_{1-6}$ alkyl, $C_{1-6}$ alkoxy, $C_{3-6}$ cycloalkyl, aryl, heteroaryl can be optionally substituted, and the substituents are H, halogen, CN, $NH_2$, hydroxyl, ester group, ureido, urethane, amido, $C_{1-6}$ alkyl, $C_{1-6}$ alkoxy, $C_{3-6}$ cycloalkyl, $C_{3-6}$ cycloalkoxy, aryl, and heteroaryl;

$R_5$ is selected from the group consisting of H, $C_{1-6}$ alkyl, $C_{3-6}$ cycloalkyl, aryl, and heteroaryl;
m and n are each independently selected from the group consisting of 0, 1, 2, 3, 4, 5, and 6.

In another preferred embodiment, the heteroaryl is a 5-10-membered heteroaryl containing 1, 2 or 3 heteroatoms selected from N, O or S, preferably a 5-6-membered heteroaryl containing 1, 2 or 3 heteroatoms selected from N, O or S, more preferably from the group consisting of

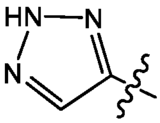, 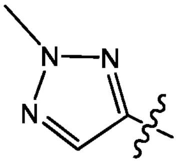, 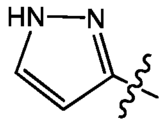, 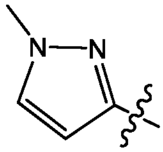,

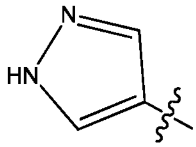, 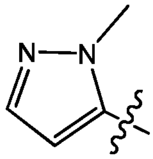, 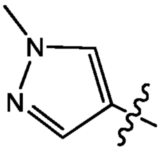, 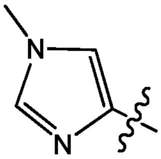, 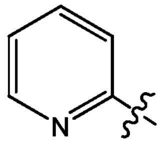, 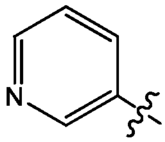,

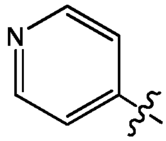, 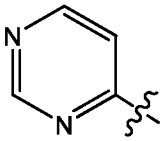, 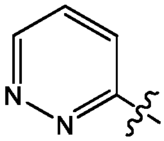, 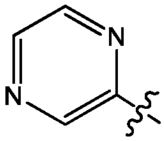, 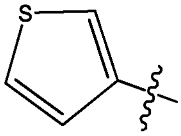, 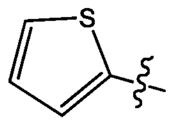,

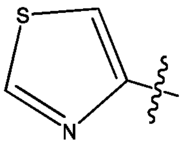, 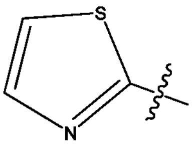, 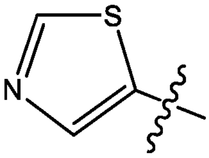, 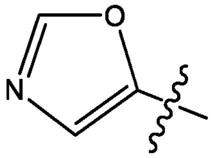, 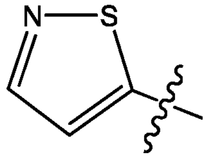,

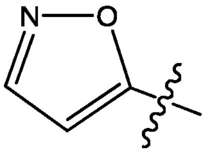, 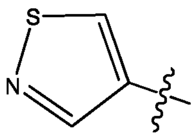, 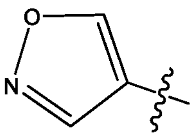, 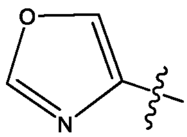, 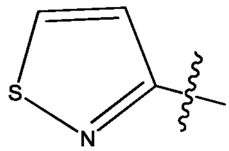,

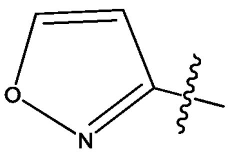, and 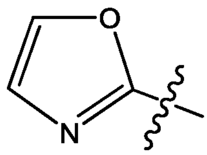.

In another preferred embodiment, the aryl is a C6-C10 aryl, preferably phenyl or naphthyl.
In another preferred embodiment, the amido is -(C=O)-$NH_2$.
In another preferred embodiment, the sulfone is -S(=O)$_2$-.
In another preferred embodiment, the sulfoxide is -S(=O)-.
In another preferred embodiment, the ester group is -(C=O)-O-R or -O-(C=O)-R, R is H or $C_{1-6}$ alkyl.
In another preferred embodiment, the ureido is -NH-(C=O)-$NH_2$.
In another preferred embodiment, the urethane is -NH-(C=O)-O-$CH_3$.
In another preferred embodiment, $R_2$ is each independently selected from the group consisting of H, halogen, CN, $NH_2$, hydroxyl, $C_{1-6}$ alkyl, $C_{1-6}$ alkoxy, $C_{3-6}$ cycloalkyl, $C_{3-6}$ cycloalkoxy, wherein the hydrogen in $NH_2$, $C_{1-6}$ alkyl, $C_{1-6}$ alkoxy, $C_{3-6}$ cycloalkyl, $C_{3-6}$ cycloalkoxy can be optionally substituted, and the substituents are H, halogen, CN, $NH_2$, hydroxyl, $C_{1-6}$ alkyl, $C_{1-6}$ alkoxy, $C_{3-6}$ cycloalkyl, $C_{3-6}$ cycloalkoxy.

In another preferred embodiment, $R_2$ is each independently selected from the group consisting of H, halogen, and $C_{1-6}$ alkyl, wherein the hydrogen in $C_{1-6}$ alkyl can be optionally substituted, and the substituent is halogen.

In another preferred embodiment, $R_3$ is each independently selected from the group consisting of H, halogen, CN, $NH_2$, hydroxyl, amido, $C_{1-6}$ alkyl, $C_{1-6}$ alkoxy, $C_{3-6}$ cycloalkyl, $C_{3-6}$ cycloalkoxy, aryl, and heteroaryl, wherein the hydrogen in $NH_2$, amido, $C_{1-6}$ alkyl, $C_{1-6}$ alkoxy, $C_{3-6}$ cycloalkyl, $C_{3-6}$ cycloalkoxy, aryl, and heteroaryl can be optionally substituted, and the substituents are H, halogen, CN, $NH_2$, hydroxyl, amido, $C_{1-6}$ alkyl, $C_{1-6}$ alkoxy, $C_{3-6}$ cycloalkyl, $C_{3-6}$ cycloalkoxy, aryl, and heteroaryl.

In another preferred embodiment, $R_3$ is each independently selected from the group consisting of H, halogen, CN, $NH_2$, hydroxyl, amido, $C_{1-6}$ alkyl, $C_{1-6}$ alkoxy, aryl, and heteroaryl, wherein the hydrogen in $NH_2$, amido, $C_{1-6}$ alkyl, $C_{1-6}$ alkoxy, aryl, and heteroaryl can be optionally substituted, and the substituents are H, halogen, CN, $NH_2$, hydroxyl, $C_{1-6}$ alkyl, and $C_{1-6}$ alkoxy.

In another preferred embodiment, $R_4$ is selected from the group consisting of H, $C_{1-6}$ alkyl, wherein the hydrogen in $C_{1-6}$ alkyl can be optionally substituted, and the substituents are H, halogen, CN, $NH_2$, hydroxyl, $C_{1-6}$ alkoxy, $C_{3-6}$ cycloalkyl, $C_{3-6}$ cycloalkoxy. In another preferred embodiment, m is selected from the group consisting of 0, 1, 2, and 3.

In another preferred embodiment, n is selected from the group consisting of 0, 1, 2, and 3.

In another preferred embodiment, m is 0.

In another preferred embodiment, n is 0 or 1.

In another preferred embodiment, A is selected from the group consisting of

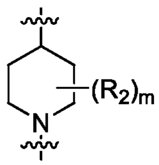 , 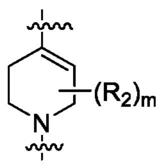 , 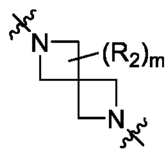 ;

$R_2$ and m are as defined above.

In another preferred embodiment, m is 0.

In another preferred embodiment, B is selected from the group consisting of

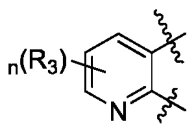 , 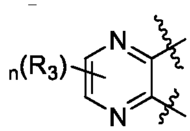 ;

$R_3$ and n are as defined above.

In another preferred embodiment, n is 0 or 1.

In another preferred embodiment, the compound is selected from the group consisting of

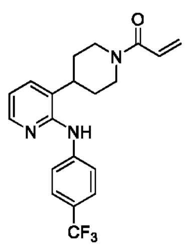
T1

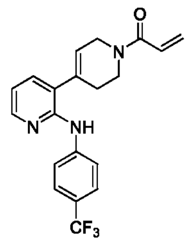
T2

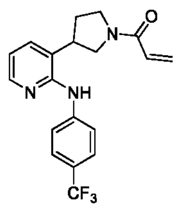
T3

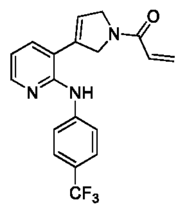
T4

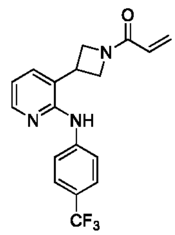
T5

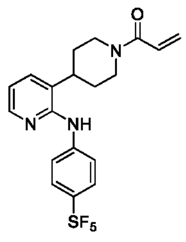
T6

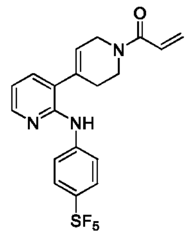
T7

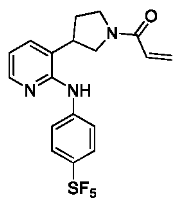
T8

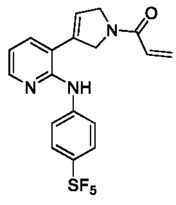
T9

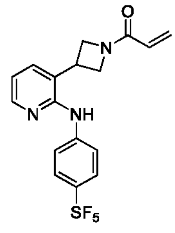
T10

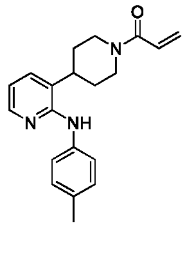
T11

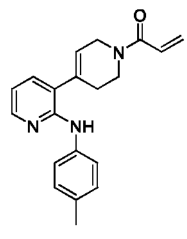
T12

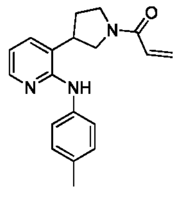
T13

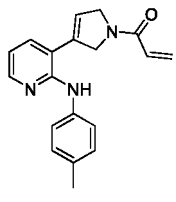
T14

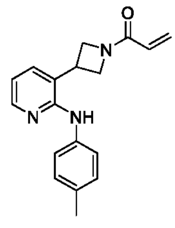
T15

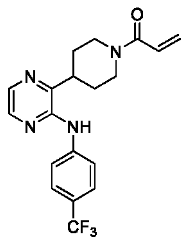
T16

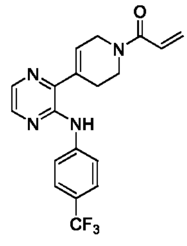
T17

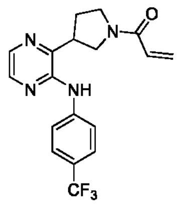
T18

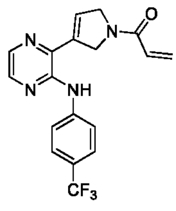
T19

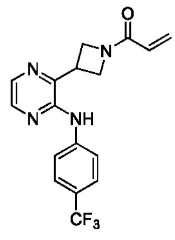
T20

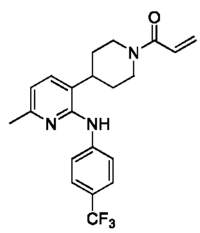
T21

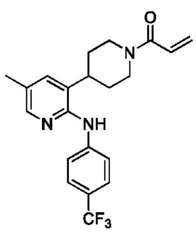
T22

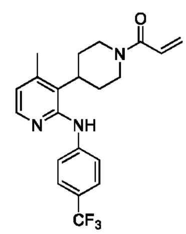
T23

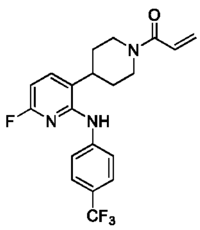
T24

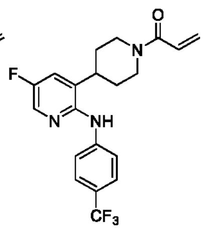
T25

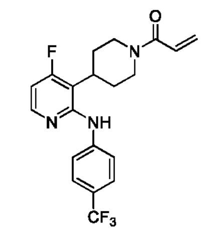
T26

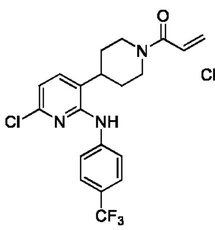
T27

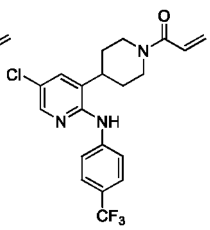
T28

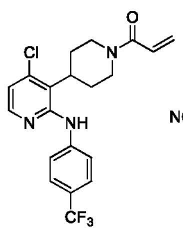
T29

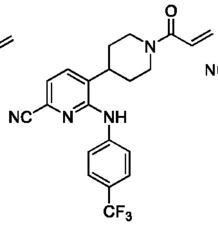
T30

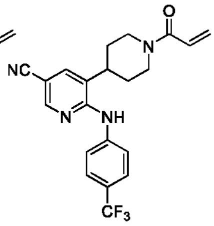
T31

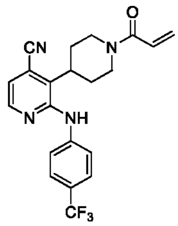
T32

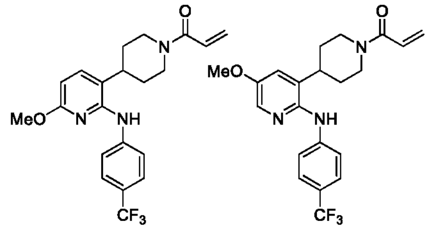

T33 T34

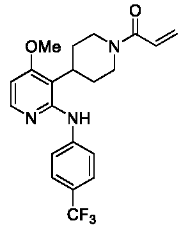

T35

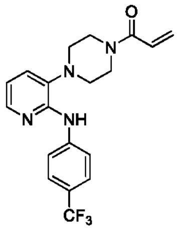

T36

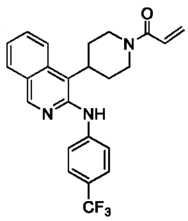

T37

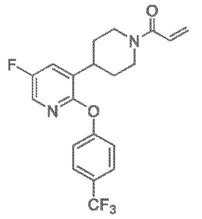

T38

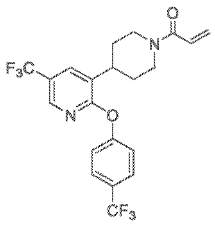

T39

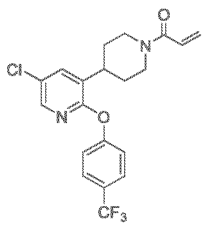

T40

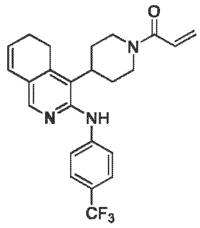

T41

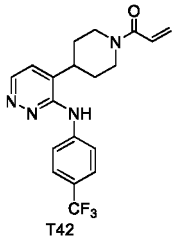

T42

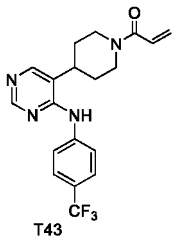

T43

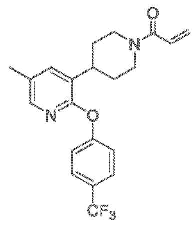

T44

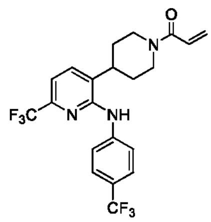

T45

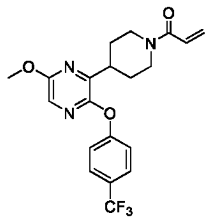

T46

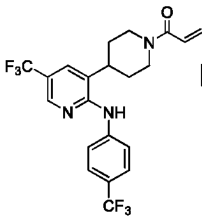

T47

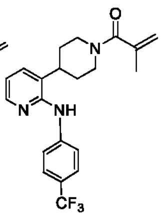

T48

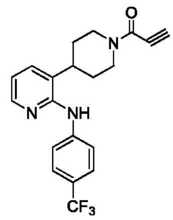

T49

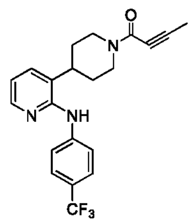

T50

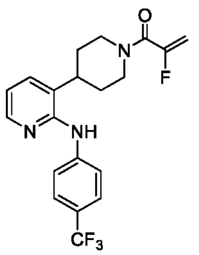

T51

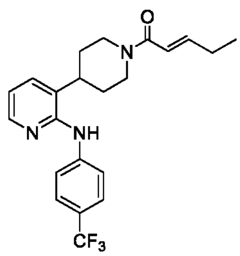

T52

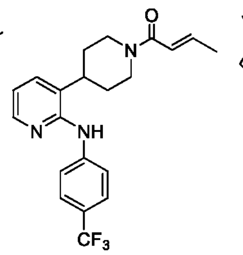

T53

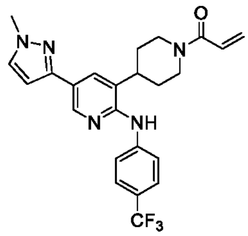

T54

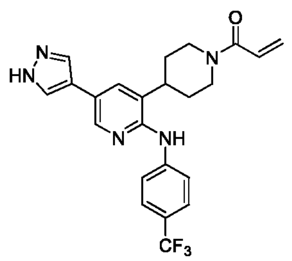

T55

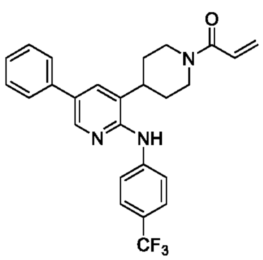

T56

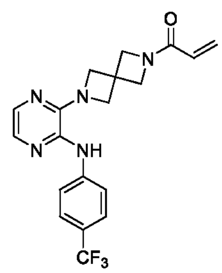

T57

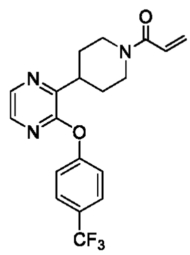

T58

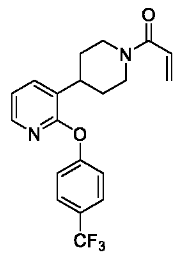

T59

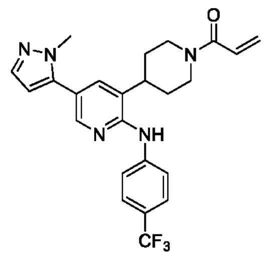

T60

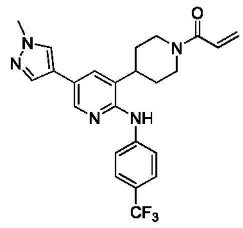
T61

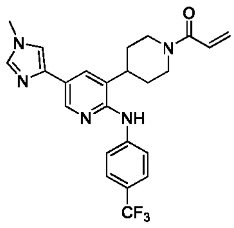
T62

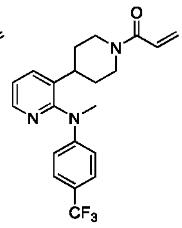
T63

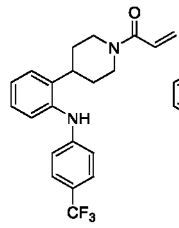
T64

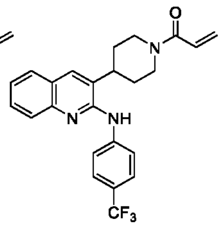
T65

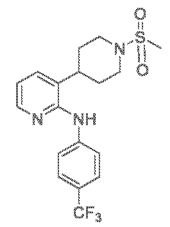
T66

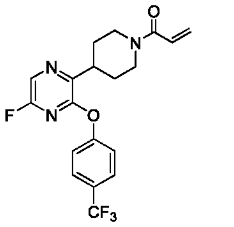
T67

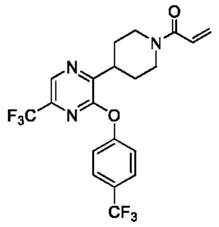
T68

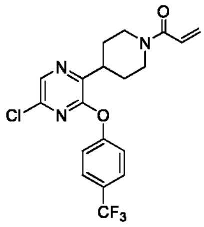
T69

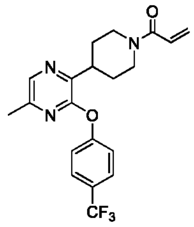
T70

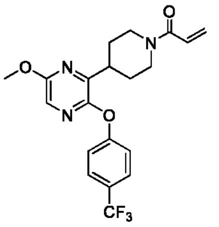
T71

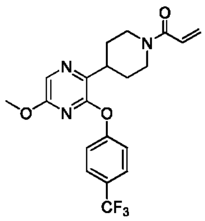
T72

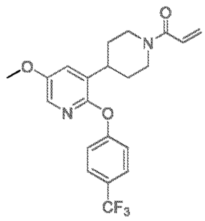
T73

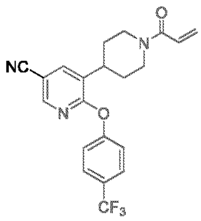
T74

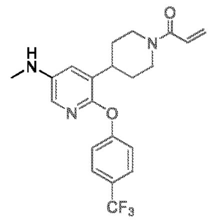
T75

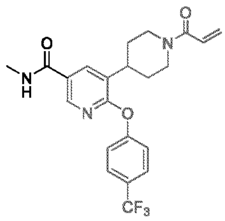
T76

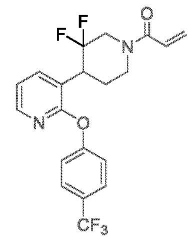
T77

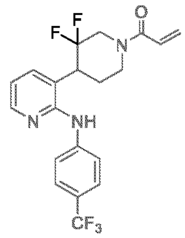
T78

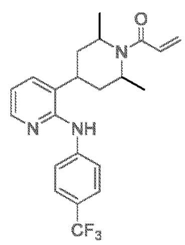
T79

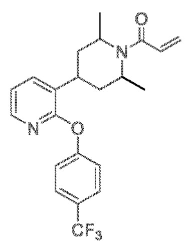
T80

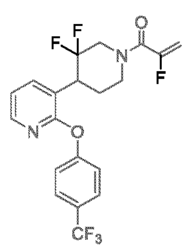
T81

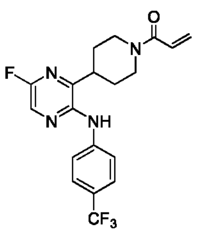
T82

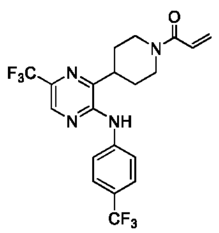
T83

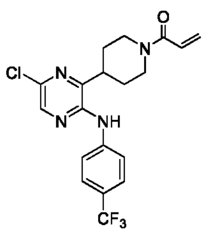
T84

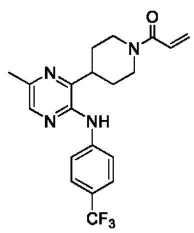
T85

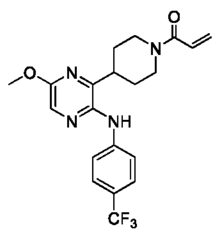
T86

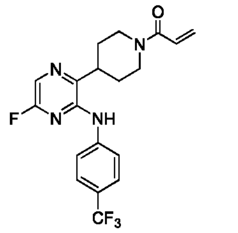
T87

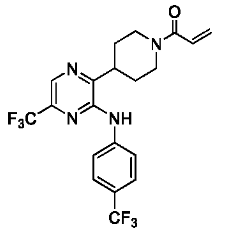
T88

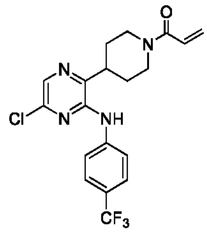
T89

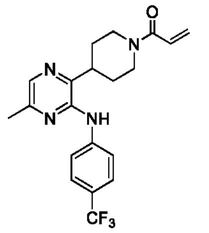
T90

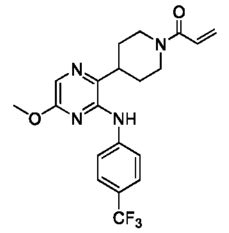
T91

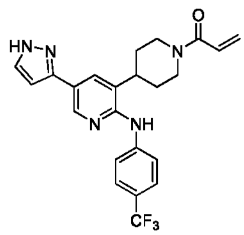
T92

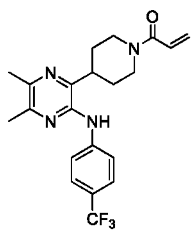
T93

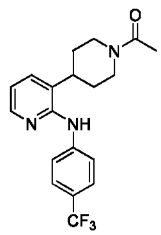
T94

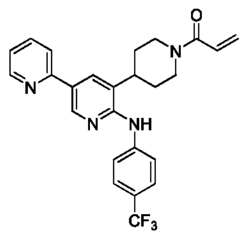
T95

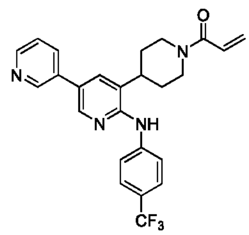
T96

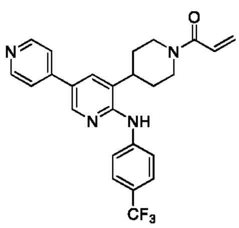
T97

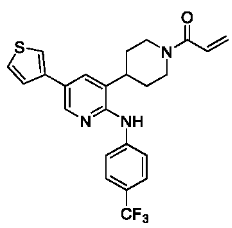
T98

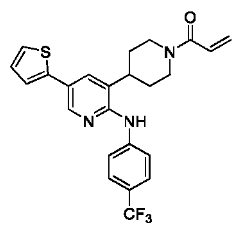
T99

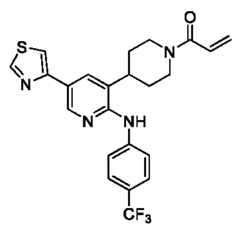
T100

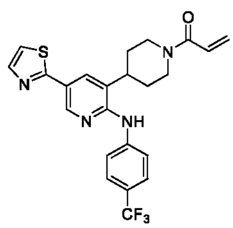
T101

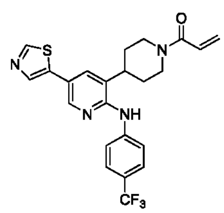
T102

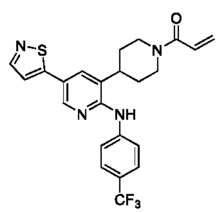
T103

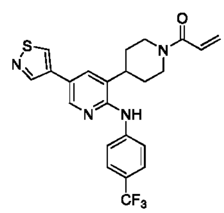
T104

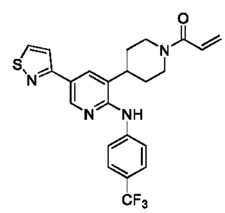
T105

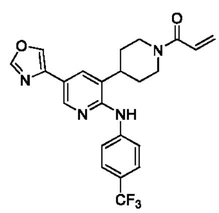
T106

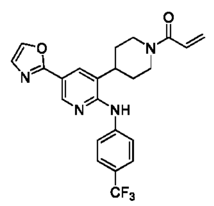
T107

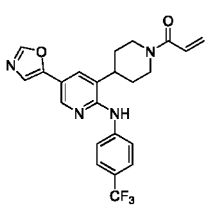
T108

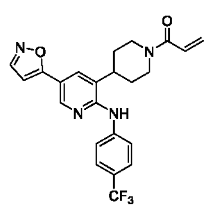
T109

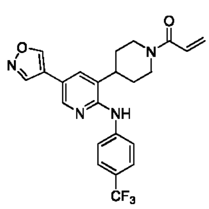
T110

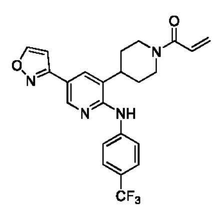
T111

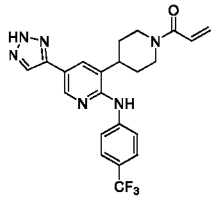
T112

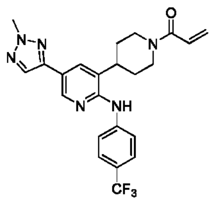
T113

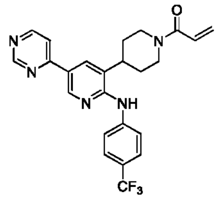
T114

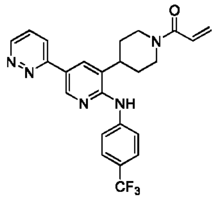
T115

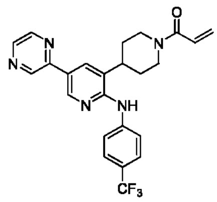
T116

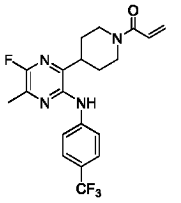
T117

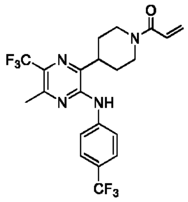
T118

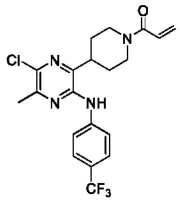
T119

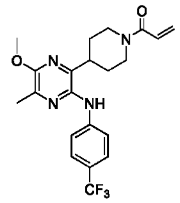
T120

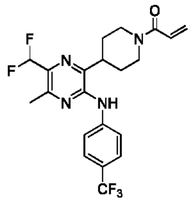
T121

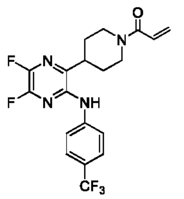
T122

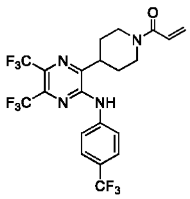
T123

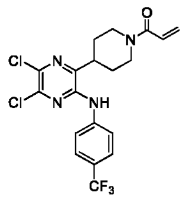
T124

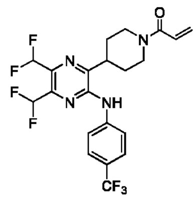
T125

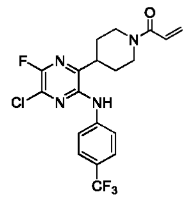
T126

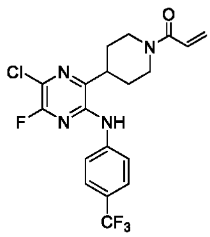
T127

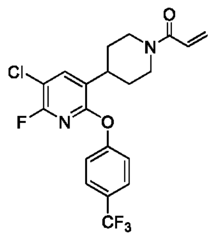
T128

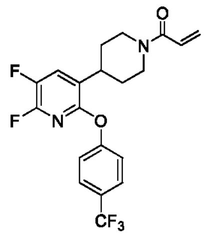
T129 。

In the second aspect of the present invention, it provides a pharmaceutical composition comprises pharmaceutically acceptable carriers and one or more safe and effective amounts of the compound or the pharmaceutically acceptable salt, solvate or prodrug thereof of the first aspect of the present invention.

In the third aspect of the present invention, it provides a use of the pharmaceutical composition of the second aspect of the present invention for the preparation of a drug, the drug is used for the prevention and/or treatment of proliferative diseases.

In another preferred embodiment, the proliferative disease is cancer.

In another preferred embodiment, the cancer is selected from the group consisting of brain glioma, prostate cancer, interpleural septal tumor, breast cancer, ovarian cancer, colon cancer, liver cancer, pancreatic cancer, and squamous cell carcinoma.

In the fourth aspect of the present invention, it provides a use of the pharmaceutical composition of the second aspect of the present invention for the preparation of a drug, the drug is used for purposes selected from the group consisting of:

1) prevention and/or treatment of diseases associated with abnormal activity of YAP/TEAD;
2) prevention and/or treatment of diseases associated with dysregulation of the Hippo pathway; and
3) preventing and/or treatment diseases associated with dysregulation of YAP or TAZ or YAP/TAZ or YAP/TEAD or YAP/TAZ/TEAD.

In another preferred embodiment, the drug is used for the prevention and/or treatment of diseases associated with YAP/TEAD or YAP/TAZ/TEAD dysregulation.

In another preferred embodiment, the disease associated with abnormal activity of YAP/TEAD is cancer.

In another preferred embodiment, the disease associated with dysregulation of the Hippo pathway is cancer.

In another preferred embodiment, the disease associated with dysregulation of YAP or TAZ or YAP/TAZ or YAP/TEAD or YAP/TAZ/TEAD is cancer.

In the fifth aspect of the present invention, it provides a combination of the compound or the pharmaceutically acceptable salt, solvate or prodrug thereof of the first aspect of the present invention with a second drug, which is used for the prevention and/or treatment of cancer;

the second drug is selected from the group consisting of ERK inhibitors, MEK inhibitors, KRAS inhibitors, BRAF inhibitors, EGFR inhibitors, Wnt inhibitors and PD-1 inhibitors.

In another preferred embodiment, the ERK inhibitors are selected from the group consisting of AZD0364, Ulixertinib, or a combination thereof.

In another preferred embodiment, the MEK inhibitors are selected from the group consisting of cobimetinib, binimetinib, or a combination thereof.

In another preferred embodiment, the KRAS inhibitors are selected from the group consisting of Adagrasib (MRTX849), JNJ-74699157 (ARS-3248), JAB-3312, or a combination thereof.

In another preferred embodiment, the BRAF inhibitors are selected from the group consisting of sorafenib, regorafenib, or a combination thereof.

In another preferred embodiment, the EGFR inhibitors are selected from the group consisting of ositinib, daclotinib, gefitinib, or a combination thereof.

In another preferred embodiment, the Wnt inhibitors are selected from the group consisting of IWR-1, ETC-159, or a combination thereof.

In another preferred embodiment, the PD-1 inhibitors are selected from the group consisting of navumab, atezumab, pembrolizumab, or a combination thereof.

In another preferred embodiment, the cancer is selected from the group consisting of brain glioma, prostate cancer, interpleural septal tumor, breast cancer, ovarian cancer, colon cancer, liver cancer, pancreatic cancer, and squamous cell carcinoma.

It should be understood that in the present invention, any of the technical features specifically described above and below (such as in the Examples) can be combined with each other, so as to constitute new or preferred technical solutions. Due to space limitations, it will not redundantly be described one by one herein.

## DETAILED DESCRIPTION OF THE INVENTION

After long-term and in-depth research, the present inventors have unexpectedly prepared a compound of formula I with a novel structure, excellent proliferation inhibitory activity and excellent YAP-TEAD inhibitory activity. Preferably, the present invention obtained a compound with the above excellent properties by optimizing $R_1$. On above basis, the inventors have completed the present invention.

## TERMS

In the present invention, unless otherwise indicated, the terms used have the ordinary meaning known to the skilled person in the art.

In the present invention, the term "halogen" refers to F, Cl, Br or I.

In the present invention, the term "$C_{1-6}$ alkyl" refers to straight-chain or branched alkyl groups comprising 1-6 carbon atoms, for example methyl, ethyl, propyl, iso-propyl, butyl, iso-butyl, tert-butyl, neo-pentyl, tert-pentyl, or the like.

In the present invention, the term "$C_{2-6}$ alkenyl" refers to a straight or branched alkenyl group with 2-6 carbon atoms containing a double bond, and non-limitingly includes vinyl, propenyl, butenyl, iso-butenyl, pentenyl, hexenyl and the like.

In the present invention, the term "$C_{2-6}$ alkynyl" refers to a straight or branched alkynyl group with 2-6 carbon atoms containing a triple bond, and non-limitingly includes ethynyl, propynyl, butynyl, iso-butynyl, pentynyl and hexynyl, etc.

In the present invention, the term "$C_{3-6}$ cyclic hydrocarbon" includes a group selected from the group consisting of $C_{3-6}$ cycloalkyl, $C_{3-6}$ cycloalkenyl, $C_{3-6}$ cycloalkynyl.

In the present invention, the term "$C_{3-6}$ cycloalkyl" refers to a cyclic alkyl group with 3-6 carbon atoms on the ring, and non-limitingly includes cyclopropyl, cyclobutyl, cyclopentyl, cyclohexyl, and the like.

In the present invention, the term " $C_{5-8}$ bridged cycloalkyl" refers to a cyclic alkyl group having a bridge and having 5-8 carbon atoms, and non-limitingly includes

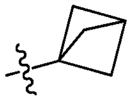, 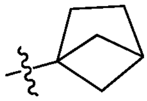, and 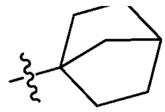,

etc.

In the present invention, the term "$C_{1-6}$ alkoxy" refers to straight-chain or branched alkoxy groups with 1-6 carbon atoms, and non-limitingly includes methoxy, ethoxy, propoxy, iso-propoxy and butoxy. Preferably $C_{1-4}$ alkoxy.

In the present invention, the term "heterocyclyl" refers to a 4-8 membered heterocvelvl containing 1 2 or 3 heteroatoms selected from N. O. S. including but not limited to the following groups:

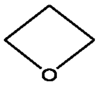, 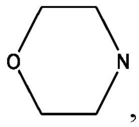, 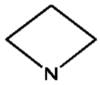, 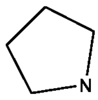, 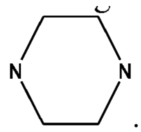.

In the present invention, the terms "aryl ring" or "aryl" have the same meaning, preferably "$C_{6-10}$ aryl". The term "$C_{6-10}$ aryl" refers to an aromatic ring with 6-10 carbon atoms without heteroatoms on the ring, such as phenyl, naphthyl, etc.

In the present invention, the term "aromatic heterocycle" or "heteroaryl" has the same meaning and refers to a heteroaromatic group containing one to more heteroatoms. For example, "C3-C10 heteroaryl" refers to an aromatic heterocyclic ring containing 1 to 4 heteroatoms selected from oxygen, sulfur, and nitrogen and 3 - 10 carbon atoms. Non-limiting examples are furanyl, thienyl, pyridinyl, pyrazolyl, pyrrolyl, N-alkylpyrrolyl, pyrimidinyl, pyrazinyl, imidazolyl, tetrazolyl, etc. The heteroaryl ring may be fused on an aryl, heterocyclyl or cycloalkyl ring, wherein the ring attached to the parent structure is a heteroaryl ring. The heteroaryl group may be optionally substituted or unsubstituted.

In the present invention, the term "halogenated" means substituted with a halogen.

In the present invention, the term "deuterated" means substituted with deuterium.

In the present invention, the term "substituted" refers to one or more hydrogen atoms on a particular group are substituted by a particular substituent. The particular substituent is a substituent described above accordingly, or a substituent in the embodiments. Unless indicated specifically, a substituted group may have a substituent group selected from a particular group at any substitutable site of the group, and the substituent group can be identical or different at each site. It should be understood by the person skilled in the art that the combinations of substituents expected in the present invention are those that are stable or chemically realizable. For example (but not limited to), the substituents are halogen, hydroxyl, carboxy (-COOH), C1-C6 alkyl, C2-C6 alkenyl, C2-C6 alkynyl, C3-C8 cycloalkyl, 3-12 membered heterocyclyl, aryl, heteroaryl, C1-C8 aldehyde, C2-C10 acyl, C2-C10 ester, amino, C1-C6 alkoxy, C1-C10 sulfonyl, etc.

In the present invention, the terms 1-6 refer to 1, 2, 3, 4, 5 or 6. Other similar terms each independently have a similar meaning. The term "more" refers to 2-6, such as 2, 3, 4, 5 or 6.

It should be understood that when a certain group is present at a number of different sites in a compound, its definition at each site is independent and may be the same or different. That is, the term "selected from the group consisting of" has the same meaning as the term "each independently selected from the group consisting of".

**Compounds and Preparation Method thereof**

The present invention provides a compound of formula I or a pharmaceutically acceptable salt, solvate or prodrug thereof:

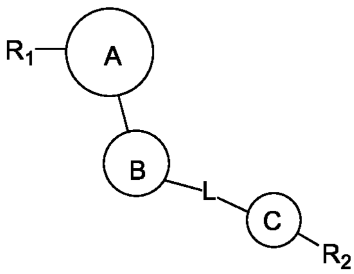

Formula I

wherein, each of the groups is as defined above.

In another preferred embodiment, any one of $R_1$, $R_2$, A, B, L, C in the compound, respectively, is independently a

corresponding group in the specific compound described in the present invention.

In another preferred embodiment, the compound is the specific compound shown in the present invention.

As used herein, the term "pharmaceutically acceptable salt" refers to a salt formed by a compound of the present invention with an acid or base suitable for use as a medicine. Pharmaceutically acceptable salts include inorganic salts and organic salts. A preferred class of salts is the salts of the compounds of the invention formed with acids. Suitable acids for forming salts include, but are not limited to inorganic acids such as hydrochloric acid, hydrobromic acid, hydrofluoric acid, sulfuric acid, nitric acid, phosphoric acid; organic acids such as formic acid, acetic acid, trifluoroacetic acid, propionic acid, oxalic acid, malonic acid, succinic acid, fumaric acid, maleic acid, lactic acid, malic acid, tartaric acid, citric acid, picric acid, benzoic acid, methylsulfonic acid, ethanesulfonic acid, p-toluenesulfonic acid, benzenesulfonic acid, naphthalene sulfonic acid; and amino acids such as proline, phenylalanine, aspartic acid and glutamic acid.

Another preferred class of salts are salts of the compounds of the invention formed with bases, such as alkali metal salts (such as sodium or potassium salts), alkaline earth metal salts (such as magnesium or calcium salts), ammonium salts (such as lower grades alkanol ammonium salts and other pharmaceutically acceptable amine salts), such as methylamine salt, ethylamine salt, propylamine salt, dimethylamine salt, trimethylamine salt, diethylamine salt, triethylamine salt, tert-butylamine salt, ethylenediamine salt, hydroxyethylamine salt, dihydroxyethylamine salt, trishydroxyethylamine salt, and an amine salt formed from morpholine, piperazine, and lysine, respectively.

The term "solvate" means that the compounds of the present invention are coordinated with solvent molecules to form complexes in specific ratios.

In addition, the compounds of the present invention include prodrugs of the compounds shown in Formula I. The term "prodrug" includes a compound which may be biologically active or inactive in itself, and which, when taken by suitable means, undergoes metabolism or chemical reactions in the body and is converted into a class of compounds of formula I, or a salt or solution comprising one of the compounds of formula I. The prodrugs include, but are not limited to, the forms of carboxylic acid esters, carbonate esters, phosphate esters, nitrate esters, sulfate esters, sulfone esters, sulfoxide esters, amino compounds, carbamates, azo compounds, phosphamide, glucosides, ethers, acetals and the like.

It should be understood that the compounds of the present invention may also be conveniently made by optionally combining various synthetic methods described in this specification or known in the art, and such combinations can be readily carried out by one skilled in the art to which the present invention belongs.

Typically, the raw materials and reagents used in the processes for the preparation of the compounds of the present invention are commercially available without special indication.

**Pharmaceutical Compositions and Administration Methods**

The present invention also provides a pharmaceutical composition comprises pharmaceutically acceptable carriers and one or more safe and effective amounts of the compound or the pharmaceutically acceptable salt, solvate or prodrug thereof.

Because of the superior antitumor activity of the compounds of the present invention, the compounds of the present invention and their various crystalline forms, pharmaceutically acceptable inorganic or organic salts, hydrates or solvates, and pharmaceutical compositions containing the compounds of the present invention as the principal active ingredient may be used for the treatment, prevention, and palliation of tumor-related diseases.

The pharmaceutical composition of the present invention comprises a safe and effective amount of a compound of the present invention or a pharmacologically acceptable salt thereof, and a pharmacologically acceptable excipient or carrier. In which, "safe and effective amount" is meant that the amount of the compound is sufficient to significantly improve the condition without causing serious side effects. Generally, the pharmaceutical composition contains 1-2000 mg of the compound of the present invention/dose, more preferably, 10-1000 mg of the compound of the present invention/dose. Preferably, the "dose" is a capsule or tablet.

"Pharmaceutically acceptable carrier" means one or more compatible solid or liquid fillers or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween®), wetting agent (such as sodium dodecyl sulfate), coloring agents, flavoring agents, stabilizers, antioxidants, preservatives, pyrogen-free water, etc..

The pharmaceutical composition is an injection, a capsule, a tablet, a pill, a powder, or a granule.

The administration mode of the compound or pharmaceutical composition of the present invention is not particularly limited, and representative administration modes include, but are not limited to, oral, intratumoral, rectal, parenteral (intravenous, intramuscular or subcutaneous) and topical administration.

Solid dosage forms for oral administration include capsules, tablets, pills, powders and granules. In these solid dosage forms, the active compound is mixed with at least one conventional inert excipient (or carrier), such as sodium citrate or dicalcium phosphate, or mixed with any of the following components: (a) fillers or compatibilizer, such as starch, lactose, sucrose, glucose, mannitol and silicic acid; (b) binders, such as hydroxymethyl cellulose, alginate, gelatin, polyvinylpyrrolidone, sucrose and arabic gum; (c) humectants, such as glycerol; (d) disintegrating agents such as agar, calcium carbonate, potato starch or tapioca starch, alginic acid, certain composite silicates, and sodium carbonate; (e) dissolution-retarding agents, such as wax, (f) absorption accelerators, such as quaternary ammonium compound; (g) wetting agents, such as cetyl alcohol and glyceryl monostearate; (h) adsorbents, such as kaolin; and (i) lubricants, such as talc, calcium stearate, magnesium stearate, solid polyethylene glycol, sodium dodecyl sulfate or mixture thereof. In capsules, tablets and pills, the dosage forms may also contain buffering agents.

Solid dosage forms such as tablets, dragees, capsules, pills and granules can be prepared with coatings and shells such as enteric coatings and other materials known in the art. They may contain opacifying agents and the release of the active compound or compound in such compositions may be released in a portion of the digestive tract in a delayed manner. Examples of embedding components that can be employed are polymeric materials and waxy materials. If necessary, the active compound may also be in microencapsulated form with one or more of the above-mentioned excipients.

Liquid dosage forms for oral administration include pharmaceutically acceptable emulsions, solutions, suspensions, syrups or tinctures. In addition to the active compound, the liquid dosage form may contain inert diluents conventionally used in the art, such as water or other solvents, solubilizers and emulsifiers, such as ethanol, isopropanol, ethyl carbonate, ethyl acetate, propylene glycol, 1,3 -butanediol, dimethylformamide and oils, especially cottonseed oil, peanut oil, corn germ oil, olive oil, castor oil and sesame oil or mixtures of these substances.

In addition to these inert diluents, the compositions may contain adjuvants such as wetting agents, emulsifying and suspending agents, sweetening agents, flavoring agents and spices.

In addition to the active compound, the suspension may contain suspending agent, such as ethoxylated isooctadecanol, polyoxyethylene sorbitol and dehydrated sorbitan ester, microcrystalline cellulose, aluminum methoxide and agar, or the mixture thereof etc.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

Dosage forms for the compounds of the invention for topical administration include ointments, powders, patches, sprays and inhalants. The active ingredient is mixed under sterile conditions with a physiologically acceptable carrier and any preservatives, buffers, or propellants which may be required if necessary.

The compounds of the invention can be administered alone or in combination with other pharmaceutically acceptable compounds (e.g., anti-tumor drugs).

The treatment method of the present invention can be administered alone or in combination with other treatment means or therapeutic drugs.

When the pharmaceutical composition is used, a safe and effective amount of the compound of the present invention is administered to a mammal(such as a human) in need of treatment, wherein the dosage at the time of administration is the pharmaceutically effective dosage, for people having a body weight of 60kg, the daily dose is usually 1~2000mg, preferably 50~1000mg. Of course, specific doses should also consider factors such as the administration route, the health of the patient, etc., which are within the skill of the skilled physician.

Compared with the prior art, the present invention has the following main advantages:

(1) The compound has excellent proliferation inhibitory activity;
(2) The compound has excellent YAP-TEAD inhibitory activity;
(3) The compound has excellent pharmacokinetic properties;
(4) The compound has excellent blood-brain barrier (BBB) crossing properties.

The present invention will be further illustrated below with reference to the specific examples. It should be understood that these examples are only to illustrate the invention but not to limit the scope of the invention. Experimental methods in which the specific conditions are not specified in the following examples are usually in accordance with conventional conditions such as the conditions described in Sambrook et al., Molecular Cloning: Laboratory Manual (New York: Cold Spring Harbor Laboratory Press, 1989), or in accordance with the conditions recommended by the manufacturer. Unless indicated otherwise, percentage and parts are calculated by weight.

Unless otherwise defined, all professional and scientific terminology used in the text have the same meanings as known to the skilled in the art. In addition, any methods and materials similar or equal with the recorded content can apply to the methods of the invention. The method of the preferred embodiment described herein and the material are only for

demonstration purposes.

**Example 1**

Compound synthesized in the present invention:

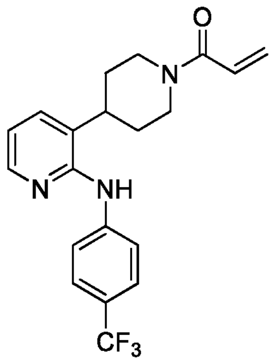

T1

The synthesis route is as follows:

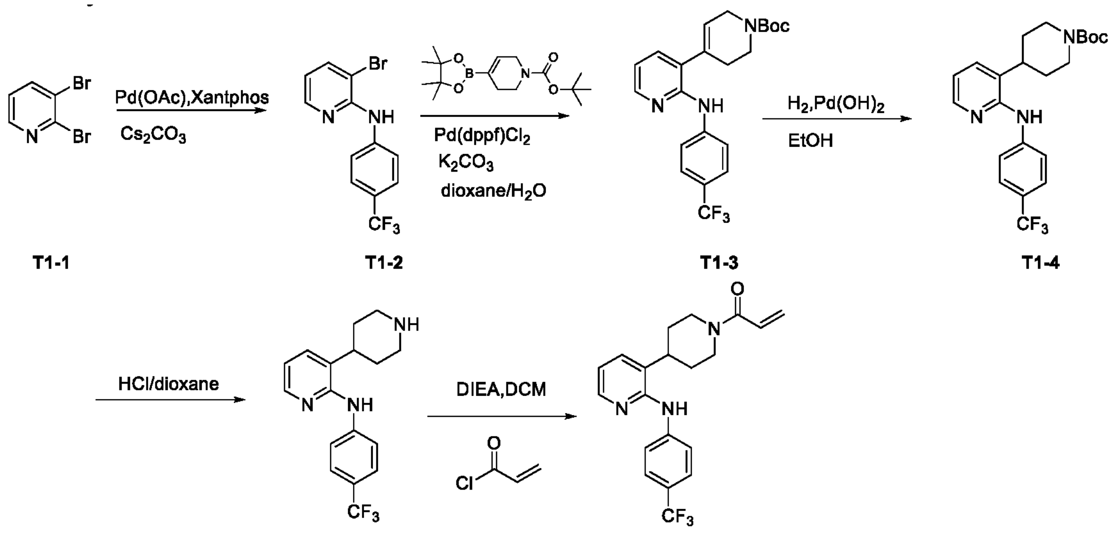


T1-1 T1-2 T1-3 T1-4

T1-5 T1

The experimental procedure is as follows:

(1) Synthesis of compound T1-2

Compound T1-1 (10 g, 42.21 mmol, 1.0 eqiv), p-trifluoroaniline (6.8 g, 42.21 mmol, 1.0 eqiv), $Cs_2CO_3$ (11.49 g, 84.43 mmol, 2.0 eqiv), $Pd(OAc)_2$ (473.86 mg, 2.11 mmol, 5% eqiv ), Xanthpos (2.44 g, 4.22 mmol, 10% eqiv), and ultra-dry dioxane (200 mL) were added in a 500 mL triple-necked vial, evacuated and replaced with nitrogen, and warmed up to 105 °C for 16 h.

The reaction was completed, cooled to room temperature, 200 mL of water was added, extracted with ethyl acetate, the organic layers were washed with saturated brine, dried over anhydrous sodium sulfate, and concentrated to obtain the crude product which was purified by column chromatography using PE 100%, PE:EA=500:1 to obtain 5.4 g of product T1-2 (white solid).

(2) Synthesis of compound T1-3

Compound T1-2 (1.2 g, 3.78 mol, 1.0 eq), pinacol borate (1.4 g, 4.54 mmol,1.2 eqiv), potassium carbonate (1.05

g, 7.57 mmol,2.0 eqiv), water/1,4-dioxane (1/10) (22 mL), Pd(dppf)$Cl_2$ (135.64 mg, 0.189 mmol, 5% eq) were added in a 100 mL single-necked vial in turn, nitrogen was replaced three times, and the reaction solution was put into the oil bath, warmed to 105 °C for 16 h, after the reaction was completed, cooled to room temperature and 20 mL of water was added, extracted with ethyl acetate (25 ml * 3), the combined organic layer was washed with saturated brine (25 mL), dried over anhydrous sodium sulfate, the filtrate was suction filtered, spin-dried and treated by column to obtain 1.5 g of product T1-3 (light yellow viscous oil).

(3) Synthesis of compound T1-4

Compound T1-3 (1.5 g ) was dissolved in ethanol (40 mL), Pd/$(OH)_2$ (150 mg, 10%) was added, and the reaction was carried out under hydrogen atmosphere for 16 hours until the raw material was disappeared, and then directly filtered and spin-dried to obtain 1.45 g of the crude product, T1-4, which was gray and viscous.

(4) Synthesis of compound T1-5

Compound T1-4 (1.45 g) was added to methanol (20 ml) followed by HCl/dioxane (40 mL, 4M) and then reacted overnight at room temperature. The reaction solution was concentrated, then saturated sodium bicarbonate was added, extracted with ethyl acetate, then concentrated and 1.14g of gray solid T1-5 was obtained after using dichloromethane to remove solvent.

(7) Synthesis of compound T1

Compound T1-5 (138.5 mg, 0.43 mmol, 1.0 eq), DIEA (111.41 mg,0.86 mmol,2.e eq), DCM (2 ml) were added to the reaction tube, then acryloyl chloride (46.81 mg, 0.52 mmol, 1.2 eq)in DCM solution (1 ml) was added dropwise at 0 °C, stirred overnight at room temperature. The solution was washed with 0.1 N HCl and extracted with EA, then separated and purified by Preparative Thin-Layer Chromatography to obtain 42.6 mg of T1 (white solid).

$^1$HNMR: $CDCl_3$ 8.6(s,1H),7.44-7.49(m,4H),7.37-7.49(d,1H),6.83-6.86(m,1H), 6.52-6.59(s,1H),6.35(s,1H),6.23-6.27(d,1H),5.56-5.67(d,1H),4.81-4.85(d,1H), 4.10-4.13(d,1H), 3.13-3.19(t,1H), 2.67-2.77(d,2H), 1.91-1.94(d,2H),1.15-1.18(m,2H)
LC-MS[M+1]: 376.5。

Referring to the synthesis method in Example 1, the following compounds were synthesized:

| Compound No. | Compound Structure | Compound Characterization |
|---|---|---|
| T3 | 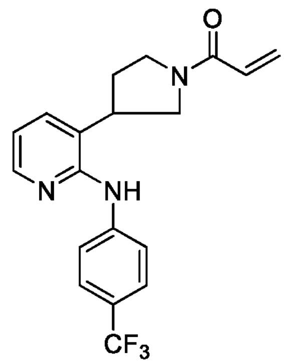 | LC-MS[M+1]: 362.1 |

(continued)

| Compound No. | Compound Structure | Compound Characterization |
|---|---|---|
| T5 | 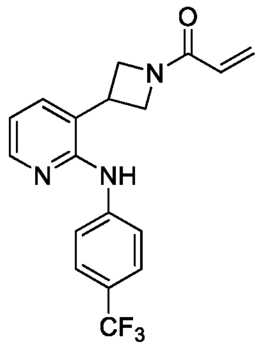 | LC-MS[M+1]: 348.1 |
| T6 | 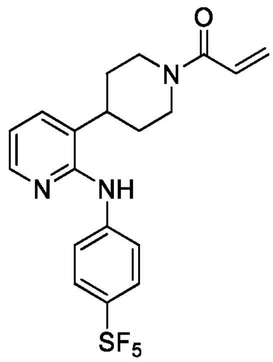 | LC-MS[M+1]: 434.1 |
| T8 | 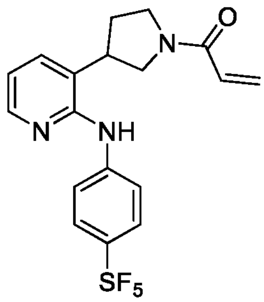 | LC-MS[M+1]: 420.1 |
| T10 | 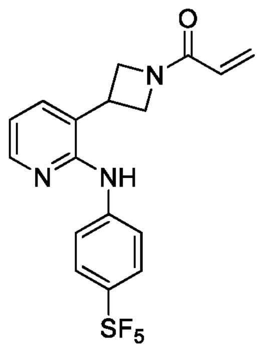 | LC-MS[M+1]: 406.1 |

(continued)

| Compound No. | Compound Structure | Compound Characterization |
|---|---|---|
| T11 | 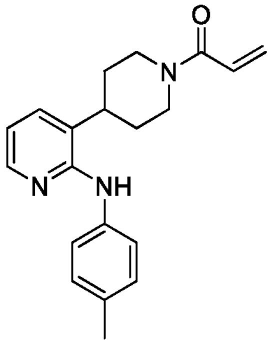 | LC-MS[M+1]: 322.2 |
| T13 | 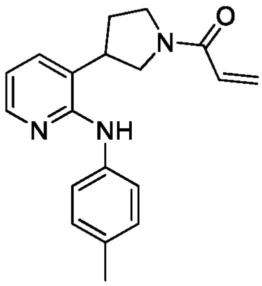 | LC-MS[M+1]: 308.2 |
| T15 | 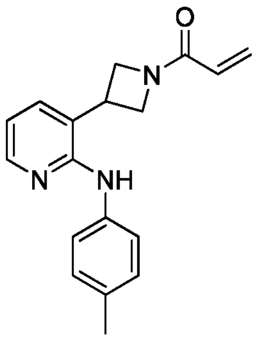 | LC-MS[M+1]: 294.1 |
| T36 | 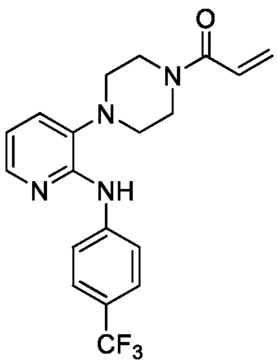 | LC-MS[M+1]: 377.1 |

(continued)

| Compound No. | Compound Structure | Compound Characterization |
|---|---|---|
| T78 | 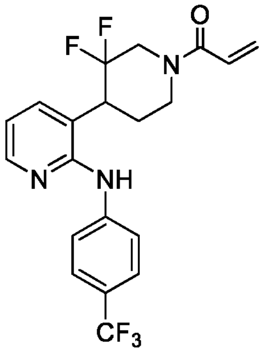 | LC-MS[M+1]: 404.2 |
| T79 | 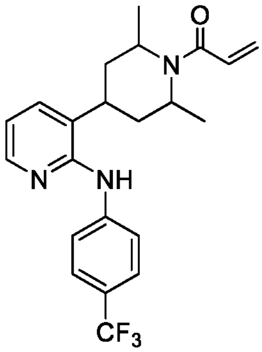 | LC-MS[M+1]: 405.2 |

**Example 2**

Compound synthesized in the present invention:

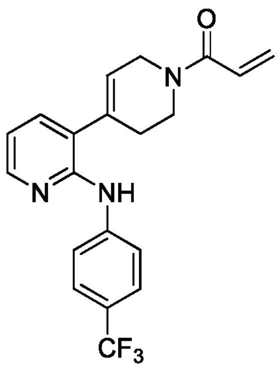

**T2**

The synthesis route is as follows:

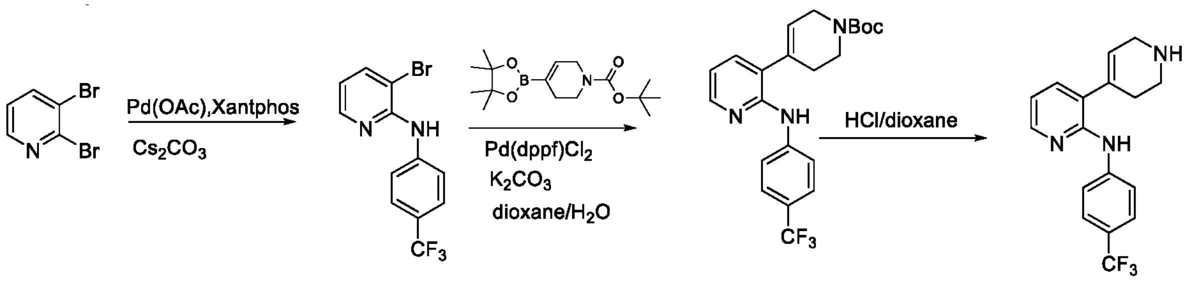


T2-1 T2-2 T2-3 T2-4

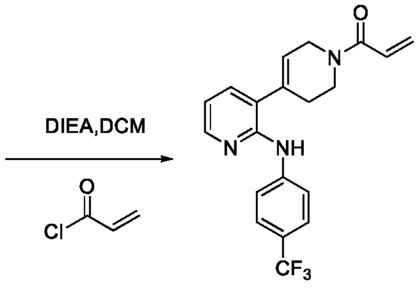


T2

The experimental procedure is as follows:

(1) Synthesis of compound T2-2

Compound T2-1 (10 g, 42.21 mmol, 1.0 eqiv), p-trifluoroaniline (6.8 g, 42.21 mmol, 1.0 eqiv), $Cs_2CO_3$ (11.49 g, 84.43 mmol, 2.0 eqiv), $Pd(OAc)_2$ (473.86 mg, 2.11 mmol, 5% eqiv ), Xanthpos (2.44 g, 4.22 mmol, 10%), and ultra-dry dioxane (200 mL) were added in a 500 mL three-necked vial, evacuated and replaced with nitrogen, and warmed to 105 °C for 16 h.

The reaction was completed, cooled to room temperature, 200 mL of water was added, extracted with ethyl acetate, the organic layers were washed with saturated brine, dried over anhydrous sodium sulfate, and concentrated to obtain the crude product which was purified by column chromatography using PE 100%, PE:EA=500:1 to obtain 5.4 g of the product T2-2 (white solid).

(2) Synthesis of compound T2-3

Compound T2-2 (1.2 g, 3.78 mol, 1.0 eq), pinacol borate (1.4 g, 4.54 mmol,1.2 eqiv), potassium carbonate (1.05 g, 7.57 mmol, 2.0 eqiv), water/1,4-dioxane (1/10) (22 mL), $Pd(dppf)Cl_2$ (135.64 mg, 0.189 mmol, 5% eq) were added sequentially in a 100 mL single-necked vial, nitrogen was replaced three times, and the reaction solution was put into the oil bath, warmed to 105 °C for 16 h, after the reaction was completed, cooled to room temperature and then 20 mL of water was added, extracted with ethyl acetate (25 ml * 3), the organic layers were combined and washed with saturated brine (25 mL), dried over anhydrous sodium sulfate, the filtrate was suction filtered, and spin-dried over the column to obtain 1.5 g of product T2-3 (light yellow viscous oil).

(3) Synthesis of compound T2-4

Compound T2-3 (560 mg) was added to methanol (16 ml) followed by HCl/dioxane (40 mL, 4M), and then the reaction solution was reacted overnight at room temperature. The reaction solution was concentrated and saturated sodium bicarbonate was added, extracted with ethyl acetate, washed with saturated saline then concentrated, and 424 mg of white solid T2-4 was obtained after using dichloromethane to remove solvent (lcms: [M+1] 320.3).

(4) Synthesis of compound T2

Compound T2-4 ( 424 mg, 1.33 mmol, 1.0 eq), DIEA (343.75 mg,2.66 mmol,2.0 e), DCM (10 ml) were added to the reaction tube, then acryloyl chloride (144.36 mg, 1.6 mmol, 1.2 eq) in DCM solution (1 ml) was added dropwise at 0 °C,

and stirred overnight at room temperature. The solution was washed with 0.1 N HCl and saturated saline, then EA was added for extraction, and then separated and purified by column to obtain 90.8 mg of T2 (white solid).

$^1$HNMR : $CDCl_3$ 8.6(s,1H),7.91-7.93(m,1H),7.71-7.73(m,2H),7.60(s,1H), 7.27(s,1H),6.77-6.84(m,2H),6.38-6.42(m,1H),6.18-6.22(d,2H),5.56-5.86(m,3H), 3.94-4.15(m,2H),3.41-3.53(m,2H),1.92-2.43(m,2H)
LC-MS[M+1]: 374.4$_o$

Referring to the synthesis method in Example 2, the following compounds were synthesized:

| Compound No. | Compound Structure | Compound Characterization |
|---|---|---|
| T4 | 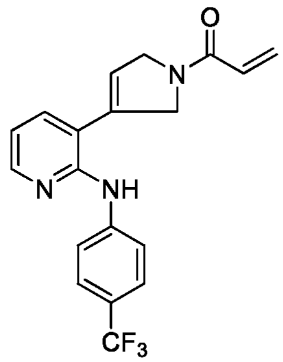 | LC-MS[M+1]: 360.5 |
| T7 | 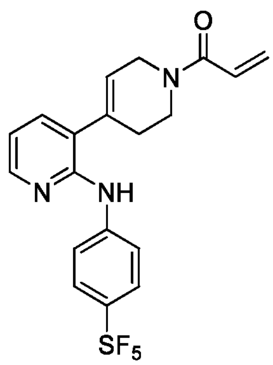 | LC-MS[M+1]: 432.1 |
| T9 | 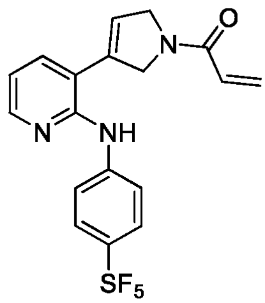 | LC-MS[M+1]: 418.1 |
| T12 | 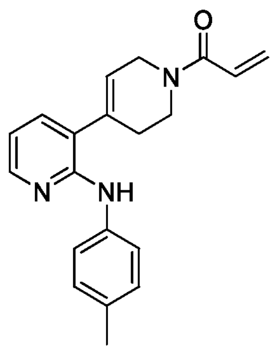 | LC-MS[M+1]: 320.1 |

(continued)

| Compound No. | Compound Structure | Compound Characterization |
|---|---|---|
| T14 | 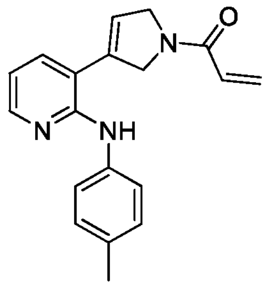 | LC-MS[M+1]: 306.1 |

**Example 3**

Compound synthesized in the present invention:

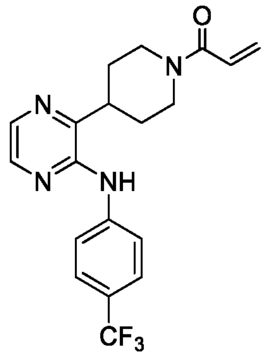

T16

The synthesis route is as follows:

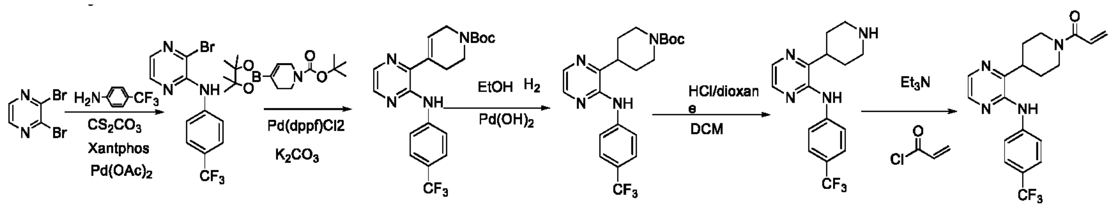


T16-1 T16-2 T16-3 T16-4 T16-5 T16

The experimental procedure is as follows:

(1) Synthesis of T16-2

T16-1 (3 g, 12.77 mmol, 1.0 eq), p-trifluoroaniline (2.06 g, 12.77 mmol, 1 eq), $CS_2CO_3$ (8.3 g, 25.54 mmol, 2 eq), Xanthpos (740.6 mg, 1.28 mmol, 10% eq), $Pd(OAc)_2$ (143.66 mg, 0.639mmol, 5% eqiv ), and ultra-dry 1,4 dioxane 70 ml were added in a reaction flask in turn, nitrogen was replaced three times, and the reaction solution was put into an oil bath, and raised to 105 °C for 16 h.

The reaction was completed, cooled to room temperature, filtered with diatomaceous earth, 100mL of water was added, extracted with ethyl acetate, the organic layers were washed with saturated brine, dried over anhydrous sodium sulfate, and concentrated to obtain the crude product, which was purified by column chromatography using PE 100%, PE:EA=500:1, to obtain 2.2g of T16-2 (white solid).

(2) Synthesis of T16-3

T16-2 (2 g, 6.31m mol, 1.0 eq), pinacol borate (2.33g, 7.57mmol,1.2 eqiv), potassium carbonate (1.74g,

12.62mmol,2.0 eqiv), water/1,4-dioxane (1/10) (4mL/40 mL), and Pd(dppf)$Cl_2$ (230.63 mg, 0.316 mmol, 5% eq) were added sequentially in a 100 mL single-necked vial, nitrogen was replaced three times, and the reaction solution was put into the oil bath, raised to 105 °C for 16 h, after the reaction was completed, cooled to room temperature, diluted with EA, filtered with diatomaceous earth, and water 80 mL was added, extracted with ethyl acetate (80 ml * 3), combined organic layer was washed with saturated saline (60 mL), dried over anhydrous sodium sulfate, the filtrate was suction filtered, spin-dried and passed through the column to obtain 2.6 g of T16-3 (light yellow solid).

(3) Synthesis of T16-4

T16-3 (1 g) was dissolved in ethanol (15 mL), Pd/$(OH)_2$ (100 mg, 10%) was added, and the reaction was carried out under hydrogen atmosphere for 16h until the raw material was disappeared, and 800 mg of crude T16-4 (white solid) was obtained by direct filtration and spin-dry.

(4) Synthesis of compound T16-5

Compound T16-4 (700 mg) was added to DCM (10 ml) followed by HCl/dioxane (20 mL, 4M), and then the reaction solution was reacted overnight at room temperature. 600m g of white solid T16-5 was obtained by direct spin-dry.

(7) Synthesis of compound T-16

Compound T16-5 ( 400 mg,1.24umol, 1.0 eq), triethylamine (376.4 mg,3.73 umol,3.0eq), DCM (3 ml) were added to the reaction tube, then acryloyl chloride (77 mg,1.36umol,1.1eq) in DCM solution (1 ml) was added dropwise at 0 °C, and stirred overnight at room temperature. The solution was washed with 0.1N HCl and then extracted with the addition of EA and then separated and purified by Preparative Thin-Layer Chromatography to obtain 110 mg of T16 (white solid).

$^1$H NMR (400 MHz, Chloroform-d) δ 8.07 (s, 2H), 7.66 - 7.58 (m, 4H), 6.63 (dd, *J* = 16.8, 10.5 Hz, 1H), 6.52 (s, 1H), 6.30 (d, *J* = 16.7 Hz, 1H), 5.72 (d, *J* = 10.8 Hz, 1H), 4.79 (s, 1H), 4.20 (d, *J* = 13.7 Hz, 1H), 3.29 (s, 1H), 2.95 (d, *J* = 34.7 Hz, 2H), 1.99 (s, 4H).

Referring to the synthesis method in Example 3, the following compounds were synthesized:

| Compound No. | Compound Structure | Compound Characterization |
|---|---|---|
| T17 | 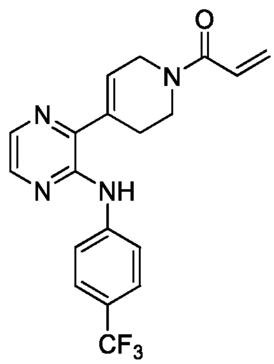 | LC-MS[M+1]: 375.1 |
| T18 | 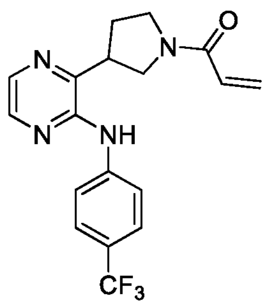 | LC-MS[M+1]: 363.1 |

(continued)

| Compound No. | Compound Structure | Compound Characterization |
|---|---|---|
| T19 | 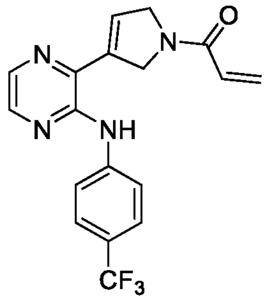 | LC-MS[M+1]: 361.1 |
| T20 | 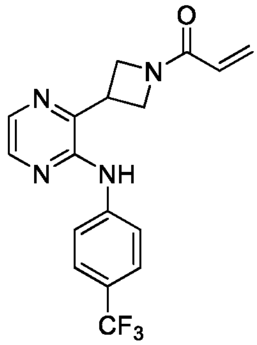 | LC-MS[M+1]: 348.1 |
| T42 | 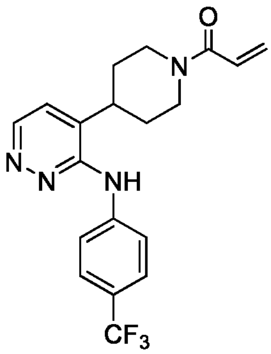 | $^1$H NMR (400 MHz, Chloroform-d) δ 8.68 (s, 1H), 8.28 (s, 1H), 7.74 (d, *J* = 8.4 Hz, 2H), 7.63 (d, *J* = 8.5 Hz, 2H), 6.69 - 6.58 (m, 2H), 6.34 (dd, *J* = 16.8, 1.9 Hz, 1H), 5.75 (dd, *J* = 10.6, 1.9 Hz, 1H), 4.93 (d, *J* = 13.2 Hz, 1H), 4.24 (d, *J* = 13.5 Hz, 1H), 3.27 (s, 1H), 2.88 - 2.68 (m, 2H), 2.10 - 2.02 (m, 2H), 1.77 (tt, *J* = 12.7, 6.3 Hz, 3H). |
| T43 | 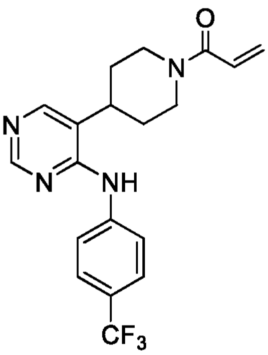 | LC-MS[M+1]: 377.1 |

(continued)

| Compound No. | Compound Structure | Compound Characterization |
|---|---|---|
| T93 | 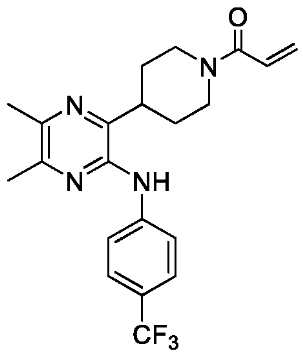 | LC-MS[M+1]: 405.2 |

**Example 4**

Compound synthesized in the present invention:

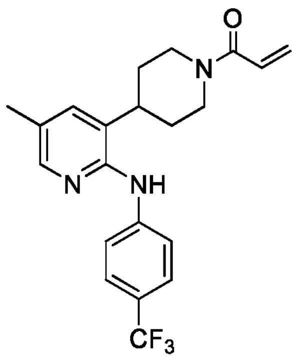

**T22**

The synthesis route is as follows:

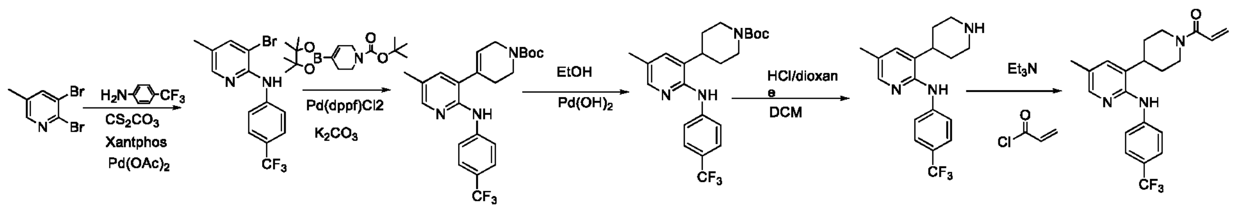


T22-1 T22-2 T22-3 T22-4 T22-5 T22

The experimental procedure is as follows:

(1) Synthesis of T22-2

T22-1 (5 g, 19.93 mmol, 1.0 eq), p-trifluoroaniline (3.21 g, 19.93 mmol, 1 eq), $CS_2CO_3$ (12.98 g, 39.85 mmol, 2 eq), Xanthpos (1.15 g, 1.99 mmol, 10% eq), $Pd(OAc)_2$ (223.68 mg, 0.996 mmol,5% eqiv ), and 70 ml of ultra-dry 1,4 dioxane were added in a reaction flask in turn, nitrogen was replaced three times, the reaction solution was put into an oil bath, and raised to 105 °C for 16h.

The reaction was completed, cooled to room temperature, filtered with diatomaceous earth, 100 mL of water was added, extracted with ethyl acetate, the organic layers were washed with saturated brine, dried over anhydrous sodium sulfate, and concentrated to obtain the crude product which was purified by column chromatography using PE 100%, PE:EA=500:1 to obtain 5.8 g T22-2 (white solid).

(2) Synthesis of T22-3

T22-2 (3 g, 9.06m mol, 1.0 eq), pinacol borate (3.36 g, 10.87 mmol,1.2 eqiv), potassium carbonate (2.5 g, 18.12 mmol, 2.0 eqiv), water/1,4-dioxane (1/10) (4 mL/40 mL), and Pd(dppf)$Cl_2$ (331.45 mg, 453 umol, 5% eq) were added sequentially in a 100 mL single-necked vial, nitrogen was replaced three times, the reaction solution was put into the oil bath, raised to 105 °C for 16 h, after the reaction was completed, cooled to room temperature, diluted with EA, filtered with diatomaceous earth, and water 80 mL was added, extracted with ethyl acetate (80 ml * 3), combined organic layer was washed with saturated saline (60 mL), dried over anhydrous sodium sulfate. The filtrate was suction filtered, spin-dried and passed through the column to obtain 3.9 g of T22-3 (light yellow solid).

(3) Synthesis of T22-4

T22-3 (3.9 g) was dissolved in ethanol (40 mL), Pd/$(OH)_2$ (390 mg, 10%) was added, and the reaction was carried out under hydrogen atmosphere for 16h until the raw material was disappeared, and 3.7 g of crude T22-4 (gray viscous) was obtained by direct filtration and spin-dry.

(4) Synthesis of compound T22-5

Compound T22-4 (1.4 g) was added into DCM (10 ml), then HCl/dioxane (25 mL, 4M) was added, and then the reaction was carried out at room temperature overnight. 1.2 g of gray solid T22-5 was obtained by direct spin-dry.

(7) Synthesis of compound T22

Compound T22-5 ( 300 mg, 0.773 mmol, 1.0 eq), triethylamine (234.3 mg,2.32 mmol,3.0 eq), and DCM (2 ml) were added to the reaction tube, then acryloyl chloride (77 mg, 0.85 mmol, 1.1 eq) in DCM solution (1 ml) was added dropwise at 0 °C, and stirred overnight at room temperature. The solution was washed with 0.1N HCl, then EA was added for extraction and then separated and purified by Preparative Thin-Layer Chromatography to obtain 65 mg of T22 (white solid).

$^1$H NMR (400 MHz, Methanol-$d_4$) δ 7.91 (dd, *J* = 2.2, 0.9 Hz, 1H), 7.52 - 7.44 (m, 3H), 7.38 (d, *J* = 8.7 Hz, 2H), 6.81 (dd, *J* = 16.8, 10.7 Hz, 1H), 6.20 (dd, *J* = 16.8, 2.0 Hz, 1H), 5.74 (dd, *J* = 10.6, 2.0 Hz, 1H), 4.73 (d, *J* = 13.4 Hz, 1H), 4.25 (d, *J* = 13.5 Hz, 1H), 3.17 (td, *J* = 8.5, 4.3 Hz, 1H), 2.84 (dd, *J* = 13.6, 10.9 Hz, 1H), 2.27 (s, 3H), 1.92 (t, *J* = 9.7 Hz, 2H), 1.61 (d, *J* = 12.7 Hz, 2H).

Referring to the synthesis method in Example 4, the following compounds were synthesized:

| Compound No. | Compound Structure | Compound Characterization |
|---|---|---|
| T21 | 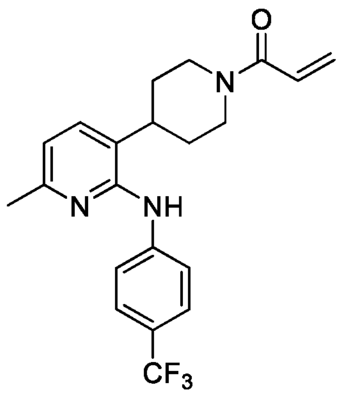 | LC-MS[M+1]: 390.1<br>1H NMR (400 MHz, Chloroform-d) δ 7.53 (s, 4H), 7.35 (d, J = 7.7 Hz, 1H), 6.79 (d, J = 7.8 Hz, 1H), 6.62 (dd, J = 16.8, 10.6 Hz, 1H), 6.40 - 6.25 (m, 2H), 5.72 (dd, J = 10.6, 2.0 Hz, 1H), 4.88 (d, J = 13.4 Hz, 1H), 4.17 (d, J = 13.8 Hz, 1H), 3.21 (t, J = 13.0 Hz, 1H), 2.82 - 2.69 (m, 2H), 2.46 (s, 3H), 1.96 (d, J = 13.4 Hz, 2H), 1.72 (s, 2H). |

(continued)

| Compound No. | Compound Structure | Compound Characterization |
|---|---|---|
| T23 | 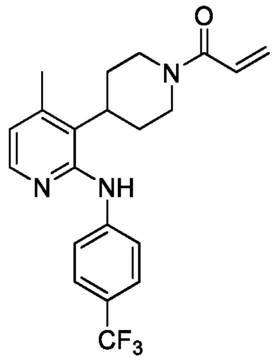 | $^1$H NMR (400 MHz, Methanol-$d_4$) δ 8.00 (d, *J*= 5.0 Hz, 1H), 7.44 (d, *J* = 8.4 Hz, 2H), 7.08 (d, *J* = 8.4 Hz, 2H), 6.98 (d, *J* = 5.1 Hz, 1H), 6.79 (dd, *J* = 16.8, 10.7 Hz, 1H), 6.19 (dd, *J* = 16.8, 2.0 Hz, 1H), 5.73 (dd, *J* = 10.6, 2.0 Hz, 1H), 4.71 (d, *J* = 13.3 Hz, 2H), 4.61 (s, 1H), 4.23 (d, *J* = 13.6 Hz, 1H), 3.47 - 3.36 (m, 1H), 3.22 (d, *J* = 12.9 Hz, 1H), 2.81 (t, *J* = 12.3 Hz, 1H), 2.47 (s, 3H), 2.23 - 2.15 (m, 2H), 1.76 (d, *J* = 12.7 Hz, 2H). |

**Example 5**

Compound synthesized in the present invention:

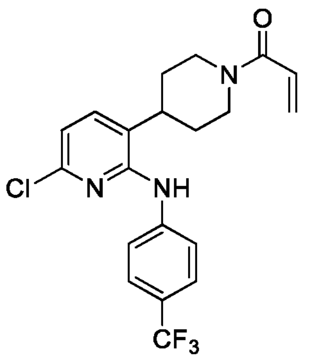

T27

The synthesis route is as follows:

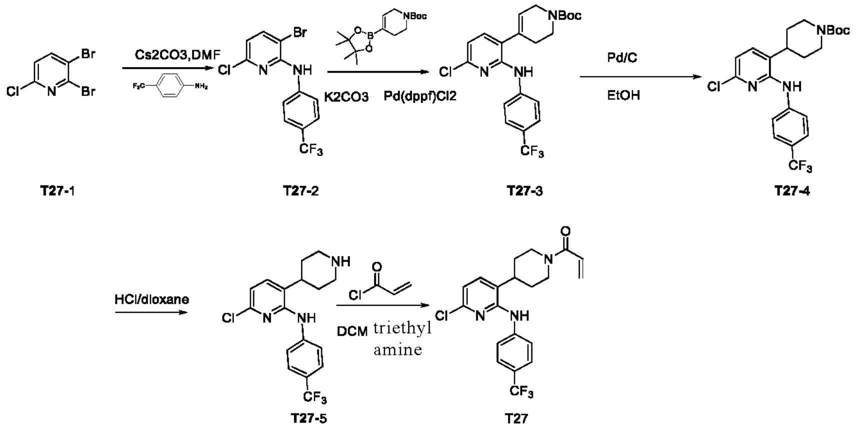


The experimental procedure is as follows:

(1) Synthesis of T27-2

T27-1 (5 g, 18.42 mmol, 1.0 eq), p-trifluoroaniline (3.58 g, 22.11 mmol, 1.2 eq), $CS_2CO_3$ (14 g, 43.2 mmol, 2.5 eq), and 50 ml of DMF were added sequentially to the reaction flask, and nitrogen was replaced three times, and the reaction

solution was put into an oil bath, and raised to 100 °C for 16 h.

The reaction was completed, cooled to room temperature, filtered with diatomaceous earth, 100 mL of water was added, extracted with ethyl acetate, the organic layers were washed with saturated brine, dried over anhydrous sodium sulfate, concentrated to obtain the crude product which was purified by column chromatography using pure PE to obtain 2.2 g T27-2 (white solid).

(2) Synthesis of T27-3

T27-2 (900m g, 2.47umol, 1.0 eq), pinacol borate (916.8mg, 2.97umol, 1.2 eqiv), potassium carbonate (682.4mg, 4.95umol, 2.0 eqiv), water/1,4-dioxane (1/10) (1 mL/10 mL), and Pd(dppf)$Cl_2$ (181mg, 0.247mol, 10% eq) were added sequentially in a 100 mL single-necked vial, nitrogen was replaced three times, and the reaction

| Compound No. | Compound Structure | Compound Characterization |
|---|---|---|
| T24 | 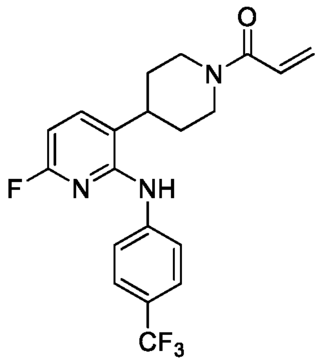 | LC-MS[M+1]: 394.1 |
| T25 | 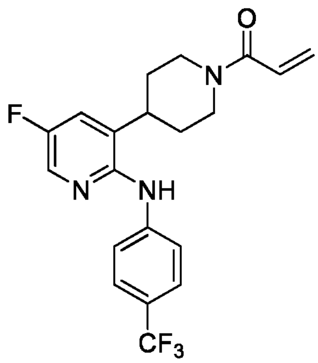 | LC-MS[M+1]: 394.1 |
| T26 | 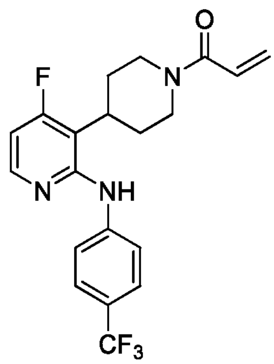 | LC-MS[M+1]: 394.1 |

(continued)

| Compound No. | Compound Structure | Compound Characterization |
|---|---|---|
| T28 | 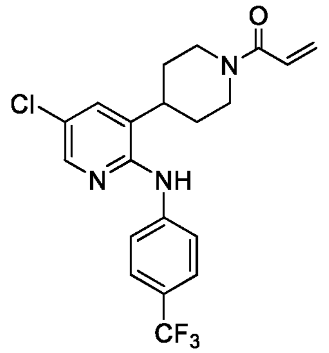 | LC-MS[M+1]: 410.1 |
| T29 | 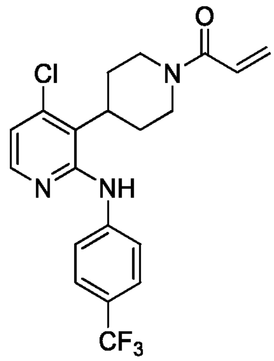 | LC-MS[M+1]: 410.1 |
| T45 | 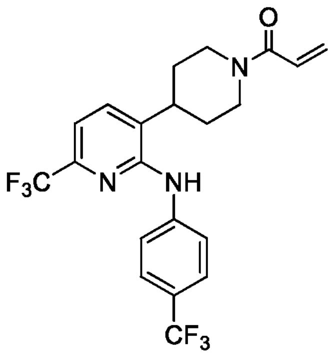 | $^1$H NMR (400 MHz, Chloroform-d) δ 7.73 (d, *J* = 8.5 Hz, 2H), 7.58 (dd, *J* = 15.1, 8.1 Hz, 3H), 7.23 (s, 1H), 6.68 - 6.57 (m, 2H), 6.34 (dd, *J* = 16.8, 1.9 Hz, 1H), 5.75 (dd, *J* = 10.5, 1.9 Hz, 1H), 4.94 (d, *J* = 13.5 Hz, 1H), 4.23 (d, *J* = 13.8 Hz, 1H), 3.28 (s, 1H), 2.82 (s, 2H), 2.03 (d, *J* = 13.4 Hz, 2H), 1.70 (s, 2H). |
| T47 | 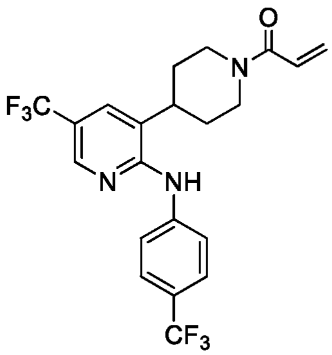 | $^1$H NMR (400 MHz, Chloroform-d) δ 8.41 (d, *J* = 2.5 Hz, 1H), 7.67 (d, *J* = 8.5 Hz, 2H), 7.64 - 7.56 (m, 3H), 6.68 - 6.57 (m, 2H), 6.33 (dd, *J* = 16.8, 1.9 Hz, 1H), 5.75 (dd, *J* = 10.5, 1.9 Hz, 1H), 4.93 (d, *J* = 12.9 Hz, 1H), 4.23 (d, *J* = 13.5 Hz, 1H), 3.27 (s, 1H), 2.81 (s, 2H), 2.05 (d, *J* = 13.4 Hz, 2H), 1.73 (td, *J* = 12.4, 3.8 Hz, 2H). |

solution was put into an oil bath, raised to 105 °C for 16 h, after the reaction was completed, cooled to room temperature, diluted with EA, filtered with diatomaceous earth. water 10 mL was added, the reaction solution was extracted with ethyl acetate (15 ml * 3), the organic layers were combined and washed with saturated saline (20 mL), dried over anhydrous sodium sulfate, and the filtrate was suction filtered, spin-dried and passed through the column to obtain 1.2 g of T27-3 (light yellow solid).

(3) Synthesis of T27-4

T27-3 (940m g ) was dissolved in ethanol (15mL), triphenylphosphine rhodium chloride ( 160 mg, 10%) was added, and reacted under high-pressure hydrogen for 24h, the raw material was not reacted completely, and T27-4 370 mg (white solid) was obtained by passing through a column.

(4)Synthesis of compound T27-5

Compound T27-4 (350 mg) was added into DCM (5 ml), then HCl/dioxane (15 mL, 4M) was added, and then the reaction was carried out at room temperature overnight. 300m g of white solid T27-5 was obtained by direct spin-dry.

(5) Synthesis of compound T27

Compound T27-5 ( 300 mg,0.85 umol, 1.0 eq), triethylamine (258 mg,2.55 umol,3.0 eq), DCM (3 ml) were added to the reaction tube, acryloyl chloride (84.1 mg,0.93 umol, 1.1 eq) in DCM solution (1 ml) was added dropwise at 0 °C, and stirred overnight at room temperature. The solution was washed with 0.1N HCl, then EA was added for extraction and then separated and purified by Preparative Thin-Layer Chromatography to obtain 180 mg of T27 (white solid).

$^1H$ NMR (400 MHz, Chloroform-d) δ 7.64 - 7.55 (m, 4H), 7.38 (d, *J* = 7.9 Hz, 1H), 6.90 (d, *J* = 8.0 Hz, 1H), 6.62 (dd, *J* = 16.8, 10.5 Hz, 1H), 6.46 (s, 1H), 6.33 (dd, *J* = 16.8, 2.0 Hz, 1H), 5.74 (dd, *J* = 10.6, 1.9 Hz, 1H), 4.91 (d, *J* = 13.3 Hz, 1H), 4.20 (d, *J* = 13.7 Hz, 1H), 3.22 (d, *J* = 13.8 Hz, 1H), 2.84 - 2.70 (m, 2H), 1.98 (d, *J* = 13.3 Hz, 2H), 1.63 (d, *J* = 13.1 Hz, 2H).

Referring to the synthesis method in Example 5, the following compounds were synthesized:

**Example 6**

Compound synthesized in the present invention:

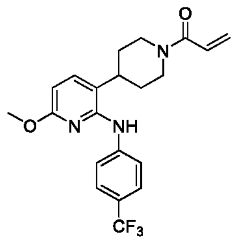

T33

The synthesis route is as follows:

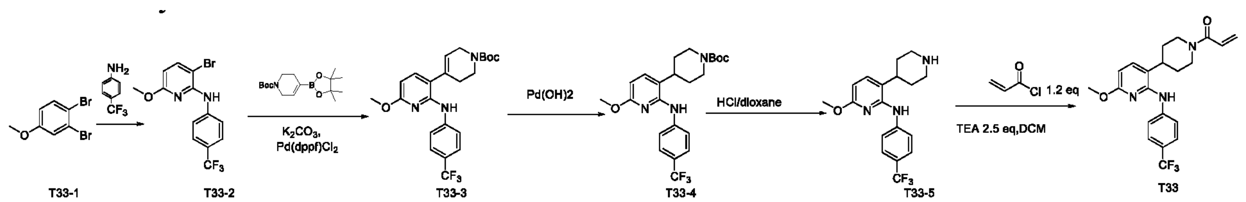


The experimental procedure is as follows:

(1) Synthesis of T33-2

T33-1 (5 g, 18.8 mmol, 1.0 eq), p-trifluoroaniline (3.03 g, 18.8 mmol, 1 eq), $CS_2CO_3$ (12.25 mg, 37.6 mmol, 2 eq), Xanthpos (1.09 g, 1.88 mmol, 10% eq), $Pd(OAc)_2$ (211.06 mg, 0.94 mmol, 5% eqiv ), and 100 ml of ultra-dry 1,4 dioxane were added in a reaction flask in turn, and nitrogen was replaced three times, and the reaction solution was put into an oil bath, and raised to 105 °C for 16 h. After the reaction was completed, cooled to room temperature, diluted with EA, filtered with diatomaceous earth, water 200 mL was added, extracted with ethyl acetate (100 ml * 3), the organic layers were combined and washed with saturated saline (200 mL), dried over anhydrous sodium sulfate, the filtrate was suction filtered, and spin-dried, and 4 g of T33-2 was obtained by column chromatography.

(2) Synthesis of T33-3

T33-2 (2.0 g, 5.7 m mol, 1.0 eq), N-Boc-1,2,5,6-tetrahydropyridine-4-boronic acid pinacol ester (2.13 g, 6.91 mmol, 1.2 eq), potassium carbonate (1.59 g, 11.5 mmol, 2.0 eq), water/1,4-dioxane (1/10 ) (2 mL/20 mL), and Pd(dppf) $Cl_2$ (210 mg, 0.287 mmol, 5% eq) were added sequentially in a 100 mL single-necked vial, nitrogen was replaced three times, and then the reaction solution was put into the oil bath, raised to 105 °C for 16 h. After the reaction was completed, cooled to room temperature, diluted with EA, filtered with diatomaceous earth. 50 mL of water was added, extracted with ethyl acetate (50 ml*3), the organic layers were combined and washed with saturated saline (50 mL), dried over anhydrous sodium sulfate, the filtrate was suction filtered and spin-dried, and 1.8 g of T33-3 was obtained by column chromatography.

(3) Synthesis of T33-4

T33-3 (1.8g, 4mmol, 1.0eq ) was dissolved in ethanol (20 mL), $Pd(OH)_2$ ( 180m g, 10%) was added, and the reaction was carried out for 20 hours at room temperature under hydrogen atmosphere in a 50 ml single-necked flask until the raw material was reacted completely, filtered and concentrated, and 1.5g product compound T33-4 was obtained by column chromatography.

(4) Synthesis of T33-5

T33-4 (1.5 mg) was added into DCM (20 ml), then HCl/dioxane (20 mL, 4M) was added, and then the reaction was carried out at room temperature overnight. 1.8g of T33-5 was obtained by direct spin-dry.

(5) Synthesis of compound T33

T33-5 ( 520 mg,1.48 mmol, 1.0 eq), triethylamine (448 mg,4.44 mmol,3.0 eq), DMF (5 ml) were added to the reaction tube, and then chloropropionyl chloride (146.6 mg,1.62 mmol, 1.1 eq) in DMF solution (2 ml) was added dropwise in an ice bath at 0 °C, and stirred overnight at room temperature. The reaction solution was extracted with water and EA, and then separated and purified by column chromatography to obtain 140 mg of T33 (white solid).

$^1H$ NMR (400 MHz, Chloroform-d) δ 7.71 - 7.47 (m, 4H), 7.36 (d, *J* = 8.3 Hz, 1H), 6.62 (dd, *J* = 16.8, 10.6 Hz, 1H), 6.40 - 6.25 (m, 3H), 5.72 (dd, *J* = 10.6, 1.9 Hz, 1H), 4.88 (d, *J* = 13.4 Hz, 1H), 4.17 (d, *J* = 13.7 Hz, 1H), 3.88 (s, 3H), 3.22 (t, *J* = 13.2 Hz, 1H), 2.87 - 2.60 (m, 2H), 1.95 (d, *J* = 13.4 Hz, 2H).

Referring to the synthesis method in Example 6, the following compounds were synthesized:

| Compound No. | Compound Structure | Compound Characterization |
|---|---|---|
| T34 | 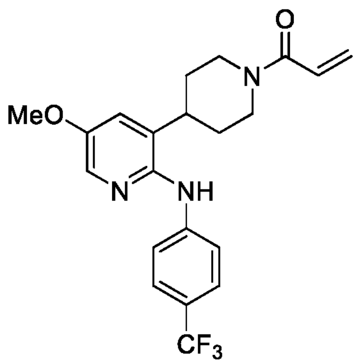 | LC-MS[M+1]: 406.1 |

(continued)

| Compound No. | Compound Structure | Compound Characterization |
|---|---|---|
| T35 | 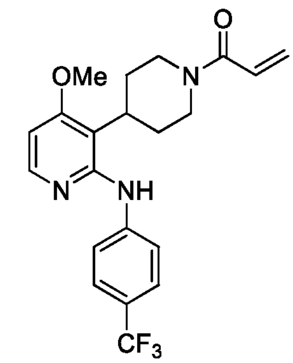 | LC-MS[M+1]: 406.1 |
| T45 | 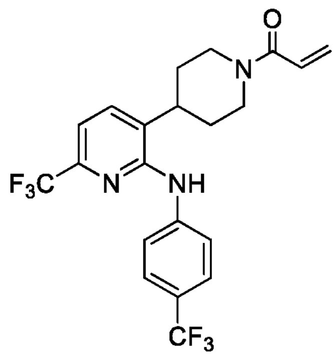 | LC-MS[M+1]: 444.1 |

**Example 7**

Compound synthesized in the present invention:

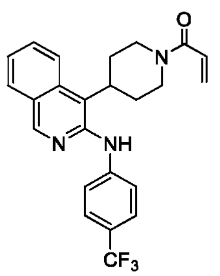

T37

The synthesis route is as follows:

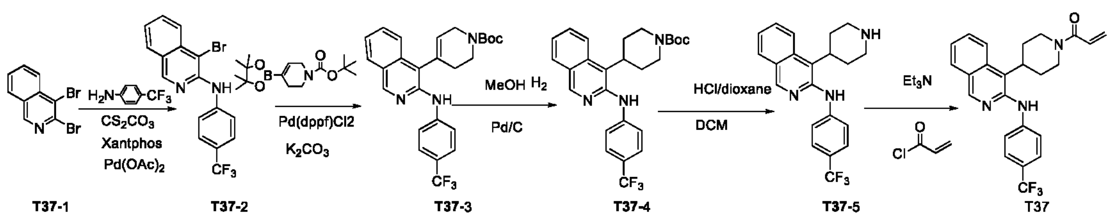


The experimental procedure is as follows:

(1) Synthesis of T37-2

T37-1 (3.6 g, 12.54 mmol, 1.0 eq), p-trifluoroaniline (2.02 g, 12.54 mmol, 1 eq), $CS_2CO_3$ (8.17 g, 25.08 mmol, 2

eq), Xanthpos (727.7 mg, 1.25 mmol, 10% eq), $Pd(OAc)_2$ (140.77 mg, 0.627 mmol,5% eqiv), 50 ml of ultra-dry 1,4 dioxane were added in microwave tube in turn, the reaction solution was reacted for 45 min at 150°C under microwave.

The reaction was completed, cooled to room temperature, 40 mL of water was added, extracted with ethyl acetate, the organic layers were washed with saturated brine, dried over anhydrous sodium sulfate, and concentrated to obtain the crude product which was purified by column chromatography with PE 100%, PE:EA=500:1 to obtain 1.3 g of T37-2.

(2) Synthesis of T37-3

T37-2 (1.3 g, 3.55m mol, 1.0 eq), pinacol borate (1.32 g, 4.26 mmol,1.2 eqiv), potassium carbonate (981.2m g, 7.1 mmol,2.0 eqiv), water/1,4-dioxane (1/10) (1.5 mL/15 mL), and $Pd(dppf)Cl_2$ (129.75 mg, 0.178 mmol, 5% eq) were added sequentially in a 100 mL single-necked vial, nitrogen was replaced three times, and the reaction solution was put into the oil bath, raised to 105 °C for 16 h. After the reaction was completed, and cooled to room temperature, diluted with EA, filtered with diatomaceous earth, water 10 mL was added, extracted with ethyl acetate (20 ml * 3), the organic layers were combined and washed with saturated saline (25 mL), dried over anhydrous sodium sulfate, and the filtrate was suction filtered, spin-dried and treated by a column to obtain 1.4 g of T37-3 (light yellow solid).

(3) Synthesis of T37-4

T37-3 (800 mg ) was dissolved in ethanol (10 mL), Pd/C ( 80 mg, 10%) was added, the reaction was carried out in a hydrogenation kettle under a 1.0 mpa hydrogen environment at 60 °C for 16h until the disappearance of raw materials, the final product was precipitated in the hydrogenation, filtered to obtain the product T37-4 350 mg (white solid)

(4) the synthesis of compound T37-5

Compound T37-4 (350 mg) was added to dichloromethane (5 ml), then HCl/dioxane (20 mL, 4M) was added, and then the reaction was carried out at room temperature overnight. And then the reaction solution was directly spin-dried to obtain T37-5 300 mg solid.

(7) Synthesis of compound T37

Compound T37-5 ( 300 mg, 1.2umol, 1.0 eq), triethylamine (364.6 mg,3.61umol,3.0 eq), DCM (2 ml) were added to the reaction tube, then acryloyl chloride (119.5 mg, 1.32umol, 1.1 eq) in DCM solution (1 ml) was added dropwise at 0 °C, and stirred overnight at room temperature. The reaction solution was extracted with water and EA, and then separated and purified by Preparative Thin-Layer Chromatography to obtain 60 mg of T37 (white solid).

$^1H$ NMR (400 MHz, Chloroform-d) $\delta$ 7.61 (d, $J$ = 8.1 Hz, 2H), 7.36 - 7.29 (m, 2H), 7.24 - 7.10 (m, 4H), 6.51 (d, $J$ = 13.6 Hz, 1H), 6.22 (d, $J$ = 16.7 Hz, 1H), 5.66 (d, $J$ = 10.6 Hz, 1H), 4.76 - 4.58 (m, 2H), 4.43 (s, 1H), 4.00 (dd, $J$ = 37.0, 13.4 Hz, 1H), 3.69 (s, 1H), 2.96 (d, $J$ = 47.1 Hz, 2H), 2.49 (d, $J$ = 26.0 Hz, 2H), 1.88 (s, 2H), 1.56 (s, 2H).

**Example 8**

Compound synthesized in the present invention:

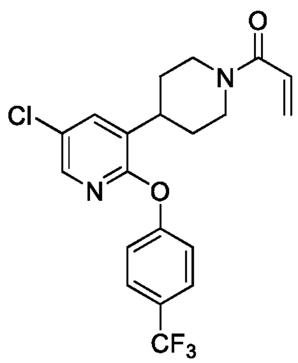

T40

The synthesis route is as follows:

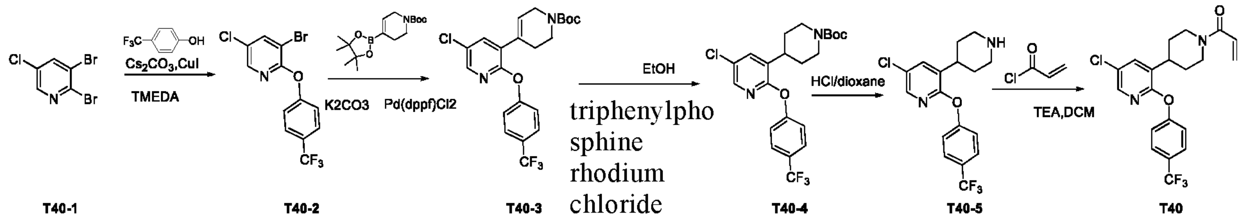


The experimental procedure is as follows:

(1) Synthesis of T40-2

T40-1 (2 g, 7.43 mmol, 1.0eq), p-trifluorophenol (1.45 g, 8.92 mmol, 1.2eq), $CS_2CO_3$ (4.83 g, 14.86 mmol, 2.0eq), CuI (140 mg, 0.74 mmol, 0.1eq), TMEDA (86 mg. 0.74mmol, 0.1eq), and DMSO (20ml) were added sequentially in a reaction flask, nitrogen was replaced three times, and the reaction was raised to 110 °C for 16 h.

The reaction was completed, cooled to room temperature, filtered with diatomaceous earth, 40 mL of water was added, extracted with ethyl acetate, the organic layers were washed with saturated brine, dried over anhydrous sodium sulfate, concentrated to obtain the crude product which was purified by column chromatography with pure PE to obtain 1.3 g of T40-2 (white solid).

(2) Synthesis of T40-3

T40-2 (1.3m g, 3.72umol, 1.0 eq), pinacol borate (1.38mg, 4.46mmol,1.2 eqiv), potassium carbonate (1.03mg, 7.44mmol,2.0 eqiv), water/1,4-dioxane (1/10) (1.5mL/15 mL), and $Pd(dppf)Cl_2$ ( 136 mg, 0.18 mmol, 5% eq) were added sequentially to a 50 mL single-necked vial, nitrogen was replaced three times, and the reaction was raised to 105 °C for 16 h. After the reaction was completed, the reaction was cooled to room temperature and then diluted with EA, filtered with diatomaceous earth, and 15 mL of water was added, extracted with ethyl acetate (15 ml * 3), the organic layers were combined and washed with saturated saline (20 mL), dried over anhydrous sodium sulfate. The filtrate was suction filtered, spin-dried and passed through the column to obtain 1.1 g of T40-3 (light yellow solid).

(3) Synthesis of T40-4

T40-3 (1.1m g ) was dissolved in ethanol (30mL), triphenylphosphine rhodium chloride ( 200 mg, 20%) was added, the reaction was carried out under high pressure hydrogen for 16h, the raw material was reacted completely, passed through the column to obtain T40-4 1g (white solid).

(4) Synthesis of compound T40-5

Compound T40-4 (1g) was added into DCM (10ml), then HCl/dioxane (20mL, 4M) was added, and then the reaction was carried out at room temperature overnight. The reaction solution was directly spin-dried to obtain 0.8 g of white solid T40-5.

(5) Synthesis of compound T40

Compound T40-5 ( 500 mg,1.4 μmol, 1.0 eq), triethylamine (424.2 mg,4.2 μmol,3.0 eq), DCM (5 ml) were added to the reaction tube, then acryloyl chloride (139.4 mg,1.54 μmol,1.1 eq) in DCM solution (2 ml) was added dropwise at 0 °C, and stirred at room temperature overnight. The solution was washed with 0.1 N HCl, then EA was added for extraction and then separated and purified by Preparative Thin-Layer Chromatography to obtain 210 mg of T40 (white solid).

$^1$H NMR (400 MHz, Chloroform-*d*) δ 7.97 (p, *J* = 2.5 Hz, 1H), 7.68 (dt, *J* = 8.3, 3.3 Hz, 2H), 7.57 - 7.50 (m, 1H), 7.20 (dd, *J* = 8.5, 3.7 Hz, 2H), 6.68 - 6.56 (m, 1H), 6.37 - 6.26 (m, 1H), 5.72 (dp, *J* = 10.5, 2.4 Hz, 1H), 4.88 (d, *J* = 13.4 Hz, 1H), 4.17 (d, *J* = 13.6 Hz, 1H), 3.22 (qd, *J* = 9.7, 8.9, 4.5 Hz, 2H), 2.76 (t, *J* = 13.4 Hz, 1H), 2.02 (d, *J* = 10.8 Hz, 2H), 1.76 - 1.62 (m, 2H).

Referring to the synthesis method in Example 8, the following compounds were synthesized:

| Compound No. | Compound Structure | Compound Characterization |
|---|---|---|
| T38 | 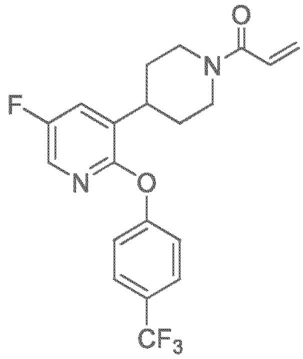 | LC-MS[M+1]: 395.1 |
| T39 | 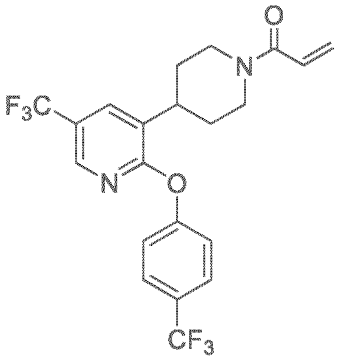 | LC-MS[M+1]: 445.1 |
| T44 | 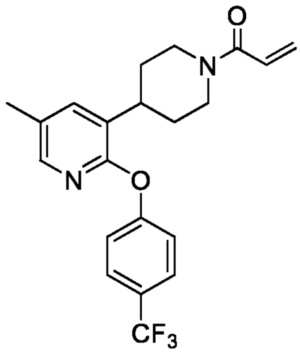 | LC-MS[M+1]: 391.1 |
| T73 | 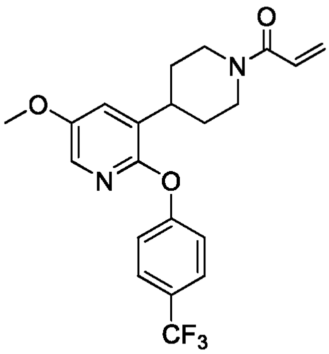 | LC-MS[M+1]: 407.1 |

(continued)

| Compound No. | Compound Structure | Compound Characterization |
|---|---|---|
| T74 | 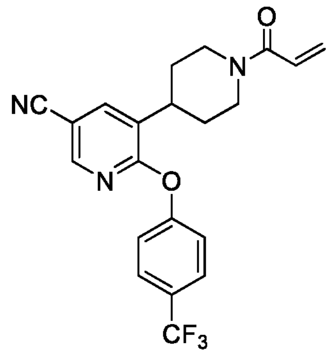 | LC-MS[M+1]: 402.1 |

### Example 9

Compound synthesized in the present invention:

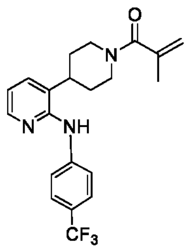

T48

The synthesis route is as follows:

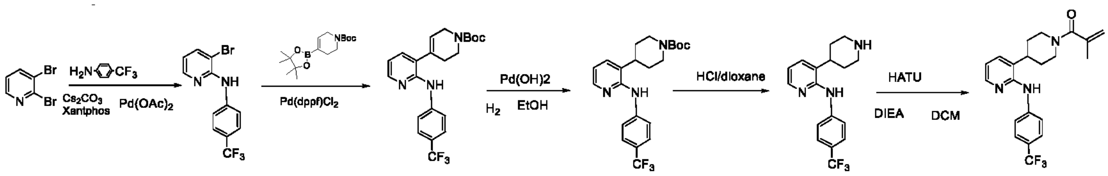


T48-1 T48-2 T48-3 T48-4 T48-5 T48

The experimental procedure is as follows:

(1) Synthesis of compound T48-2

Compound T48-1 (10 g, 42.21 mmol, 1.0 eqiv), p-trifluoroaniline (6.8 g, 42.21 mmol, 1.0 eqiv), $Cs_2CO_3$ (11.49 g, 84.43 mmol, 2.0 eqiv), $Pd(OAc)_2$ (473.86 mg, 2.11 mmol, 5% eqiv ), Xanthpos (2.44 g, 4.22 mmol, 10% eqiv), and ultra-dry dioxane (200 mL) were added to a 500 mL triple-necked vial, evacuated and replaced with nitrogen, and raised to 105 °C for 16 h.

The reaction was completed, cooled to room temperature, 200 mL of water was added, extracted with ethyl acetate, the organic layers were washed with saturated brine, dried over anhydrous sodium sulfate, and concentrated to obtain the crude product which was purified by column chromatography with PE 100%, PE:EA=500:1 to obtain 5.4 g of product T48-2 (white solid).

(2) Synthesis of compound T48-3

Compound T48-2 (1.2 g, 3.78 mol, 1.0 eq), pinacol borate (1.4 g, 4.54 mmol,1.2 eqiv), potassium carbonate (1.05 g, 7.57 mmol,2.0 eqiv), water/1,4-dioxane (1/10) (22 mL), and $Pd(dppf)Cl_2$ (135.64 mg, 0.189 mmol, 5% eq) were added sequentially to a 100 mL single-necked vial, and nitrogen was replaced three times, and the reaction solution was put into

the oil bath, raised to 105 °C for 16 h. After the reaction was completed, the reaction solution was cooled to room temperature, 20 mL of water was added, extracted with ethyl acetate (25 ml * 3), the organic layers were combined and washed with saturated brine (25 mL), dried over anhydrous sodium sulfate, and the filtrate was suction filtered, spin-dried and passed through the column to obtain 1.5 g of product T48-3 (light yellow viscous oil).

(3) Synthesis of compound T48-4

Compound T48-3 (1.5 g) was dissolved in ethanol (40 mL), Pd/$(OH)_2$ (150 mg, 10%) was added, and the reaction was carried out under hydrogen atmosphere for 16h until the disappearance of the raw material, and then the reaction solution was directly filtered and spin-dried to obtain 1.45 g of the crude product T48-4 (gray and viscous).

(4) Synthesis of compound T48-5

Compound T48-4 (1.45 g) was added to methanol (20 ml), then HCl/dioxane (40 mL, 4M) was added, then the reaction was carried out at room temperature overnight. The reaction solution was concentrated and then saturated sodium bicarbonate was added, extracted with ethyl acetate, then concentrated, and dichloromethane was used to remove solvent to obtain 1.14 g of gray solid T48-5.

(7) Synthesis of compound T48

Compound $\alpha$-methacrylic acid ( 96.55 mg, 0.78 mmol, 1eq), DIEA (201.79 mg,0.1.56 mmol, 2eq), and DCM (3 ml) were added to the reaction tube, and stirred at room temperature for 10 min, and then compound T48-5 ( 300 mg, 0.935 mmol, 1.2eq) was added, and the reaction was stirred at room temperature overnight. The organic layers were extracted with water and DCM, washed once with brine, dried over anhydrous sodium sulfate, filtered and purified by Preparative Thin-Layer Chromatography with purified EA to obtain 100 mg of white solid.

1H NMR (400 MHz, DMSO-d6) $\delta$ 8.45 (s, 1H), 8.05 (dd, J = 4.8, 1.8 Hz, 1H), 7.71 (d, J = 8.6 Hz, 2H), 7.59 (dd, J = 7.6, 1.8 Hz, 1H), 7.53 (d, J = 8.6 Hz, 2H), 6.89 (dd, J = 7.5, 4.8 Hz, 1H), 5.06 (dt, J = 53.9, 1.5 Hz, 2H), 4.48 (s, 1H), 3.97 (s, 1H), 3.22 (td, J = 10.3, 8.7, 6.0 Hz, 2H), 2.74 (s, 1H), 1.85 (d, J = 1.4 Hz, 3H), 1.79 (d, J = 12.3 Hz, 2H), 1.45 (d, J = 13.5 Hz, 2H).

Referring to the synthesis method in Example 9, the following compounds were synthesized:

| Compound No. | Compound Structure | Compound Characterization |
|---|---|---|
| T49 | 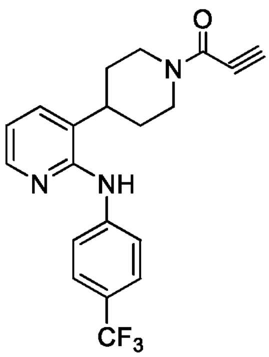 | $^1$H NMR (400 MHz, Chloroform-*d*) $\delta$ 8.19 (dd, *J* = 4.9, 1.8 Hz, 1H), 7.53 (q, *J* = 8.9 Hz, 4H), 7.45 (dd, *J* = 7.7, 1.8 Hz, 1H), 6.92 (dd, *J* = 7.6, 4.9 Hz, 1H), 6.35 (s, 1H), 4.83 - 4.74 (m, 1H), 4.64 - 4.55 (m, 1H), 3.26 (td, *J* = 13.2, 2.8 Hz, 1H), 3.15 (s, 1H), 2.87 - 2.74 (m, 2H), 2.08 - 1.94 (m, 2H), 1.71 (ddd, *J* = 25.0, 12.6, 4.3 Hz, 2H). |
| T50 | 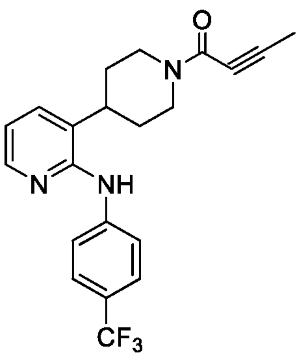 | LC-MS[M+1]: 388.4<br>$^1$H NMR (400 MHz, DMSO-$d_6$) $\delta$ 8.48 (s, 1H), 8.08 (d, *J* = 4.7 Hz, 1H), 7.74 (d, *J* = 8.4 Hz, 2H), 7.62 (s, 1H), 7.58 (t, *J* = 7.7 Hz, 3H), 6.92 (dd, *J* = 7.5, 4.8 Hz, 1H), 4.45 (dd, *J* = 27.4, 13.2 Hz, 2H), 3.26 (s, 2H), 2.80 (t, *J* = 12.5 Hz, 1H), 2.03 (s, 3H), 1.85 (t, *J* = 16.5 Hz, 2H), 1.62 - 1.51 (m, 1H), 1.43 (s, 1H). |

(continued)

| Compound No. | Compound Structure | Compound Characterization |
|---|---|---|
| T51 | 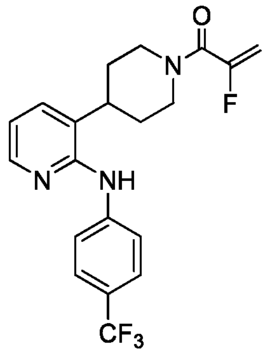 | LC-MS[M+1]: 394.4<br>$^1$H NMR (400 MHz, Chloroform-d) δ 8.19 (dd, *J* = 4.9, 1.8 Hz, 1H), 7.59 - 7.49 (m, 4H), 7.47 (dd, *J* = 7.7, 1.8 Hz, 1H), 6.93 (dd, *J* = 7.6, 4.8 Hz, 1H), 6.35 (s, 1H), 5.40 - 5.08 (m, 2H), 4.72 (s, 1H), 4.19 (s, 1H), 3.22 (s, 1H), 2.83 (tt, *J* = 11.9, 3.4 Hz, 2H), 2.05 - 1.96 (m, 2H), 1.71 (qd, *J* = 12.6, 4.2 Hz, 2H). |
| T52 | 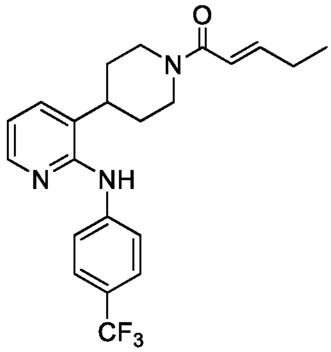 | LC-MS[M+1]: 404.4<br>$^1$H NMR (400 MHz, DMSO-$d_6$) δ 8.48 (s, 1H), 8.08 (dd, *J* = 4.8, 1.8 Hz, 1H), 7.75 (d, *J* = 8.6 Hz, 2H), 7.64 - 7.55 (m, 3H), 6.92 (dd, *J* = 7.6, 4.8 Hz, 1H), 6.72 (dt, *J* = 15.0, 6.5 Hz, 1H), 6.49 (dt, *J* = 14.9, 1.6 Hz, 1H), 4.62 (d, *J* = 13.1 Hz, 1H), 4.22 (d, *J* = 13.4 Hz, 1H), 3.22 (dt, *J* = 25.1, 12.1 Hz, 2H), 2.79 - 2.67 (m, 1H), 2.26 - 2.14 (m, 2H), 1.83 (d, *J* = 12.6 Hz, 2H), 1.47 (s, 2H), 1.02 (t, *J* = 7.4 Hz, 3H). |
| T53 | 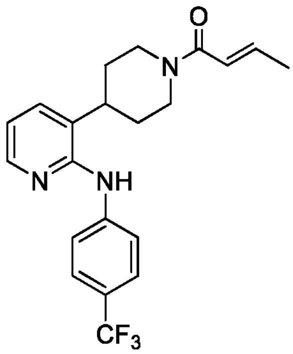 | LC-MS[M+1]: 389.2<br>$^1$H NMR (400 MHz, DMSO-$d_6$) δ 8.48 (s, 1H), 8.08 (dd, *J* = 4.8, 1.8 Hz, 1H), 7.75 (d, *J* = 8.5 Hz, 2H), 7.58 (td, *J* = 5.1, 4.7, 2.4 Hz, 3H), 6.92 (dd, *J* = 7.6, 4.8 Hz, 1H), 6.75 - 6.62 (m, 1H), 6.55 (dd, *J* = 15.0, 1.6 Hz, 1H), 4.61 (d, *J* = 12.9 Hz, 1H), 4.21 (d, *J* = 13.1 Hz, 1H), 3.27 - 3.16 (m, 2H), 2.74 (d, *J* = 12.8 Hz, 1H), 1.84 (dt, *J* = 8.1, 4.1 Hz, 5H), 1.46 (s, 2H). |

**Example 10**

Compound synthesized in the present invention:

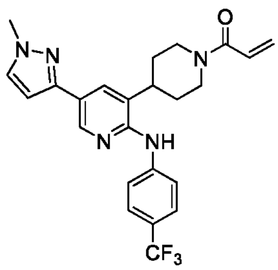

**T54**

The synthesis route is as follows:

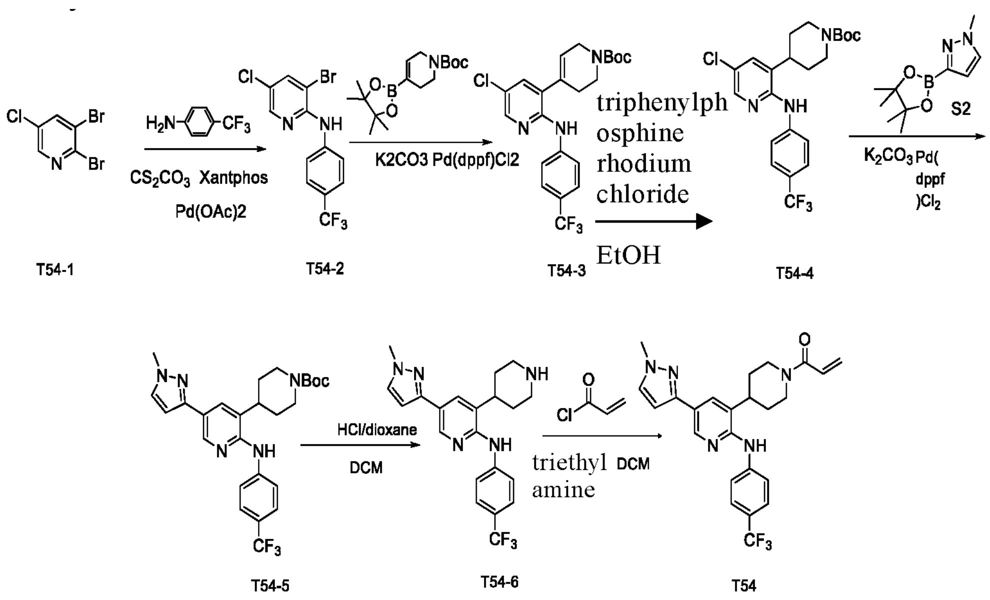


The experimental procedure is as follows:

(1) Synthesis of T54-2

T54-1 (3.6 g, 12.54 mmol, 1.0 eq), p-trifluoroaniline (2.02 g, 12.54 mmol, 1 eq), $CS_2CO_3$ (8.17 g, 25.08 mmol, 2 eq), Xanthpos (727.7 mg, 1.25 mmol, 10% eq), $Pd(OAc)_2$ (140.77 mg, 0.627 mmol,5% eqiv ), and 50 ml of ultra-dry 1,4 dioxane were added sequentially to the microwave tube, and reacted for 45 min at 150°C under microwave.

The reaction was completed, cooled to room temperature, 40 mL of water was added, extracted with ethyl acetate, the organic layers were washed with saturated brine, dried over anhydrous sodium sulfate, and concentrated to obtain the crude product which was purified by column chromatography with PE 100%, PE:EA=500:1 1 to obtain 1.3 T54-2.

(2) Synthesis of T54-3

T54-2 (1.3 g, 3.55 m mol, 1.0 eq), pinacol borate (1.32 g, 4.26 mmol,1.2 eqiv), potassium carbonate (981.2 m g, 7.1 mmol,2.0 eqiv), water/1,4-dioxane (1/10) (1.5 mL/15 mL), and Pd(dppf)$Cl_2$ (129.75 mg, 0.178 mmol, 5% eq) were added sequentially to a 100 mL single-necked vial, and nitrogen was replaced three times, and the reaction solution was put into the oil bath, raised to 105 °C for 16 h. After the reaction was completed, cooled to room temperature, and diluted with EA , filtered with diatomaceous earth, and water 10 mL was added, extracted with ethyl acetate (20 ml * 3), the organic layers were combined and washed with saturated brine (25 mL), dried over anhydrous sodium sulfate, and the filtrate was suction filtered, spin-dried and treated by a column to obtain 1.4 g of T54-3 (light yellow solid).

(3) Synthesis of T54-4

T54-3 (1.4 g ) was dissolved in ethanol (15 mL), triphenylphosphine rhodium chloride ( 210 mg, 20%) was added, and reacted under high-pressure hydrogen for 24 h. The raw material was not reacted completely, and 1.4 g of T54-4 (white solid) was obtained by passing through a column.

(4) Synthesis of T54-5

T54-4 (1.5g, 3.29mmol, 1.0eq), S2 (1.72g, 8.23mmol, 2.5eq), $NaHCO_3$ (1.24g, 14.81mmol, 4.5eq), $Pd(OAC)_2$ (73.7mg, 0.33mmol, 0.1eq) and Pcy3 (184.53mg. 0.66mmol, 0.2eq) were added to the reaction vial, 1,4 dioxane/EtOH/-water (7:2: 1) (11.9ml/3.4ml/1.7ml) as solvent were added, nitrogen was replaced three times, and the reaction solution was put into the oil bath, and raised to 105 °C for 12h. After the reaction was completed, cooled to room temperature, 20 mL of water was added, and extracted with ethyl acetate (25 ml*3), the organic layers were combined and washed with saturated brine (25 mL), dried over anhydrous sodium sulfate, and the filtrate was suction filtered, spin-dried and passed through a column to obtain 1.53 g of T54-5.

(5) Synthesis of compound T54-6

Compound T54-5 (1.53 g) was added to DCM (10 ml) followed by HCl/dioxane (25 mL, 4 M), and then the reaction solution was reacted overnight at room temperature. The reaction solution was directly spin-dried to obtain 1.6 g of white solid T54-6.

(6) Synthesis of compound T54

Compound T54-6 (1.6 mg,4 mmol, 1.0 eq), triethylamine (1.2 g,12 mmol,3.0 eq), and DCM (12 ml) were added to the reaction vial, then acryloyl chloride (397.3 mg, 4.4 mmol, 1.1 eq) in DCM solution (6 ml) was added dropwise at 0 °C, and stirred at room temperature overnight. The solution was washed with 0.1N HCl, extracted with EA, and then purified by column chromatography with petroleum ether and EA to obtain 452 mg of T54 (white solid).

$^1$H NMR (400 MHz, Chloroform-d) δ 8.32 (d, *J* = 2.2 Hz, 1H), 7.72 (s, 1H), 7.62 - 7.48 (m, 6H), 6.63 (dd, *J* = 16.8, 10.6 Hz, 1H), 6.46 - 6.26 (m, 2H), 5.74 (dd, *J* = 10.5, 1.9 Hz, 1H), 4.92 (d, *J* = 13.4 Hz, 1H), 4.24 - 4.16 (m, 1H), 3.96 (s, 3H), 3.24 (t, *J* = 12.8 Hz, 1H), 2.95 - 2.73 (m, 2H), 2.07 - 1.98 (m, 2H), 1.73 (s, 2H).

Referring to the synthesis method in Example 10 and Example 14, the following compounds were synthesized:

| Compound No. | Compound Structure | Compound Characterization |
|---|---|---|
| T55 | 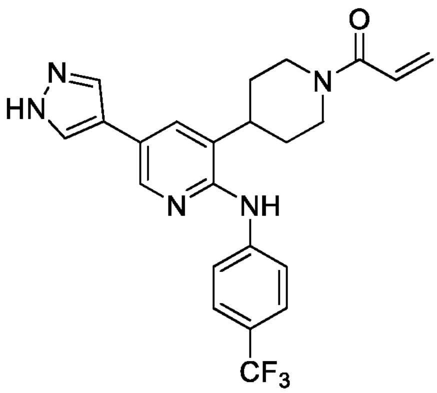 | $^1$H NMR (400 MHz, Chloroform-d) δ 8.37 (d, *J* = 2.1 Hz, 1H), 7.84 (s, 1H), 7.54 (q, *J* = 8.7 Hz, 5H), 6.64 (dd, *J* = 16.8, 10.5 Hz, 1H), 6.41 - 6.30 (m, 2H), 5.74 (d, *J* = 10.5 Hz, 1H), 4.93 (d, *J* = 13.4 Hz, 1H), 4.25 - 4.15 (m, 1H), 3.26 (s, 1H), 2.96 - 2.72 (m, 2H), 2.03 (d, *J* = 13.8 Hz, 4H). |
| T56 | 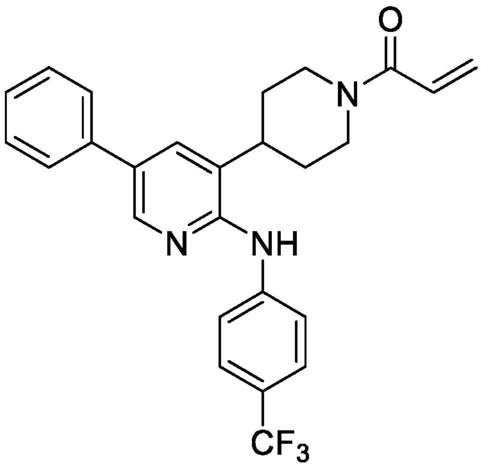 | $^1$H NMR (400 MHz, Chloroform-d) δ 8.43 (d, *J* = 2.3 Hz, 1H), 7.66 (d, *J* = 2.3 Hz, 1H), 7.58 (s, 4H), 7.54 (d, *J* = 7.1 Hz, 2H), 7.46 (t, *J* = 7.5 Hz, 2H), 7.37 (t, *J* = 7.3 Hz, 1H), 6.63 (dd, *J* = 16.8, 10.6 Hz, 1H), 6.42 (s, 1H), 6.33 (dd, *J* = 16.8, 1.9 Hz, 1H), 5.74 (dd, *J* = 10.6, 1.9 Hz, 1H), 4.93 (d, *J* = 13.3 Hz, 1H), 4.22 (d, *J* = 13.7 Hz, 1H), 3.27 (t, *J* = 12.8 Hz, 1H), 2.92 - 2.75 (m, 2H), 2.06 (d, *J* = 13.6 Hz, 2H). |

(continued)

| Compound No. | Compound Structure | Compound Characterization |
|---|---|---|
| T92 | 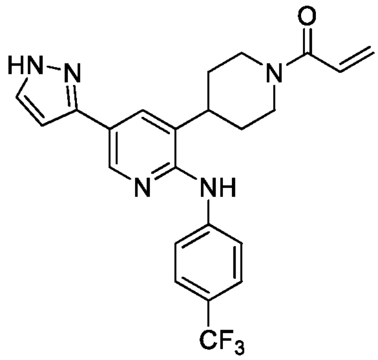 | LC-MS[M+1]: 442.1 |
| T96 | 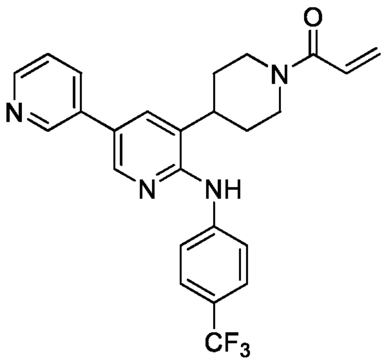 | LC-MS[M+1]: 453.1 |
| T97 | 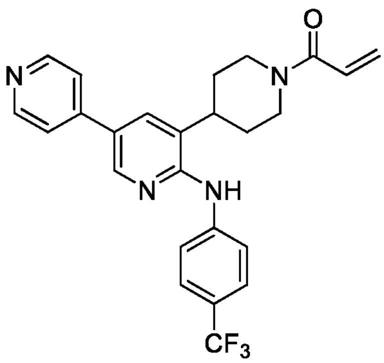 | LC-MS[M+1]: 453.1 |
| T98 | 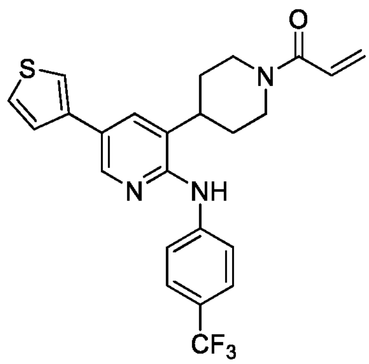 | LC-MS[M+1]: 458.1 |

(continued)

| Compound No. | Compound Structure | Compound Characterization |
|---|---|---|
| T99 | 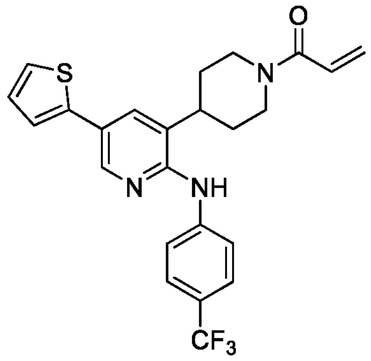 | LC-MS[M+1]: 458.1 |
| T100 | 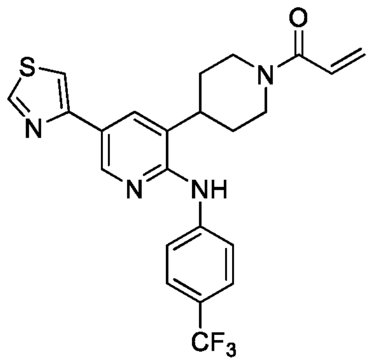 | LC-MS[M+1]: 459.1 |
| T101 | 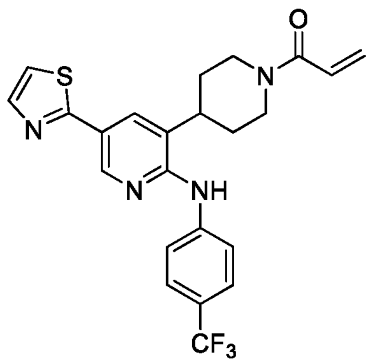 | LC-MS[M+1]: 459.1 |
| T102 | 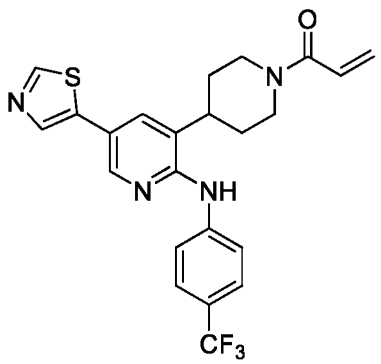 | LC-MS[M+1]: 459.1 |

(continued)

| Compound No. | Compound Structure | Compound Characterization |
|---|---|---|
| T103 | 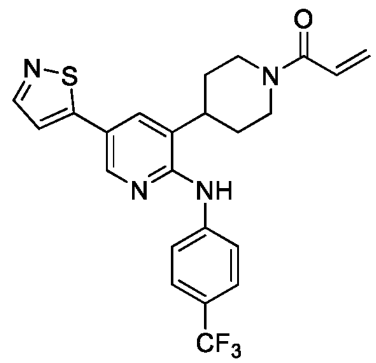 | LC-MS[M+1]: 459.1 |
| T104 | 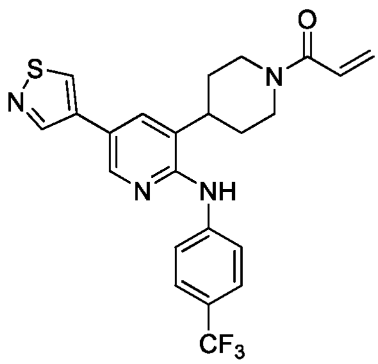 | LC-MS[M+1]: 459.1 |
| T105 | 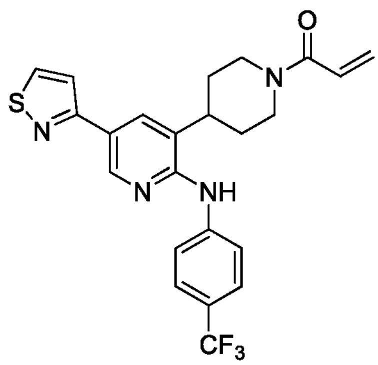 | LC-MS[M+1]: 459.1 |
| T106 | 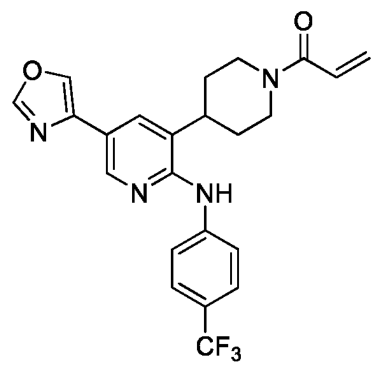 | LC-MS[M+1]: 443.2 |

(continued)

| Compound No. | Compound Structure | Compound Characterization |
|---|---|---|
| T107 | 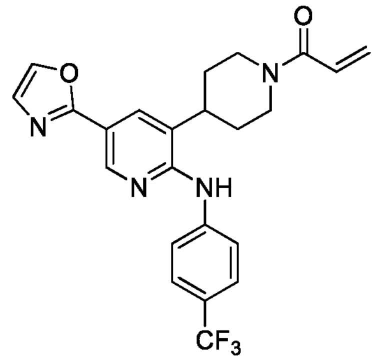 | LC-MS[M+1]: 443.2 |
| T108 | 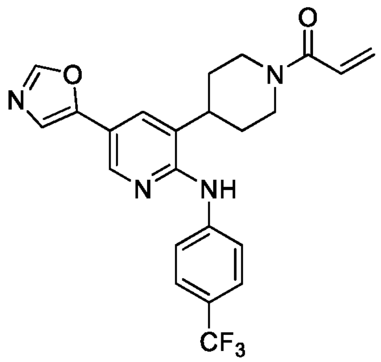 | LC-MS[M+1]: 443.2 |
| T110 | 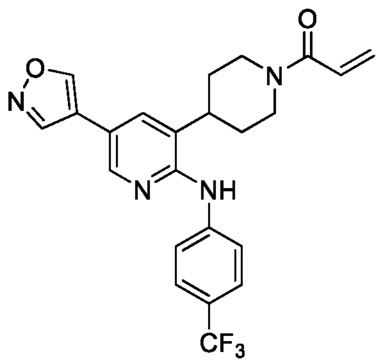 | LC-MS[M+1]: 443.2 |
| T111 | 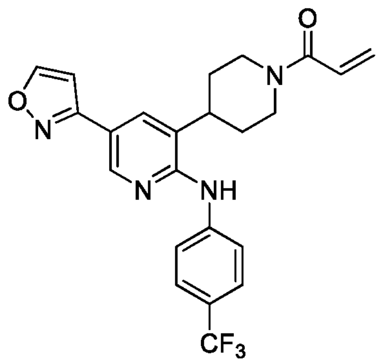 | LC-MS[M+1]: 443.2 |

(continued)

| Compound No. | Compound Structure | Compound Characterization |
|---|---|---|
| T114 | 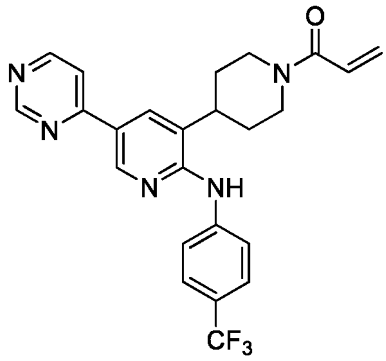 | LC-MS[M+1]: 454.2 |
| T115 | 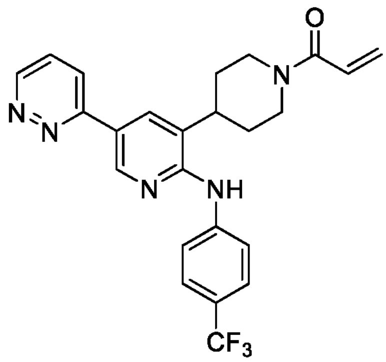 | LC-MS[M+1]: 454.2 |
| T116 | 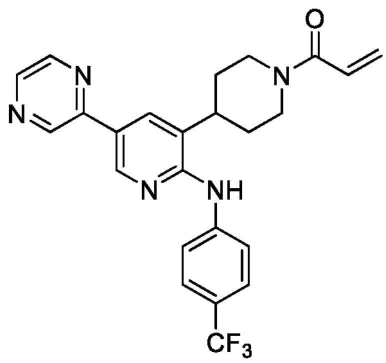 | LC-MS[M+1]: 454.2 |
| T95 | 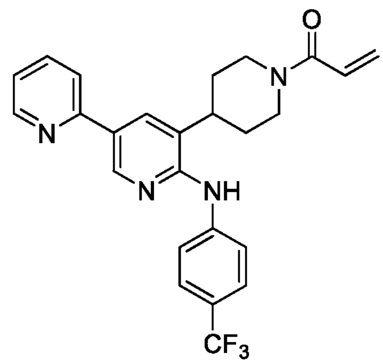 | LC-MS[M+1]: 453.2 |

**Example 11**

Compound synthesized in the present invention:

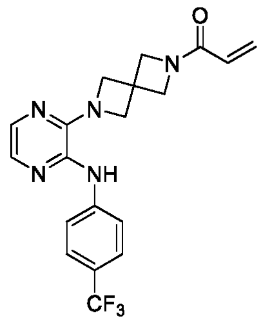

T57

The synthesis route is as follows:

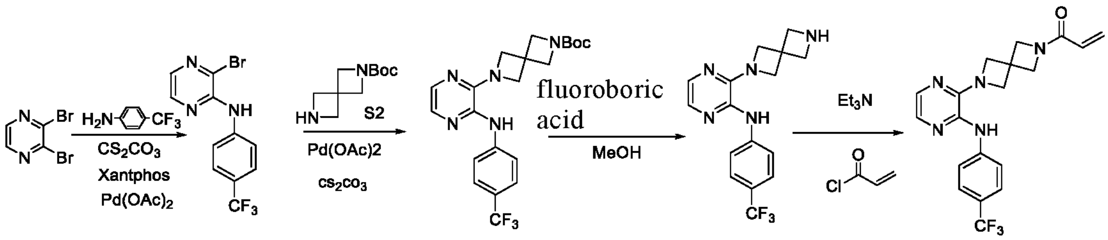


T57-1 T57-2 T57-3 T57-4 T57

The experimental procedure is as follows:

(1) Synthesis of compound T57-2

Compound T57-1 (5 g, 21.01 mmol, 1.0 eqiv), p-trifluoroaniline (4.73 g, 21.01 mmol, 1.0 eqiv), $Cs_2CO_3$ (13.66 g, 42.02 mmol, 2.0 eqiv), $Pd(OAc)_2$ (235.85 mg, 1.05 mmol, 5% eqiv ), Xanthpos (1.22 g, 2.11 mmol, 10% eqiv), and ultra-dry dioxane (50 mL) were added to a 100 mL three-necked vial, evacuated and replaced with nitrogen, and raised the temperature to 105 °C for 16 h.

The reaction was completed, cooled to room temperature, 200 mL of water was added, extracted with ethyl acetate, the organic layers were washed with saturated brine, dried over anhydrous sodium sulfate, and concentrated to obtain the crude product which was purified by column chromatography with PE 100%, PE:EA=500:1 to obtain 910 mg of product T57-2 (white solid).

(2) Synthesis of compound T57-3

Compounds T57-2 (800 g, 2.52 mmol, 1.0 eqiv), S2 (499 mg, 2.52 mmol, 1.0 eqiv), $Cs_2CO_3$ (1.6 g, 5.04 mmol, 2.0 eqiv), $Pd(OAc)_2$ (28.3 mg,0.126 mmol,5% eqiv ), Xanthpos (146m g, 0.252mmol, 10% eqiv), and ultra-dry dioxane (10mL) were added to a 100 mL three-necked vial, evacuated and replaced with nitrogen, raised the temperature to 105 °C for 16 h. After nitrogen was replaced three times, the reaction solution was put into an oil bath, raised the temperature to 105 °C for 16 h. After the reaction was completed, cooled to room temperature and then 20 mL of water was added, extracted with ethyl acetate (25 ml*3). The organic layers were combined and washed with saturated saline (25 mL), dried over anhydrous sodium sulfate, and the filtrate was suction filtered, spin-dried and passed through a column to obtain 721 mg of T57-3 (white solid).

(3) Synthesis of compound T57-4

Compound T57-3 (500 mg) was added to MeOH (5 ml), then fluoroboric acid (5 mL, 50%) was added, and then the reaction was carried out at room temperature for 3 h. The reaction solution was extracted with aqueous sodium bicarbonate and EA three times, washed once with saturated NaCl, dried over anhydrous $Na_2SO_4$, and filtered and spin-dried to obtain 457 mg of product T57-4.

(4) Synthesis of compound T57

Compound T57-4 (450 mg,1.34 mmol, 1.0 eq), triethylamine (406.02 mg,4.02 mmol,3.0 eq), DCM (4 ml) were

added to the reaction vial, then acryloyl chloride (133.7 mg, 1.47 mmol, 1.1 eq) in DCM solution (2 ml) was added dropwise at 0°C, and stirred overnight at room temperature. The solution was washed with 0.1N HCl, then EA was added for extraction, and then purified by column chromatography using petroleum ether and EA to obtain 135 mg of T57 (yellow solid).

1H NMR (400 MHz, DMSO-d6) δ 8.53 (s, 1H), 7.77 - 7.69 (m, 3H), 7.66 (d, J = 2.9 Hz, 1H), 7.58 (d, J = 8.6 Hz, 2H), 6.30 (dd, J = 17.0, 10.3 Hz, 1H), 6.09 (dd, J = 17.0, 2.3 Hz, 1H), 5.67 (dd, J = 10.3, 2.3 Hz, 1H), 4.39 (s, 2H), 4.23 (s, 4H), 4.11 (s, 2H).

**Example 12**

Compound synthesized in the present invention:

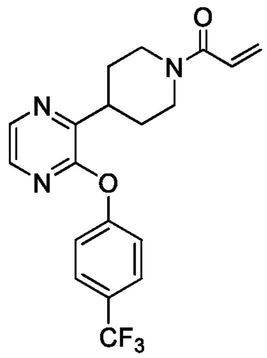

**T58**

The synthesis route is as follows:

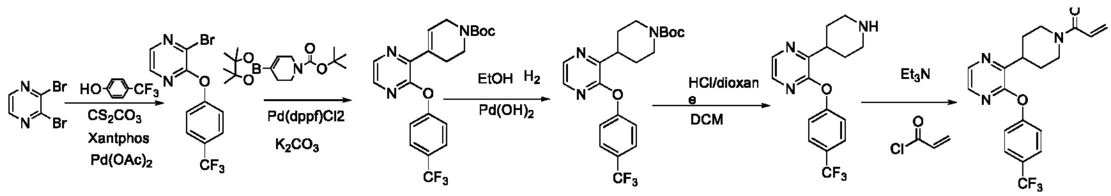


T58-1 T58-2 T58-3 T58-4 T58-5 T58

The experimental procedure is as follows:

(1) Synthesis of T58-2

T58-1 (5 g, 21.01 mmol, 1.0 eq), p-trifluorophenol (3.4 g, 21.01 mmol, 1 eq), $CS_2CO_3$ (10.24 g, 42.02 mmol, 2 eq), Xanthpos (1.22 mg, 2.1 mmol, 10% eq), Pd3$(dba)_2$ (962.3 mg, 1.05 mmol,5% eqiv ), and 50 ml of ultra-dry 1,4 dioxane were added sequentially to a reaction flask, nitrogen was replaced three times, and then the reaction solution was put into an oil bath, and raised to 105 °C for 16 h.

The reaction was completed, cooled to room temperature, filtered with diatomaceous earth, 100 mL of water was added, extracted with ethyl acetate, the organic layers were washed with saturated brine, dried over anhydrous sodium sulfate, concentrated to obtain the crude product which was purified by column chromatography with PE 100%, PE:EA=500:1 to obtain 4 g T58-2 (white solid).

(2) Synthesis of T58-3

T58-2 (2 g, 6.27m mol, 1.0 eq), pinacol borate (2.91 g, 9.41 mmol,1.5 eqiv), potassium carbonate (1.73 g, 12.54 mmol,2.0 eqiv), water/1,4-dioxane (1/10) (2 mL/20 mL), and Pd(dppf)$Cl_2$ (229.2 mg, 0.31 mmol, 5% eq) were added sequentially in a 100 mL single-necked vial, nitrogen was replaced three times, and the reaction solution was put into the oil bath, raised to 105 °C for 16 h. After the reaction was completed, cooled to room temperature, and diluted with EA, filtered with diatomaceous earth, and water 300 mL was added, extracted with ethyl acetate (30 ml * 3), the organic layers were combined and washed with saturated saline (30 mL), dried over anhydrous sodium sulfate. The filtrate was suction filtered, spin-dried and passed through the column to obtain 2.01 g of T58-3 (light yellow solid).

(3) Synthesis of T58-4

T58-3 (500m g ) was dissolved in ethanol (8mL), $Pd/(OH)_2$ (50mg, 10%) was added, and the reaction was carried out under hydrogen atmosphere for 5 hours until the disappearance of the raw material, and 490m g of crude T58-4 (white solid) was obtained by direct filtration and spin-dry.

(4) Synthesis of compound T58-5

Compound T58-4 (490 mg) was added to DCM (6 ml), then HCl/dioxane (6 mL, 4 M) was added, and then the reaction was carried out at room temperature overnight. The reaction solution was directly spin-dried to obtain 470m g of gray solid T58-5.

(7) Synthesis of compound T58

Compound T58-5 ( 470 mg,1.45 mmol, 1.0 eq), triethylamine (439.4 mg,4.35 umol,3.0 eq), and DCM (5 ml) were added to the reaction tube, then acryloyl chloride (144.4 mg, 1.6 mmol, 1.1 eq) in DCM solution (2 ml) was added dropwise at 0 °C, and stirred overnight at room temperature. The solution was washed with 0.1N HCl, then EA was added for extraction and then separated and purified by Preparative Thin-Layer Chromatography to obtain 210 mg of T58 (white solid).

1H NMR (400 MHz, Chloroform-d) δ 8.24 (d, J = 2.6 Hz, 1H), 7.94 (d, J = 2.6 Hz, 1H), 7.70 (d, J = 8.6 Hz, 2H), 7.24 (s, 2H), 6.63 (dd, J = 16.9, 10.6 Hz, 1H), 6.29 (dd, J = 16.9, 1.9 Hz, 1H), 5.70 (dd, J = 10.6, 1.9 Hz, 1H), 4.82 (d, J = 13.4 Hz, 1H), 4.18 (d, J = 13.8 Hz, 1H), 3.45 (td, J = 10.7, 5.4 Hz, 1H), 3.26 (t, J = 12.7 Hz, 1H), 2.85 (t, J = 12.9 Hz, 1H), 2.11 - 1.83 (m, 4H).

**Example 13**

Compound synthesized in the present invention:

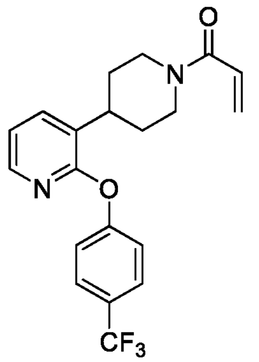

T59

The synthesis route is as follows:

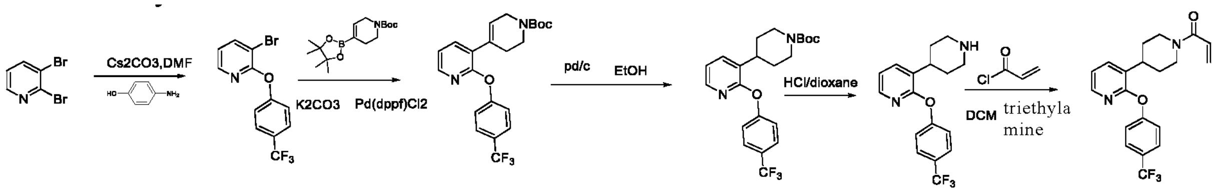


T59-1 T59-2 T59-3 T59-4 T59-5 T59

The experimental procedure is as follows:

(1) Synthesis of T59-2

T59-1 (2 g, 8.51 mmol, 1.0 eq), p-trifluorophenol (2.06 g, 12.77 mmol, 1.5 eq), $CS_2CO_3$ (6.9 g, 21.28 mmol, 2.5 eq), and 20 ml of DMF were added sequentially to the reaction flask, and nitrogen was replaced three times, and the

reaction solution was put into an oil bath, and raised to 80 °C for 16 h.

The reaction was completed, cooled to room temperature, filtered with diatomaceous earth, 100 mL of water was added, extracted with ethyl acetate, the organic layers were washed with saturated brine, dried over anhydrous sodium sulfate, concentrated to obtain the crude product which was purified by column chromatography using pure PE to obtain 1.34 g T59-2 (white solid).

(2) Synthesis of T59-3

T59-2 (1.34 g, 4.21 mmol, 1.0 eq), pinacol borate (1.56 g, 5.05 mmol,1.2 eqiv), potassium carbonate (1.16 g, 8.42 mol,2.0 eqiv), water/1,4-dioxane (1/10) (1.5 mL/15 mL), and Pd(dppf)$Cl_2$ (153.88mg,0.21mol, 5% eq) were added sequentially to a 100 mL single-necked vial, nitrogen was replaced three times, and the reaction solution was put into the oil bath, raised to 105 °C for 16 h. After the reaction was completed, cooled to room temperature, diluted with EA, filtered with diatomaceous earth, and water 10 mL was added, extracted with ethyl acetate (15 ml * 3), the organic layers were combined and washed with saturated saline (20 mL), dried over anhydrous sodium sulfate. The filtrate was suction filtered, spin-dried and passed through the column to obtain 1.73 g of T59-3 (light yellow solid).

(3) Synthesis of T59-4

T59-3 (1.73 g) was dissolved in ethanol (20 mL), palladium hydroxide (173 mg, 10%) was added, and the reaction was carried out under hydrogen at atmospheric pressure and room temperature for 12 h. After the raw materials were reacted completely, 1.3 mg of T59-4 (white solid) was obtained by passing through a column.

(4) the synthesis of compound T59-5

Compound T59-4 (400mg) was added into DCM (5ml), then HCl/dioxane (15mL, 4M) was added, and then the reaction was carried out at room temperature overnight. The reaction solution was directly spin-dried to obtain 358 mg of white solid T59-5.

(5) Synthesis of compound T59

Compound T59-5 ( 300 mg,0.932 mmol, 1.0 eq), triethylamine (282.3 mg,2.795 mol,3.0 eq), and DCM (3 ml) were added to the reaction tube, then acryloyl chloride (92.8 mg,1.03 mol,1.1 eq) in DCM solution (1 ml) was added dropwise at 0 °C, and stirred at room temperature overnight. The solution was washed with 0.1N HCl, and then extracted with EA, and separated and purified by Preparative Thin-Layer Chromatography to obtain 110 mg of T59 (white solid).

$^1$H NMR (400 MHz, Chloroform-d) δ 8.04 (dd, *J* = 4.8, 1.8 Hz, 1H), 7.66 (d, *J* = 8.6 Hz, 2H), 7.57 (dd, *J* = 7.5, 1.9 Hz, 1H), 7.20 (d, *J* = 8.4 Hz, 2H), 7.05 (dd, *J* = 7.5, 4.9 Hz, 1H), 6.63 (dd, *J* = 16.8, 10.6 Hz, 1H), 6.31 (dd, *J* = 16.8, 1.9 Hz, 1H), 5.71 (dd, *J* = 10.6, 1.9 Hz, 1H), 4.88 (d, *J*= 13.4 Hz, 1H), 4.15 (d, *J*= 13.7 Hz, 1H), 3.24 (tt, *J* = 12.2, 3.8 Hz, 2H), 2.76 (t, *J* = 12.8 Hz, 1H), 2.02 (t, *J* = 12.3 Hz, 2H), 1.72 (d, *J* = 12.1 Hz, 2H).

Referring to the synthesis method in Example 13, the following compounds were synthesized:

| Compound No. | Compound Structure | Compound Characterization |
|---|---|---|
| T77 | 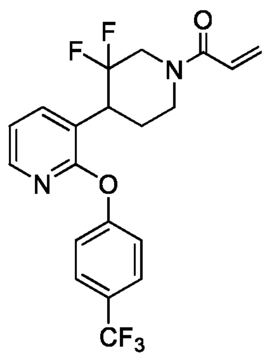 | LC-MS[M+1]: 413.1 |

(continued)

| Compound No. | Compound Structure | Compound Characterization |
|---|---|---|
| T80 | 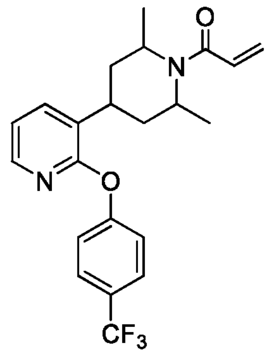 | LC-MS[M+1]: 405.2 |
| T81 | 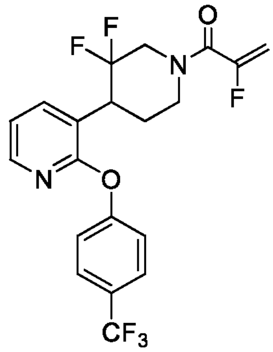 | LC-MS[M+1]: 431.1 |

**Example 14**

Compound synthesized in the present invention:

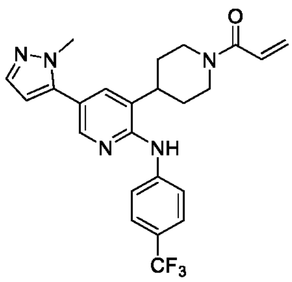

**T60**

The synthesis route is as follows:

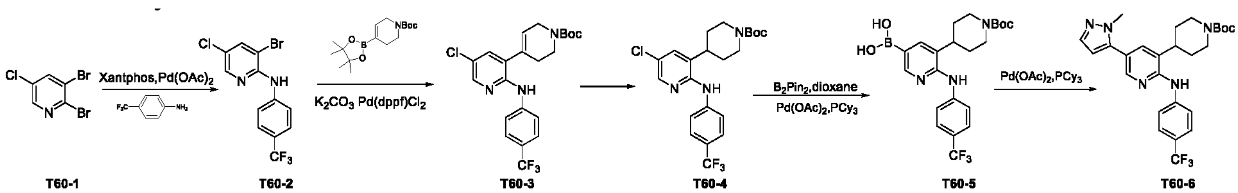


T60-1 T60-2 T60-3 T60-4 T60-5 T60-6

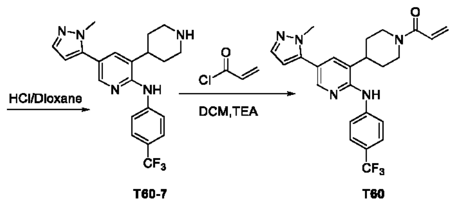


The experimental procedure is as follows:

(1) Synthesis of T60-2

T60-1 (25 g, 93.28 mmol, 1.0 eq), p-trifluoroaniline (15.02 g, 93.28 mmol, 1 eq), $CS_2CO_3$ (60.6 g, 186.56 mmol, 2 eq), Xanthpos (5.39 g, 9.328 mmol, 10% eq), $Pd(OAc)_2$ (1.044 g,4.66 mmol,5% eqiv), and 250 ml of ultra-dry 1,4 dioxane were added sequentially to a microwave tube, reacted at 150 °C under microwave for 1 h. The reaction was completed, cooled to room temperature, 40 mL of water was added, extracted with ethyl acetate, the organic layers were washed with saturated brine, dried over anhydrous sodium sulfate, concentrated to obtain the crude product, and purified by column chromatography to obtain 17.76 g of T60-2.

(2) Synthesis of T60-3

T60-2 (17.76 g, 45.08 mmol, 1.0 eq), N-Boc-1,2,5,6-tetrahydropyridine-4-boronic acid pinacol ester (22.6 g, 67.62 mmol, 1.5 eq), potassium carbonate (13.5 g, 90.16 mmol, 2.0 eq), water/1,4- dioxane (1/10) (17.8/178 mL), and $Pd(dppf)Cl_2$ (3.3 g, 4.508 mmol, 10% eq) were added sequentially to a 500 mL single-necked vial, and nitrogen was replaced three times, the reaction was carried out in oil bath at 105 °C for 16 h. After the reaction was completed, the reaction solution was cooled to room temperature, diluted with EA, filtered with diatomaceous earth, extracted with water and ethyl acetate, the organic layers were combined and washed with saturated brine, dried over anhydrous sodium sulfate, concentrated to obtain the crude product, and purified by column chromatography to obtain 15.5 g of T60-3.

(3) Synthesis of T60-4

T60-3 (15.5 g, 32.97 mmol, 1.0 eq ) was dissolved in ethanol (250 mL), triphenylphosphine rhodium oxide ( 2.3 g, 15%) was added, the reaction was carried out in hydrogenation kettle in a hydrogen gas environment of 1.0 mpa at 60 °C for 48 hours until the raw materials were completely reacted, filtered and concentrated, purified by column chromatography to obtain the product compound 12.37g of T60-4.

(4) Synthesis of compound T60-5

Compound T60-4 (3.7 g, 8.17 mmol, 1eq), bisboronic acid pinacol ester (5.2 g, 20.47 mmol, 2.5eq), tricyclohexylphosphine (457 mg, 1.634 mmol 0.2eq), palladium acetate (183 mg, 0.817 mmol 0.1eq), and cesium fluoride (5.59 g, 36.77 mmol 4.5eq) were added to ultra-dry 1,4 dioxane (37 ml), and reacted at 105 °C for 3.5 h. The lcms showed that the most are the product, and the reaction was completed, filtered with diatomaceous earth, and concentrated to be dried to obtain the crude product, 5.5 g of tan solid T60-5.

(5) Synthesis of compound T60-6

Compound T60-5 ( 253 mg, 0.543 mol, 2.5 eq), 5-bromo-1-methylpyrazole (32 mg, 0.217 mmol, 1.0 eq), sodium bicarbonate (82.1 mg, 0.977 mmol, 4.5 eq), tricyclohexylphosphine (12.2 mg, 0.0434 mmol 0.2 eq), and palladium acetate ( 4.9 mg, 0.0217 mmol 0.1eq) were added to 1,4 dioxane/ethanol/water=7:2:1 (2 mL), heated to 105 °C for 16 h. After the reaction was completed, filtered with diatomaceous earth, extracted with EA, and the organic phase was concentrated and purified by column chromatography to obtain 70 mg of T60-6.

(6) Synthesis of compound T60-7

Compound T60-6 (70 mg) was added to dichloromethane (1 ml) followed by HCl/dioxane (2 mL, 4M), and then reacted at room temperature overnight. And then the reaction solution was directly spin-dried to obtain the crude product, 76 mg of solid T60-7.

(7) Synthesis of compound T60

Compound T60-7 ( 76 mg, 0.25 mmol, 1.0 eq), triethylamine (76 mg,0.75 mmol,3.0 eq), and DCM (1 ml) were added to the reaction tube, then acryloyl chloride (25 mg, 0.275 mmol, 1.1 eq) in DCM solution (1 ml) was added dropwise at 0 °C, and stirred at room temperature overnight. The reaction solution was extracted with water and EA, then separated and purified by Preparative Thin-Layer Chromatography to obtain 5 mg of T60 (white solid).

$^1$H NMR (400 MHz, Chloroform-d) δ 8.24 (s, 1H), 7.61 (d, *J* = 14.6 Hz, 3H), 7.53 (s, 1H), 7.46 (s, 1H), 6.69 - 6.48 (m, 2H), 6.32 (d, *J* = 19.7 Hz, 2H), 5.74 (d, *J* = 10.6 Hz, 1H), 4.93 (s, 1H), 4.22 (s, 1H), 3.89 (s, 3H), 3.27 (s, 1H), 2.83 (s, 2H), 2.06 (d, *J* = 13.2 Hz, 2H), 1.77 - 1.66 (m, 2H).
LC-MS[M+1]: 456.5

Referring to the synthesis method in Example 14, the following compounds were synthesized:

| Compound No. | Compound Structure | Compound Characterization |
|---|---|---|
| T61 | 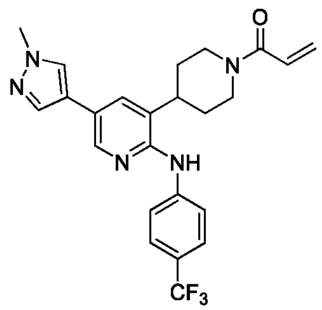 | 1H NMR (400 MHz, Chloroform-d) δ 8.32 (s, 1H), 7.72 (s, 1H), 7.54 (dt, J = 24.1, 11.5 Hz, 6H), 6.63 (t, J = 14.0 Hz, 1H), 6.36 (s, 2H), 5.74 (d, J = 10.6 Hz, 1H), 4.92 (s, 1H), 4.20 (s, 1H), 3.96 (s, 3H), 3.25 (s, 1H), 2.80 (dd, J = 28.1, 13.7 Hz, 2H), 2.02 (d, J = 13.0 Hz, 2H), 1.70 (s, 2H). |
| T62 | 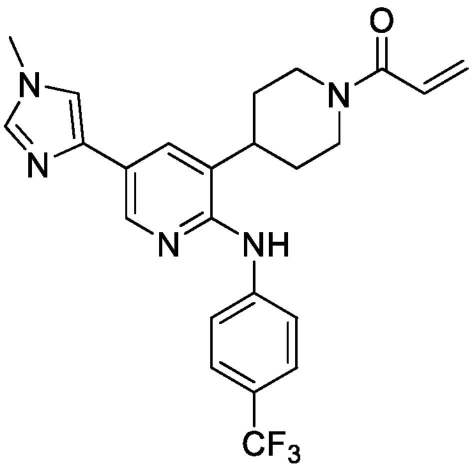 | $^1$H NMR (400 MHz, DMSO-$d_6$) δ 8.55 - 8.45 (m, 2H), 7.88 (d, *J* = 2.3 Hz, 1H), 7.73 (d, *J* = 8.5 Hz, 2H), 7.66 (d, *J* = 4.1 Hz, 2H), 7.57 (d, *J* = 8.5 Hz, 2H), 6.87 (dd, *J* = 16.7, 10.5 Hz, 1H), 6.13 (dd, *J* = 16.6, 2.5 Hz, 1H), 5.69 (dd, *J* = 10.4, 2.5 Hz, 1H), 4.64 (d, *J* = 12.9 Hz, 1H), 4.23 (d, *J* = 13.4 Hz, 1H), 3.68 (s, 3H), 3.30 - 3.19 (m, 2H), 2.78 (t, *J* = 12.6 Hz, 1H), 1.87 (d, *J* = 12.7 Hz, 2H), 1.61 - 1.46 (m, 2H). |

## Example 15

Compound synthesized in the present invention:

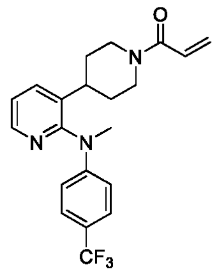

**T63**

The synthesis route is as follows:

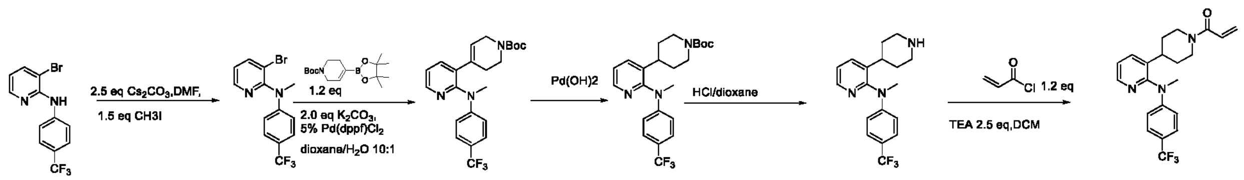


The experimental procedure is as follows:

(1) Synthesis of T63-2

T63-1 (1.0 g, 3.154 mmol, 1.0eq), $CS_2CO_3$ (2.56 g, 7.87 mmol, 2.5eq), CH3I, (660 mg, 4.68 mmol, 1.5eq ), and 50 ml of DMF were added sequentially in a microwave tube and the reaction was carried out for 4 h at room temperature.

After the reaction was completed, 10 mL of water was added, and extracted with 50 ml of ethyl acetate, the organic layers were washed with saturated brine, dried over anhydrous sodium sulfate, and concentrated to obtain crude product, 1.38 g of oil T63-2, containing about 70%.

(2) Synthesis of T63-3

T63-2 (1.38 g, 2.91m mol, 1.0 eq), N-Boc-1,2,5,6-tetrahydropyridine-4-boronic acid pinacol ester (1.12 g, 3.62 mmol, 1.2 eq), potassium carbonate (820m g, 5.8 mmol, 2.0 eqiv), water/1,4-dioxane (1/10) (1.0 mL/10 mL), and Pd(dppf) $Cl_2$ (110 mg, 0.15 mmol, 5% eq) were added sequentially to a 100 mL single-necked vial, nitrogen was replaced three times, and the reaction solution was put into the oil bath, raised to 105 °C for 16 h. After the reaction was completed, cooled to room temperature, and diluted with EA, filtered with diatomaceous earth and 10 mL of water was added, extracted with ethyl acetate (20 ml * 3), the organic layers were combined and washed with saturated brine (25 mL), dried over anhydrous sodium sulfate, and the filtrate was suction filtered, spin-dried and purified by column chromatography to obtain 1.2 g of T63-3 (oil).

(3) Synthesis of T63-4

T63-3 (650 mg, 1.50 mmol, 1.0 eq ) was dissolved in ethanol (10 mL), $Pd(OH)_2$ (65m g, 10%) was added, and the reaction was carried out in a 50 ml single-necked flask under hydrogen atmosphere at room temperature for 20 h until the raw material was reacted completely, and then filtered and concentrated to obtain the product compound, 620 g of T63-4.

(5) Synthesis of T63-5

T63-4 (620 mg) was added to DCM (20 ml), then HCl/dioxane (20 mL, 4M) was added, then the reaction was carried out at room temperature overnight. The reaction solution was directly spin-dried to obtain 520 of white solid T63-5.

(6) Synthesis of compound T63

T63-5 ( 520 mg, 1.55 mmol, 1.0 eq), triethylamine (471 mg,4.66 mmol,3.0 eq), and DCM (5 ml) were added to the reaction tube, and then acryloyl chloride (148 mg, 1.36 mmol, 1.05 eq) in DCM solution (5 ml) was added dropwise from an ice bath at 0 °C, and stirred at room temperature overnight. The reaction solution was extracted with water and EA, then

separated and purified by column chromatography to obtain 100 mg of T63 (white solid).

$^1$H NMR (400 MHz, Chloroform-*d*) δ 8.45 (dd, *J* = 4.7, 1.9 Hz, 1H), 7.66 (dd, *J* = 7.8, 1.9 Hz, 1H), 7.43 (dd, *J* = 7.9, 1.4 Hz, 2H), 7.31 - 7.23 (m, 2H), 6.68 - 6.50 (m, 3H), 6.29 (dd, *J* = 16.8, 1.9 Hz, 1H), 5.69 (dd, *J* = 10.5, 1.9 Hz, 1H), 4.79 (d, *J* = 13.4 Hz, 1H), 4.06 (d, *J* = 13.7 Hz, 1H), 3.35 (s, 3H), 3.06 - 2.85 (m, 2H), 2.55 (t, *J* = 12.9 Hz, 1H), 1.82 - 1.71 (m, 2H), 1.69 - 1.50 (m, 2H).

**Example 16**

Compound synthesized in the present invention:

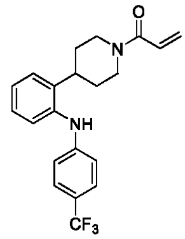

T64

The synthesis route is as follows:

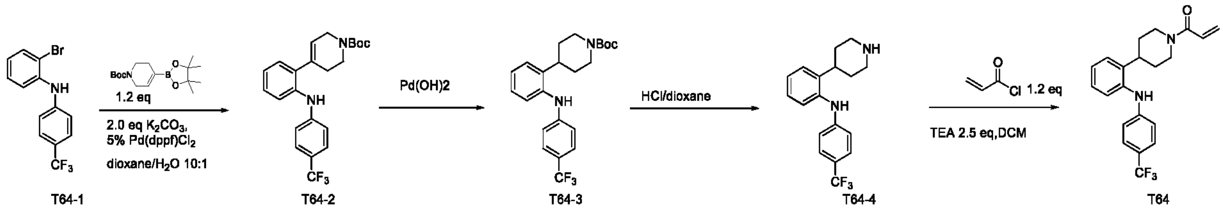


T64-1 T64-2 T64-3 T64-4 T64

The experimental procedure is as follows:

(1) Synthesis of T64-2

T64-1 (1.0 g, 3.17 m mol, 1.0 eq), N-Boc-1,2,5,6-tetrahydropyridine-4-boronic acid pinacol ester (1.17 g, 3.84 mmol, 1.2 eq), potassium carbonate (876 m g, 6.34 mmol, 2.0 eq), water/1,4-dioxane (1/ 10) (1.0 mL/10 mL), and Pd(dppf)$Cl_2$ (110 mg, 0.15 mmol, 5% eq) were added sequentially to a 100 mL single-necked vial, nitrogen was replaced three times, and the reaction solution was put into the oil bath, raised to 105 °C for 16 h. After the reaction was completed, cooled to room temperature, diluted with EA, filtered with diatomaceous earth, and 10 mL of water was added, extracted with ethyl acetate (20 ml * 3), the organic layers were combined and washed with saturated saline ( 25 mL), dried over anhydrous sodium sulfate, and the filtrate was suction filtered, spin-dried and purified by column chromatography to obtain 744 g of T64-2.

(2) Synthesis of T64-3

T64-2 (400mg, 0.95mmol, 1.0eq ) was dissolved in ethanol (10 mL), $Pd(OH)_2$ (40m g, 10%) was added, and the reaction was carried out for 20 hours at room temperature under hydrogen atmosphere in a 50 ml single-necked flask until the raw material was reacted completely, and then filtered and concentrated to obtain the product compound, 410mg T64-3.

(3) Synthesis of T64-4

T64-3 (410 mg) was added to DCM (20 ml), then HCl/dioxane (20 mL, 4M) was added, and then the reaction was carried out at room temperature overnight. The reaction solution was directly spin-dried to obtain T64-4 320 mg.

(4) Synthesis of compound T64

T64-4 ( 130, 0.402 mmol, 1.0 eq), triethylamine (123 mg,1.2 mmol,3.0 eq), and DCM (1 ml) were added to the

reaction tube, then chloropropanoyl chloride (51 mg, 0.40 mmol, 1.0 eq) in DCM solution (1 ml) was added dropwise at 0 °C, and stirred at room temperature overnight. The reaction solution was extracted with water and DCM, then separated and purified by column chromatography to obtain 50 mg of T64 (white solid).

$^1$H NMR (400 MHz, Chloroform-d) δ 7.35 (d, *J* = 8.4 Hz, 2H), 7.23 - 7.20 (m, 1H), 7.19 - 7.14 (m, 3H), 6.68 (d, *J* = 8.4 Hz, 2H), 6.53 (dd, *J* = 16.8, 10.6 Hz, 1H), 6.22 (dd, *J* = 16.9, 1.9 Hz, 1H), 5.62 (dd, *J* = 10.6, 2.0 Hz, 1H), 5.54 (s, 1H), 4.79 - 4.70 (m, 1H), 4.02 (d, *J* = 13.7 Hz, 1H), 3.10 - 2.92 (m, 2H), 2.63 - 2.52 (m, 1H), 1.75 (dd, *J* = 13.3, 3.4 Hz, 2H), 1.57 - 1.51 (m, 1H).

**Example 17**

Compound synthesized in the present invention:

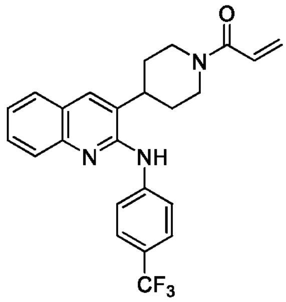

**T65**

The route synthesis is as follows:

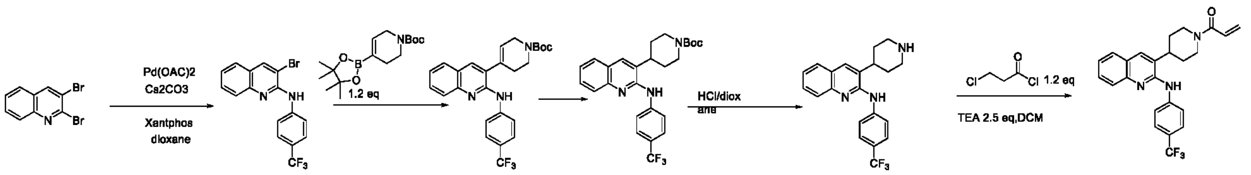


T65-1 T65-2 T65-3 T65-4 T65-5 T65

The experimental procedure is as follows:

(1) Synthesis of T65-2

T65-1 (1.5 g, 5.2 mmol, 1.0 eq), p-trifluoroaniline (855 g, 5.2 mmol, 1 eq), $CS_2CO_3$ (720 mg, 10.4 mmol, 2 eq), Xanthpos (300 g, 0.52 mmol, 10% eq), $Pd(OAc)_2$ (60 g, 0.26 mmol,5% eqiv ), and ultra-dry 1,4 dioxane 15 ml were added sequentially in a reaction flask, nitrogen was replaced three times, and the reaction solution was put into the oil bath, raised to 105 °C for 16 h. After the reaction was completed, cooled to room temperature, and diluted with EA, filtered with diatomaceous earth, water 20 mL was added, extracted with ethyl acetate (30 ml * 3), the organic layers were combined and washed with saturated saline (25 mL), dried over anhydrous sodium sulfate, the filtrate was suction filtered, spin-dried and purified by column chromatography to obtain 1.6 g of T65-2.

(2) Synthesis of T65-3

T65-2 (1.46 g, 3.97 m mol, 1.0 eq), N-Boc-1,2,5,6-tetrahydropyridine-4-boronic acid pinacol ester (1.47 g, 3.97 mmol, 1.2 eq), potassium carbonate (1.1 g, 7.94 mmol, 2.0 eq), water/1,4-dioxane (1/ 10) (1.4 mL/14 mL), and $Pd(dppf)Cl_2$ (146 mg, 0.2 mmol, 5% eq) were added sequentially in a 100 mL single-necked vial, nitrogen was replaced three times, and the reaction solution was put into the oil bath, raised to 105 °C for 16 h. After the reaction was completed, cooled to room temperature, and diluted with EA, filtered with diatomaceous earth, water 10 mL was added, extracted with ethyl acetate (20 ml * 3), the organic layers were combined and washed with saturated saline ( 25 mL), and dried over anhydrous sodium sulfate, and the filtrate was suction filtered, spin-dried and purified by column chromatography to obtain 1.3 g of T65-3.

(3) Synthesis of T65-4

T65-3 (1.2g, 2.55mmol, 1.0eq ) was dissolved in ethanol (10 mL), $Pd(OH)_2$ (120m g, 10%) was added, and the reaction was carried out at room temperature under hydrogen atmosphere in a 50 ml single-necked flask for 20 hours until the raw material was reacted completely, and then filtered and concentrated to obtain the product compound of 1.03g T65-4.

(4) Synthesis of T65-5

T65-4 (1.03 mg) was added to DCM (20 ml), then HCl/dioxane (20 mL, 4M) was added, and then the reaction was carried out at room temperature overnight. The reaction solution was directly spin-dried to obtain 800 mg of T65-5.

(5) Synthesis of compound T65

T65-5 ( 200 mg,0.539 mmol, 1.0 eq), triethylamine (163 mg, 1.617 mmol,3.0 eq), and DCM (1 ml) were added to the reaction tube, then chloropropanoyl chloride (72 mg,0.566 mmol,1.05 eq) in DCM solution (1 ml) was added dropwise at 0 °C, and stirred at room temperature overnight. The reaction solution was extracted with water and DCM, and separated and purified by column chromatography to obtain 40 mg of T65 (white solid).

$^1H$ NMR (400 MHz, Chloroform-d) δ 7.90 - 7.69 (m, 4H), 7.56 (td, *J* = 12.3, 10.4, 7.5 Hz, 4H), 7.29 (t, *J* = 7.5 Hz, 1H), 6.74 - 6.50 (m, 2H), 6.27 (dd, *J* = 16.8, 2.0 Hz, 1H), 5.68 (dd, *J* = 10.5, 2.0 Hz, 1H), 4.89 (d, *J* = 13.0 Hz, 1H), 4.18 (d, *J*= 13.3 Hz, 1H), 3.24 (s, 1H), 2.85 (d, *J* = 13.4 Hz, 2H), 2.06 (d, *J* = 13.4 Hz, 2H).

**Example 18**

Compound synthesized in the present invention:

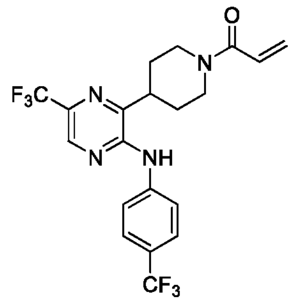

**T83**

The synthesis route is as follows:

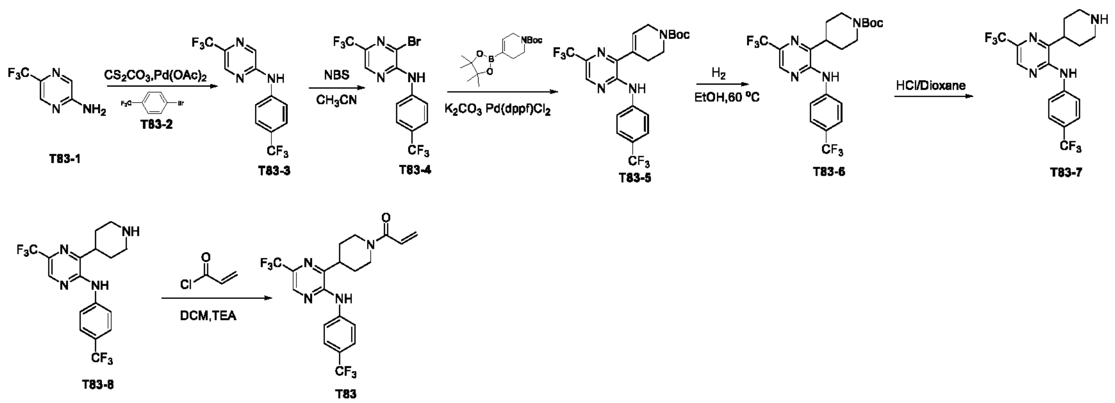


The experimental procedure is as follows:

Synthesis of T83-3

Compound T83-1 (4 g, 1.0 eq), T83-2 (8.28 g, 1.5 eq), $CS_2CO_3$ (16 g, 2.0 eq), and Xantphos (1.4 g, 0.10 eq) were dissolved in dioxane (40 mL), $N_2$ was replaced, and raised up to 80 °C, $Pd(OAc)_2$ (275.3 mg, 0.05 eq) was added, raised up to 105 °C, and stirred overnight. The sample was tested on the next day, LCMS result was correct, TLC: OK. Post-processing: extracted with water and EA, spin-dried, and samples were mixed and treated by column chromatography to obtain T83-3 (3.98 g).

Synthesis of T83-4

Compound T83-3 (3.98 g, 1.0 eq), NBS (4.6 g, 2.0 eq) was dissolved in $CH_3CN$ (40 mL), $N_2$ was replaced, and then raised to 90 °C, heated and stirred overnight, the sample was tested on the next day, LCMS result was correct, TLC: OK. Post-processing: extracted with water and EA, spin-dried and samples were mixed and treated by column chromatography to obtain T83-4 (972 mg), LCMS result: correct.

Synthesis of T83-5

T83-4 (1 g, 1.0 eq), bisboronic acid pinacol ester(1.24 g, 1.5 eq), $K_2CO_3$ (737 mg, 2.0 eq), and $Pd(dppf)Cl_2$ (196 mg, 0.1 eq) were added and dissolved in dioxane (10 mL) and $H_2O$ (1 mL), replaced with N2, and the reaction was heated to 105 °C overnight, and LCMS showed that the most was product. Post-processing: diluted with EA, filtered with diatomaceous earth, the filtrate was concentrated to be dried, spin-dried, and the samples were mixed, and then treated by column chromatography to obtain T83-5 (1.05 g).

Synthesis of T83-6

T83-5 (1.15g) was dissolved in ethanol (19mL), $Pd(OH)_2$ (115mg, 0.1eq) was added, replaced with $H_2$, about 1MPa at atmospheric pressure, and the reaction was warmed up to 60 °C overnight, and LCMS showed that the most was product, and the TLC result was OK. Post-processing: filtered with diatomaceous earth, the filtrate was concentrated to be dried, spin-dried, and the samples were mixed and then treated by column chromatography to obtain T83-6 ( 1.2g).

Synthesis of T83-7

T83-6 (1.2g) was dissolved in dichloromethane (6mL) and hydrochloric acid 1,4-dioxane (12ml), replaced with $N_2$, the reaction was carried out at room temperature for about 2h, the LCMS showed that the most was product, TLC: OK. Post-processing: spin-dried, ethanol was added and then spin-dried, repeated 3 times to obtain crude product T83-7 (1.2g), LCMS result was correct.

Synthesis of T83

T83-7 (300 mg, 1.0 eq) was dissolved in dichloromethane solution (2 mL), TEA (258 mg, 3.0 eq) was added under ice-bath conditions, and a mixture containing acryloyl chloride (84.4 mg, 1.1 eq) and dichloromethane (1 mL) was added dropwise slowly under the protection of $N_2$, and then the reaction was return ed to room temperature for about 4 h. The LCMS showed that the most was product. Post-processing: spin-dried, and the samples were mixed, T83 (144 mg) was obtained by column chromatography.

$^1$H NMR (400 MHz, Chloroform-d) δ 8.47 - 8.42 (m, 1H), 8.14 (d, *J* = 1.4 Hz, 1H), 7.62 - 7.52 (m, 3H), 6.77 (s, 1H), 6.60 (dd, *J* = 16.8, 10.5 Hz, 1H), 6.31 (dd, *J* = 16.8, 1.9 Hz, 1H), 5.72 (dd, *J* = 10.6, 1.9 Hz, 1H), 4.86 (d, *J* = 13.5 Hz, 1H), 4.15 (d, *J* = 14.0 Hz, 1H), 3.15 (t, *J* = 13.0 Hz, 1H), 3.06 (tt, *J* = 12.0, 3.6 Hz, 1H), 2.69 (t, *J* = 12.7 Hz, 1H), 1.88 (d, *J* = 13.1 Hz, 2H), 1.72 (t, *J* = 12.2 Hz, 2H).

**Bioactivity test experiment**

Experimental example 1 Cell anti-proliferation test

Proliferation inhibition assay of human pleural mesothelioma cells NCI-H226 by YAP-TEAD inhibitor compounds Experimental materials and equipment: Human pleural mesothelioma cells NCI-H226 were purchased from Nanjing Cobioer Biosciences CO.,LTD. RPMI-1640 medium (Bio-Channel), DMSO (dimethyl sulfoxide), CCK8 (WST-8) cell analysis kit (Beyotime), 0.25% EDTA-Tripsin (trypsin solution), 1xPBS (phosphate buffer, PH7.2), 96-well plate (Corning), fetal bovine serum (FBS), 10,000 U/mL penicillin-G/streptomycin, high-speed cryo-centrifuge (EPPENDORF 5810R),

enzyme-linked immunoassay (Tecan Spark).

Experiment preparation:

1. Cell spreading

A) Tumor cells were cultured in RPMI-1640 (containing 10% FBS and 100 U/mL penicillin-G/streptomycin) at 37°C, 5% $CO^2$ and saturated humidity until 80-90% density.
B) Removed the culture medium from 10 cm Petri dishes;
C) Rinsed the cells once with 10 ml 1xPBS;
D) Added 4 ml 0.25% EDTA-Tripsin into 37 °C, 5% $CO^2$ incubator for trypsin digestion for 5 minutes, transferred to 15 ml centrifuge tube, centrifuged at 200 g for 5 minutes, discarded the supernatant to get cell precipitate;
E) Resuspend with 4 ml DMEM medium, count and adjust to 10,000 cells/ml.
F) Added cell suspension to 96-well plate with 100 μL per well volume and incubate overnight at 37°C, 5% $CO^2$ incubator.

2. Compound treatment

Compound dilution

A) Preparation of gradient dilution solution of tested compounds: the test compounds were configured into 1 mM reservoir solution. Then 1.5 μl of the reservoir solution was dissolved in 1.5 ml of DMSO-free culture solution, and then a 3-fold serial gradient dilution was performed with 0.1% DMSO culture solution for a total of 9 concentrations, and the concentrations of the compounds after dilution were as follows:
333.33 nM, 111.11 nM, 37.03 nM, 12.35 nM, 4.15 nM, 1.37 nM, 0.46 nM, 0.15 nM
B) After thorough mixing, 100 μL of culture compound solution was taken separately to replace the culture solution in the cell culture plate, with 4 replicate wells for each concentration;
C) The cells were transferred to the incubator and incubated for 3 days.

3. CCK8(WST-8) cell analysis and detection

A) Removed the cell culture plate and added 10 μl of CCK-8 (WST-8) solution per well in the biosafety cabinet.
B) Placed the cell culture plate back into the incubator and continued incubation for 3 hours.
C) Determined the absorbance value by selecting 450 nm on a TECAN enzyme-linked immunoassay detector.

4. Data Analysis

The cell viability (% Cell Viability) was calculated using the following formula.

$$\text{Cell Viability (\%)} = [A(\text{dosing})-A(\text{blank})]/[\ A(0\ \text{dosing})-A(\text{blank})]\times 100$$

A (dosing): absorbance of wells with cells, CCK8 solution and drug solution
A (blank): absorbance of wells with medium and CCK8 solution but no cells
A (0 dosing): absorbance of wells with cells, CCK8 solution and no drug solution
Cell viability: Cell proliferation viability or cytotoxicity viability IC50 values were obtained by curve fitting with GraphPad Prism 8 software.

Experimental example 2 Nano Luciferase method to detect YAP-TEAD inhibitor activity

(1) Experimental materials and equipment: 293T cells were purchased from Nanjing Cobioer Biosciences CO.,LTD. DMEM medium (high sugar, phenol red free, Bio-Channel), DMSO (dimethyl sulfoxide), Lipo6000™ transfection reagent (Beyotime), pGL3B-8xGTiiC-nLuc-CMV-fLuc plasmid, 0.25% EDTA Tripsin (trypsin solution), 1xPBS (phosphate buffer, PH7.2), 96-well white cell culture plate (PerkinElmer), Fetal Bovine Serum (FBS), 10,000 U/mL penicillin-G/streptomycin, High-speed cryo-centrifuge (EPPENDORF 5810R), 37°C $CO^2$ incubator, Vi-cell® cell counter, Envision microplate reader (PerkinElmer).

Reagents

Reagents Catalog Number Concentration
Nano luciferase assay reagent MES pH 6.0 69892-25G 100 mM
CDTA CAS:125572-95-4 1 mM
Tergitol NP10-100ML 0.5% (v/v)
Mazu DF 204 A8311-50ML 0.05% (v/v)
KCl CAS:7447-40-7 150 mM
DTT Roche 10197777001 1 mM
thiourea T8656-50G35 mM
Furimazine 1374040-24-0 20μM Added before use

(2) Transient transfection of 293T cells

Resuscitated 293T cells were inoculated into 10cm petri dishes and placed in a 5% $CO^2$ incubator at a constant temperature of 37°C. To ensure transfection efficiency, cells in the logarithmic phase (cell density of about 50%-70%) should be used.

One day before transfection, cells in logarithmic phase were digested with Trypsin-EDTA, the reaction was terminated by adding medium, and the cell suspension was made by blowing and mixing with a pipette.

The cell concentration was determined using Vi-cell and diluted to a suspension of 5*10^5 cells per milliliter. Once the cell suspension was prepared, it was gently mixed and 10 ml of liquid was added to a 10 cm petri dish. This resulted in 5*10^6 cells per 10cm petri dish. The cells were incubated in a 5% $CO^2$ incubator at 37°C for 1 day.

Take two clean and sterile centrifuge tubes, added 750 μL of opti-MEM Medium without antibiotics and serum respectively, then added 15 μg of plasmid (pGL3B-8xGTiiC-nLuc-CMV-fLuc) to one of the tubes and gently blown with a pipette for mixing; the other tube was added with Lipo6000 transfection reagent and gently blown with a pipette for mixing. After standing at room temperature for 5 minutes, the culture medium containing DNA was gently added to the culture medium containing Lipo6000 transfection reagent, mixed by gently inverting the centrifuge tube, and stood at room temperature for 5 minutes.

The above mixture was added evenly dropwise to a 10-cm petri dish and replaced with fresh complete medium after 6 h of incubation.

(3) 96-well plate spreading

After 1 day of transfection, Trypsin EDTA was used to digest the cells, medium was added to terminate the reaction, and a cell suspension was made by blowing and mixing with a pipette.

Cell concentration was determined using Vi-cell, and diluted to a suspension of 20,000 cells per milliliter.

After the cell suspension was prepared, it was gently mixed and 100 μL was added to each well in a 96-well plate so that the density of cells to be measured was 2000 per well.

(4) Addition of compounds

The inoculated cell culture plates were incubated in an incubator, and the compounds in concentration gradients were added after about 24 hours.

The 10 mM compound reservoir solution was diluted to 50 μM with culture medium, and 50 μM compound solution was added sequentially to the third column of the deep-well plate, and 216 μL of culture medium containing 0.5% DMSO to the fourth to eleventh columns.

Gradient dilution: 100 μL of solution was pipetted from the third column and added to the fourth column, after mixing; another 100 μL of solution was pipetted from the fourth column and added to the fifth column, and the operation was repeated until the eleventh column.

Using a multichannel pipette, 25 μL of compound solution was pipetted from the deep-well plate and added to the 96-well culture plate, and each compound was repeated four times on the 96-well plate. A concentration gradient with a maximum concentration of 10,000 nM, 1:3.16, was eventually formed on the 96-well plate.

(5) Added Nano-luciferase detection reagent, read the number

**[0297]** The 96-well plate was incubated in a 5% $CO^2$ incubator at a constant temperature of 37°C for 48 hours, then removed and equilibrated at room temperature for 10 minutes.

100 μL of detection reagent was added to each well and placed on a horizontal shaker at low speed for 10 minutes to fully lyse the cells. The fluorescence value of each well was detected using PerkinElmer Envision microplate reader.

(6) Calculation of results

Using 0nM as a control, the values for each well were converted to percentages and the IC50 was calculated by nonlinear fitting using [Inhibitor] vs. response (three parameters) in GrahpPad prism software.

As shown in Table 1, where A≤100nM; 100nM<B<1μM; 5μM≥C≥1μM

Table 1

| Compound | Structure | NCI-H226 Activity ($IC_{50}$) | Nano Luciferas detection ($IC_{50}$) |
|---|---|---|---|
| T1 | 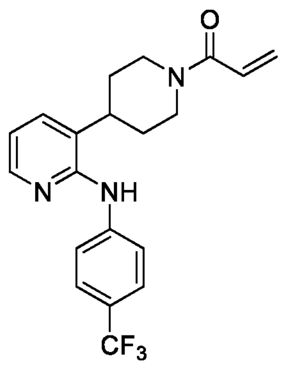 | A | A |
| T2 | 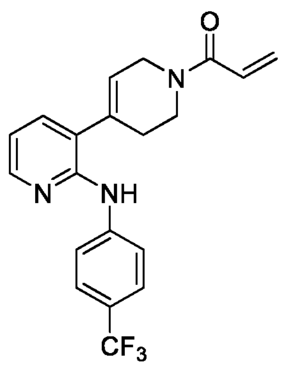 | A | A |
| T3 | 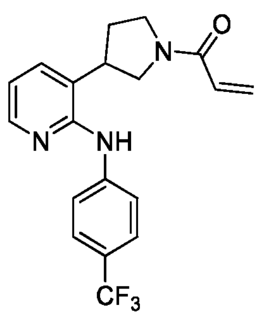 | B | B |

(continued)

| Compound | Structure | NCI-H226 Activity ($IC_{50}$) | Nano Luciferas detection ($IC_{50}$) |
|---|---|---|---|
| T4 | 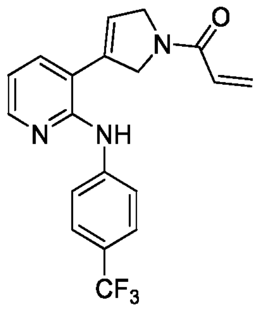 | A | A |
| T5 | 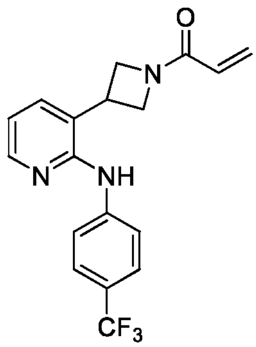 | B | B |
| T6 | 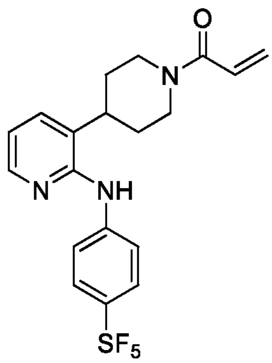 | A | A |
| T7 | 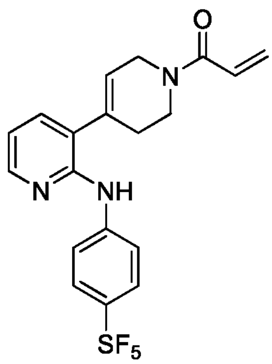 | A | A |

(continued)

| Compound | Structure | NCI-H226 Activity ($IC_{50}$) | Nano Luciferas detection ($IC_{50}$) |
|---|---|---|---|
| T8 | 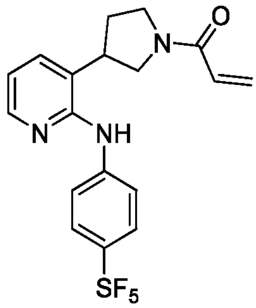 | A | A |
| T9 | 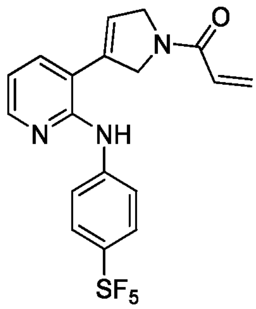 | A | A |
| T10 | 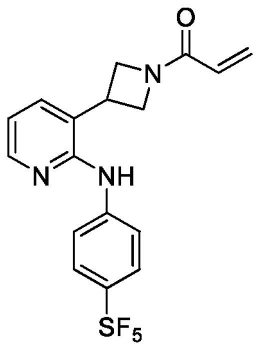 | A | A |
| T11 | 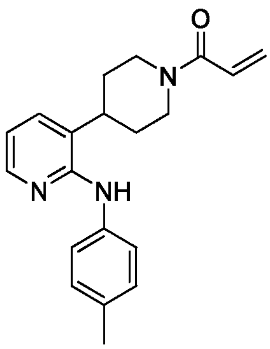 | A | A |

(continued)

| Compound | Structure | NCI-H226 Activity ($IC_{50}$) | Nano Luciferas detection ($IC_{50}$) |
|---|---|---|---|
| T12 | 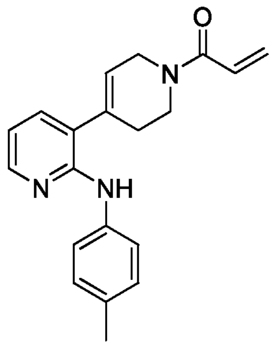 | A | A |
| T13 | 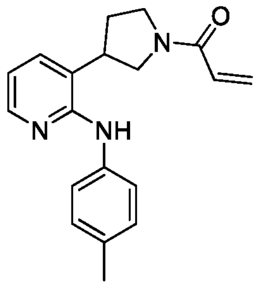 | A | A |
| T14 | 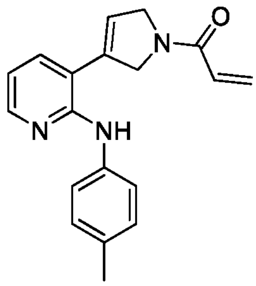 | A | A |
| T15 | 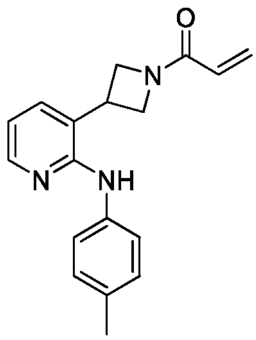 | A | A |
| T16 | 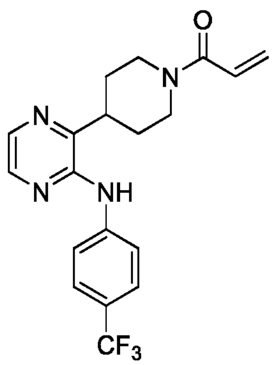 | A | A |

(continued)

| Compound | Structure | NCI-H226 Activity ($IC_{50}$) | Nano Luciferas detection ($IC_{50}$) |
|---|---|---|---|
| T17 | 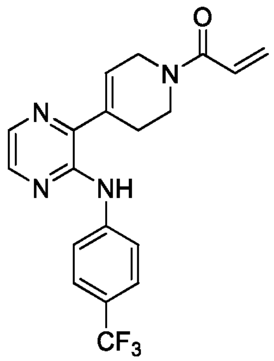 | A | A |
| T18 | 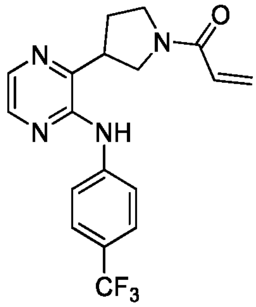 | A | A |
| T19 | 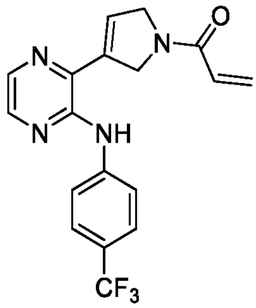 | A | A |
| T20 | 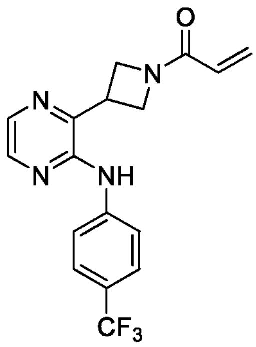 | A | A |

(continued)

| Compound | Structure | NCI-H226 Activity ($IC_{50}$) | Nano Luciferas detection ($IC_{50}$) |
|---|---|---|---|
| T21 | 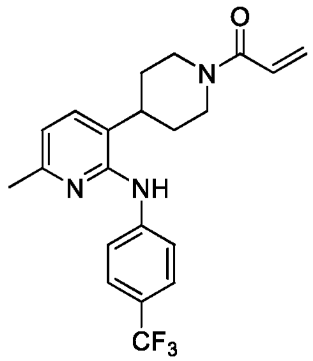 | A | A |
| T22 | 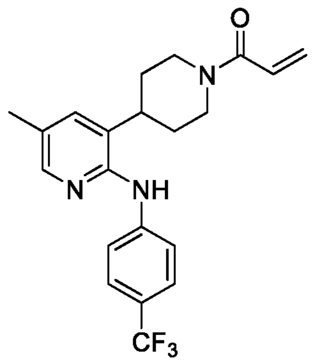 | A | A |
| T23 | 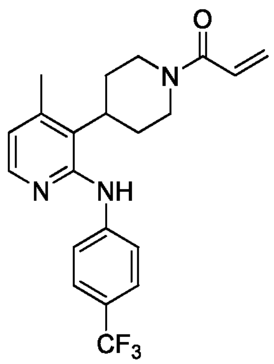 | A | A |
| T24 | 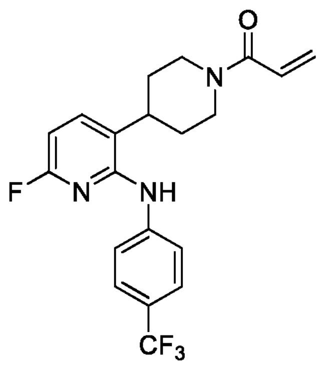 | A | A |

(continued)

| Compound | Structure | NCI-H226 Activity ($IC_{50}$) | Nano Luciferas detection ($IC_{50}$) |
|---|---|---|---|
| T25 | 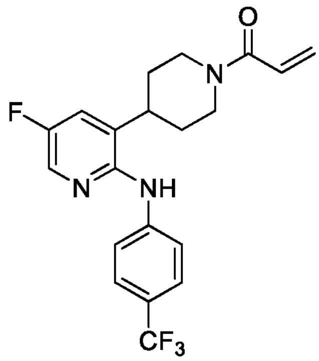 | A | A |
| T26 | 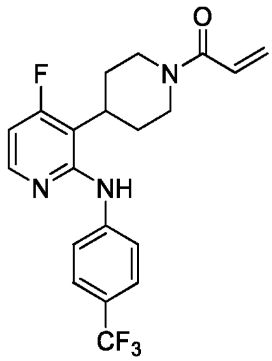 | A | A |
| T27 | 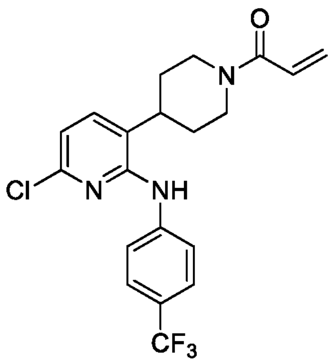 | B | A |
| T28 | 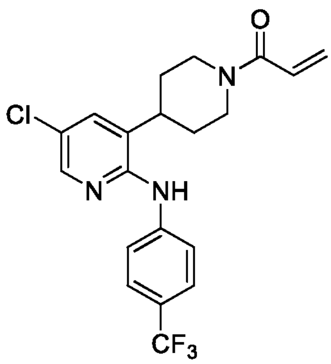 | A | A |

(continued)

| Compound | Structure | NCI-H226 Activity ($IC_{50}$) | Nano Luciferas detection ($IC_{50}$) |
|---|---|---|---|
| T29 | 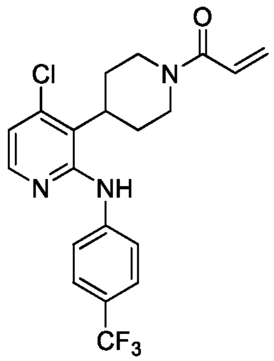 | A | A |
| T33 | 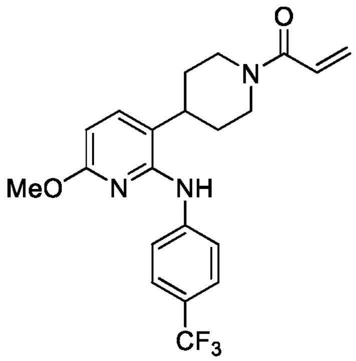 | B | A |
| T34 | 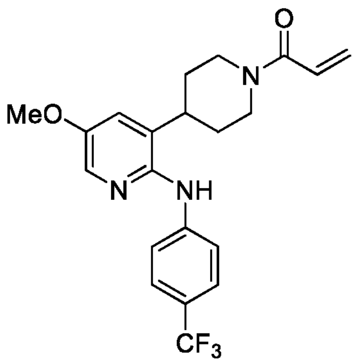 | B | B |
| T35 | 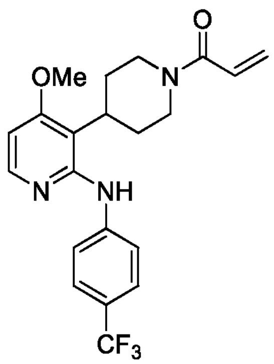 | B | B |

(continued)

| Compound | Structure | NCI-H226 Activity ($IC_{50}$) | Nano Luciferas detection ($IC_{50}$) |
|---|---|---|---|
| T36 | 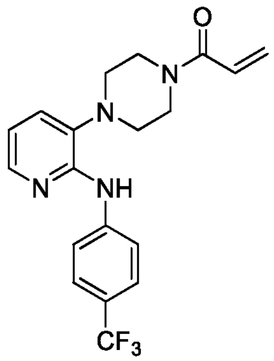 | B | B |
| T37 | 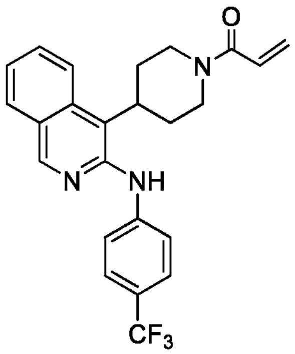 | B | B |
| T38 | 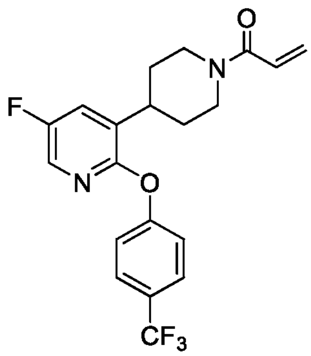 | B | B |
| T39 | 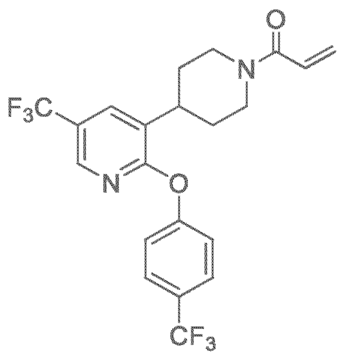 | B | B |

(continued)

| Compound | Structure | NCI-H226 Activity ($IC_{50}$) | Nano Luciferas detection ($IC_{50}$) |
|---|---|---|---|
| T40 | 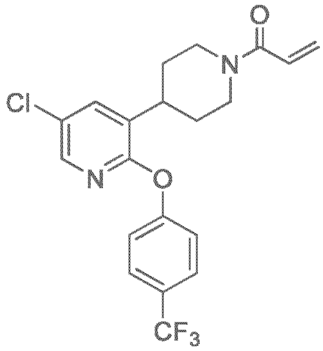 | A | A |
| T42 | 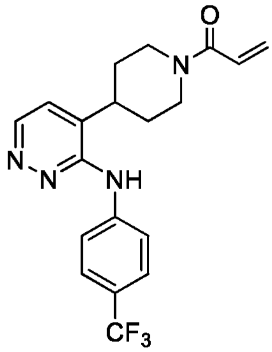 | B | B |
| T43 | 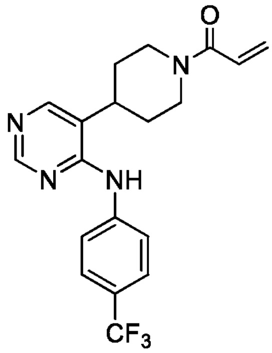 | B | B |
| T44 | 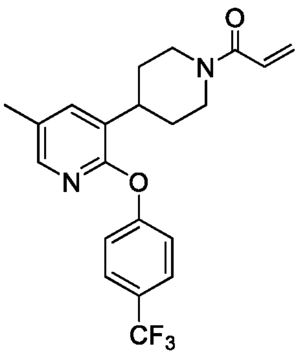 | B | B |

(continued)

| Compound | Structure | NCI-H226 Activity ($IC_{50}$) | Nano Luciferas detection ($IC_{50}$) |
|---|---|---|---|
| T45 | 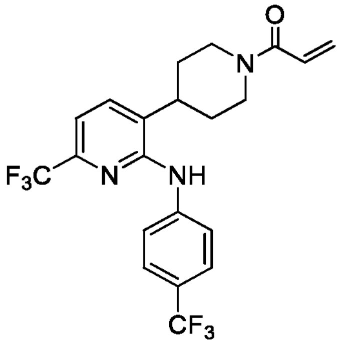 | C | B |
| T47 | 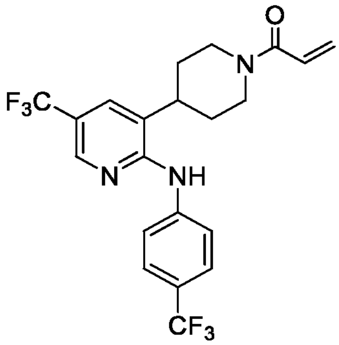 | B | B |
| T48 | 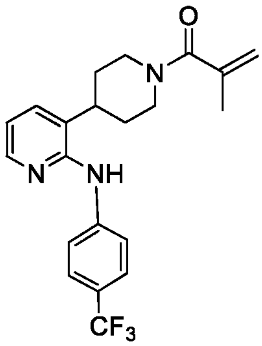 | C | C |
| T49 | 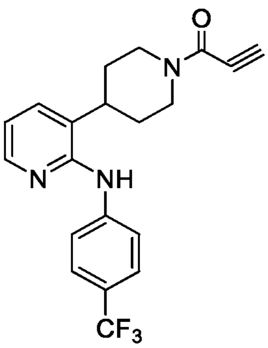 | C | A |

(continued)

| Compound | Structure | NCI-H226 Activity ($IC_{50}$) | Nano Luciferas detection ($IC_{50}$) |
|---|---|---|---|
| T50 | 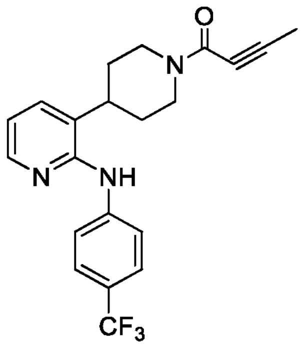 | C | C |
| T51 | 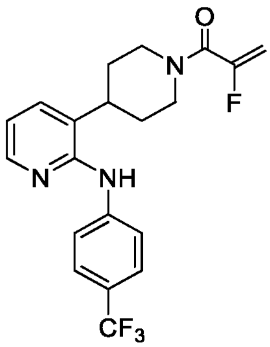 | B | B |
| T52 | 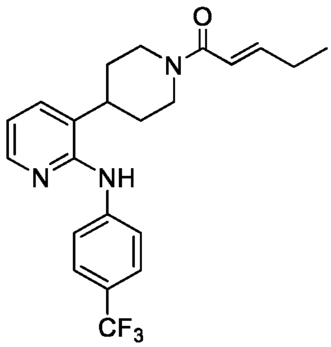 | C | B |
| T53 | 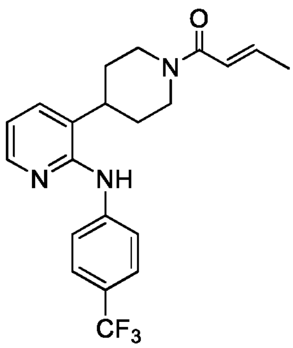 | C | B |

(continued)

| Compound | Structure | NCI-H226 Activity ($IC_{50}$) | Nano Luciferas detection ($IC_{50}$) |
|---|---|---|---|
| T54 | 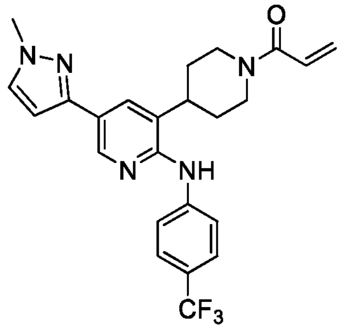 | B | A |
| T55 | 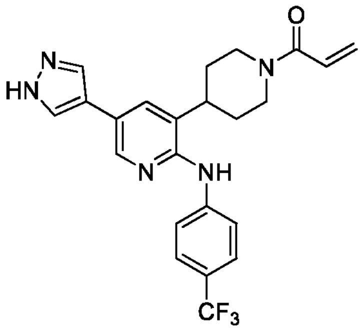 | B | B |
| T56 | 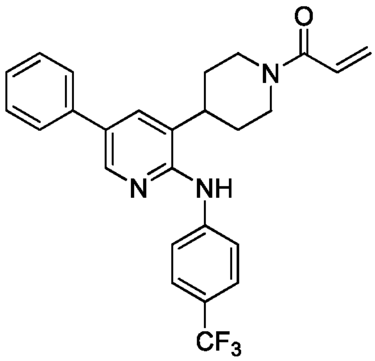 | A | A |
| T57 | 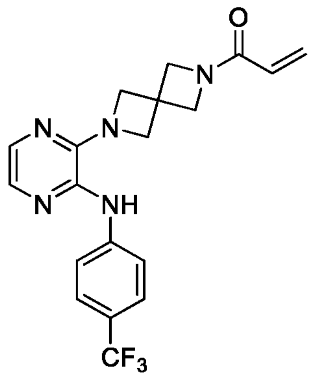 | A | A |

(continued)

| Compound | Structure | NCI-H226 Activity ($IC_{50}$) | Nano Luciferas detection ($IC_{50}$) |
|---|---|---|---|
| T58 | 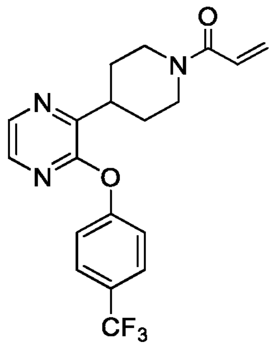 | A | A |
| T59 | 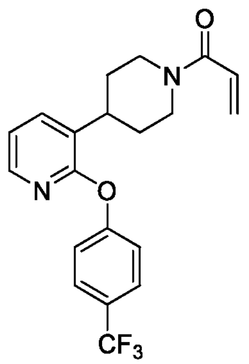 | A | A |
| T60 | 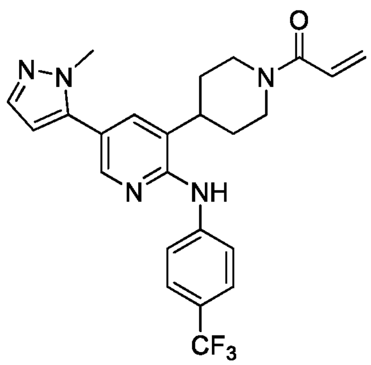 | C | B |
| T61 | 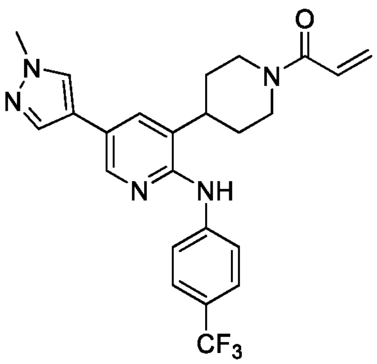 | C | A |

(continued)

| Compound | Structure | NCI-H226 Activity ($IC_{50}$) | Nano Luciferas detection ($IC_{50}$) |
|---|---|---|---|
| T62 | 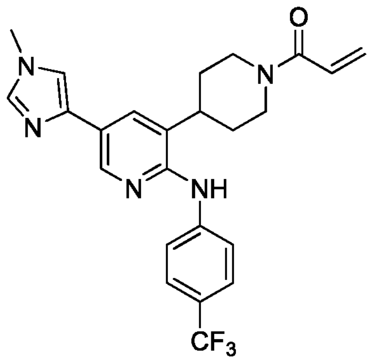 | B | A |
| T63 | 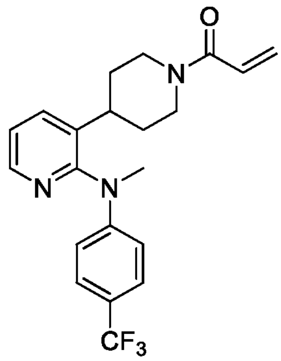 | B | B |
| T64 | 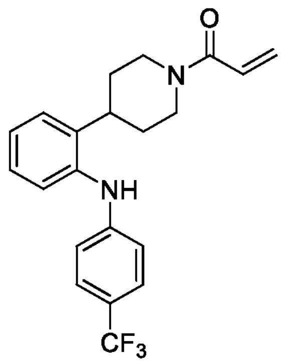 | A | A |
| T65 | 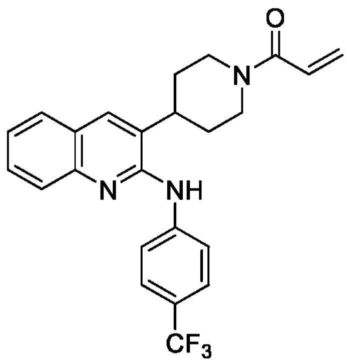 | B | B |

(continued)

| Compound | Structure | NCI-H226 Activity ($IC_{50}$) | Nano Luciferas detection ($IC_{50}$) |
|---|---|---|---|
| T73 | 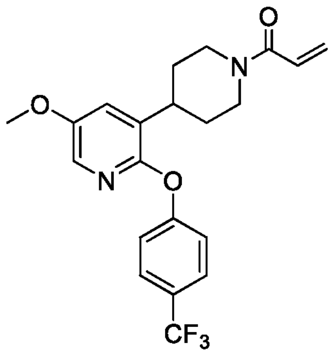 | B | B |
| T74 | 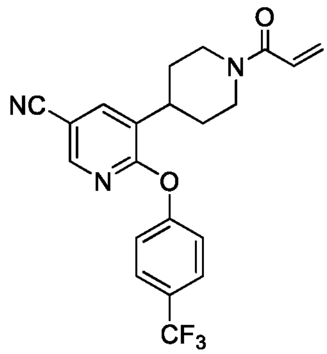 | B | B |
| T77 | 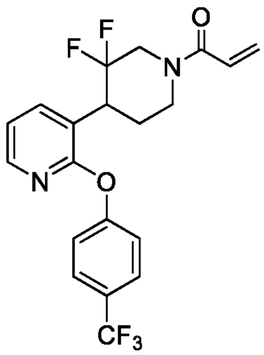 | A | A |
| T78 | 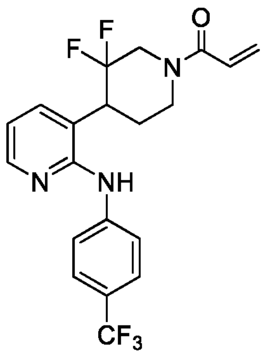 | A | A |

(continued)

| Compound | Structure | NCI-H226 Activity ($IC_{50}$) | Nano Luciferas detection ($IC_{50}$) |
|---|---|---|---|
| T79 | 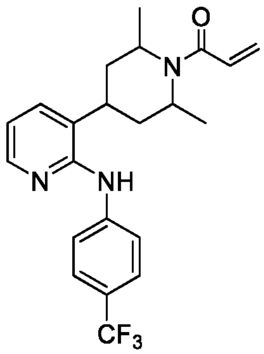 | A | A |
| T80 | 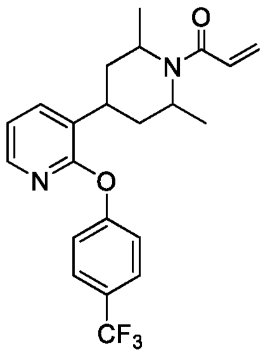 | A | A |
| T81 | 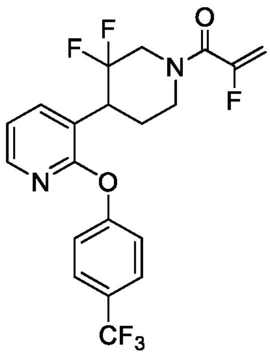 | B | B |
| T83 | 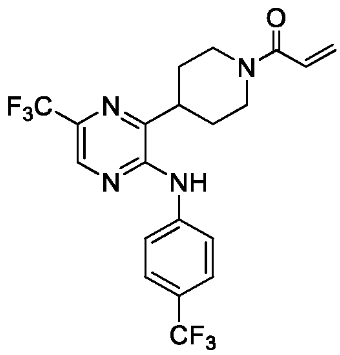 | A | A |

(continued)

| Compound | Structure | NCI-H226 Activity ($IC_{50}$) | Nano Luciferas detection ($IC_{50}$) |
|---|---|---|---|
| T92 | 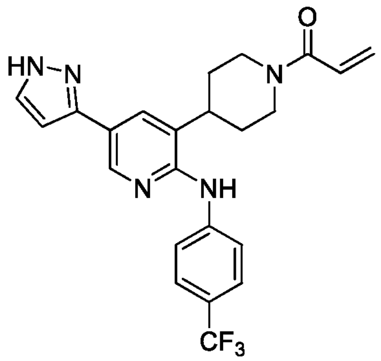 | B | A |
| T93 | 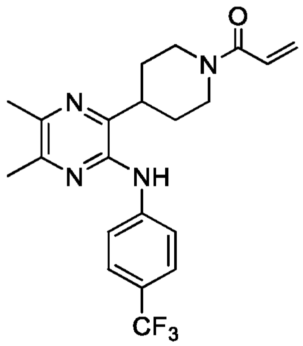 | B | B |
| T95 | 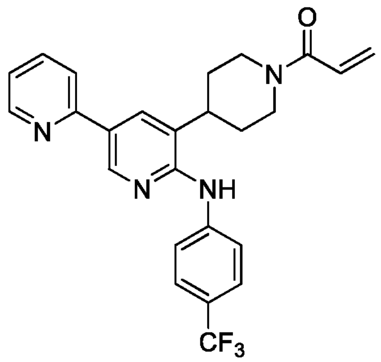 | C | B |
| T96 | 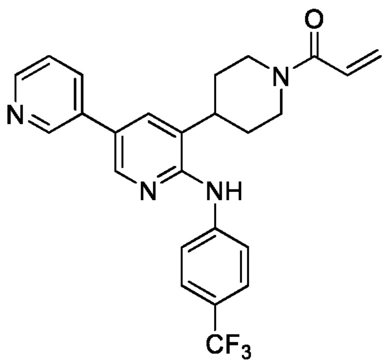 | C | B |

(continued)

| Compound | Structure | NCI-H226 Activity ($IC_{50}$) | Nano Luciferas detection ($IC_{50}$) |
|---|---|---|---|
| T97 | 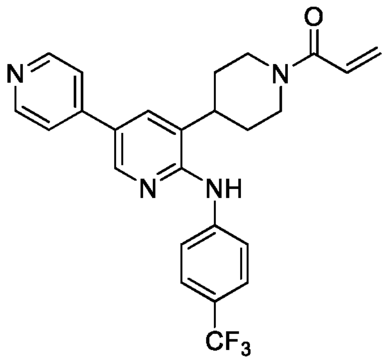 | C | B |
| T98 | 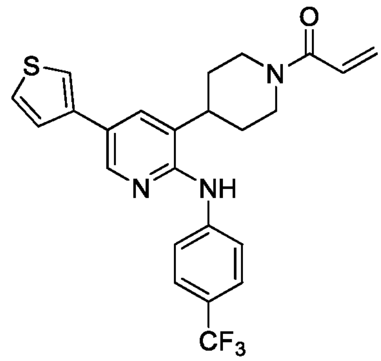 | C | B |
| T99 | 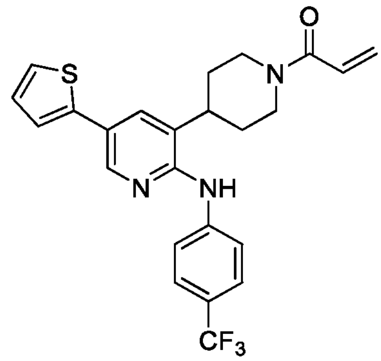 | C | B |

Table 2

| Compound | Structure | NCI-H226 Activity ($IC_{50}$) | Nano Luciferas detection ($IC_{50}$) |
|---|---|---|---|
| Reference compound 1 | 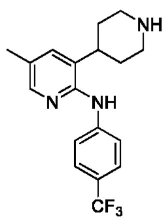 | > 5000 nM | > 5000 nM |

(continued)

| Compound | Structure | NCI-H226 Activity ($IC_{50}$) | Nano Luciferas detection ($IC_{50}$) |
|---|---|---|---|
| Reference compound 2 | 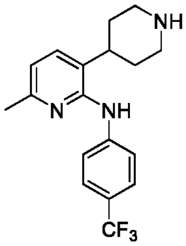 | > 5000 nM | > 5000 nM |
| Reference compound 3 | 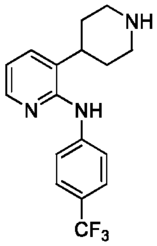 | > 5000 nM | > 5000 nM |
| Reference compound 4 | 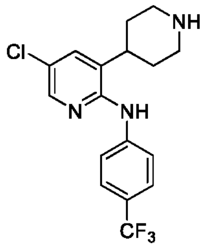 | > 5000 nM | > 5000 nM |
| Reference compound 5 | 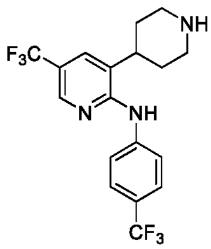 | > 5000 nM | > 5000 nM |
| Reference compound 6 | 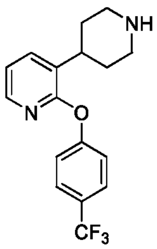 | > 5000 nM | > 5000 nM |
| Reference compound 7 | 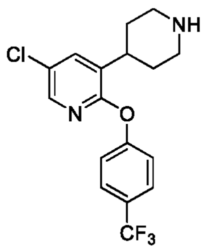 | > 5000 nM | > 5000 nM |

Experimental example 3 Pharmacokinetic study of the drug

Oral administration solvent: 5% DMSO+10% Solutol HS15+85% saline

Oral administration preparation process: weighed the appropriate amount of powder, added the appropriate volume of DMSO, vortex, then added the appropriate volume of Solutol solution, vortex, and added the appropriate amount of saline, vortex ultrasound into a homogeneous and clarified state of the preparation solution.

Clarified solution preparation, 2 samples, 100 μL/sample, stored at 2~8 °C.

The animal room was equipped with an air-conditioning system and well ventilated, and the indoor temperature was maintained in the range of 20~26 °C, and the humidity was maintained in the range of 40%~70%. The animal room was artificially illuminated with 12 hours of light and 12 hours of dark (except for the working light that needed to be turned on for experimental operation and cleaning), and the experimental animals were free to feed and drink.

Animals were purchased and fed normally for at least 3 days, and rats that were examined by a veterinarian and had qualified physical conditions were enrolled in this experiment; each rat was labeled with a tail mark. Animals in the oral administration group were fasted overnight the day before administration, and resumed feeding and drinking freely 4 hours after administration.

Animal grouping and drug administration

| Group | Animal No. | Number of males | Dosage (mg/kg) | Volume (mL/kg) | Concentration (mg/mL) | Route of administration | Frequency of administration |
|---|---|---|---|---|---|---|---|
| 1 | R1-R3 | 3 | 10 | 10 | 1 | PO | Single |
| 2 | R4-R6 | 3 | | | | | |
| 3 | R7-R9 | 3 | | | | | |
| 4 | R10-R12 | 3 | | | | | |
| 5 | R13-R15 | 3 | | | | | |
| Note: 1) Free access to water before and after administration; 2) All experiments were conducted according to the actual body weight of the animals. | | | | | | | |

Before administration, checked the state of the administered preparation, ensured the homogeneity of the preparation by vortexing, stirring or oscillating, and calculated the theoretical volume of the administered drug for each SD rat in each group according to the following formula.

$$\text{theoretical volume of the administered drug(mL)} = \frac{\text{dosage } (\text{mg}\cdot\text{kg}^{-1})}{\text{preparation concentration } (\text{mg}\cdot\text{mL}^{-1})} \times \text{Animal weight (kg)}$$

Mode of administration: Oral administration to rats, fasted overnight prior to administration.

Sample collection information

| Group | Euthanasia time | Sample collection |
|---|---|---|
| 1 | 2 h | Plasma, cerebrospinal fluid, brain tissue (post perfusion) |
| 2 | 4 h | |
| 3 | 6 h | |
| 4 | 8 h | |
| 5 | 24 h | |
| Note: Time window for blood collection ± 1 minute for blood collection sites within 1 hour (excluding pre-administration sites), and time window for blood collection ± 5% for other time points; samples are sent out for testing. | | |

Table 3

| Compoun d No. | Tmax (h) | Cmax (ng/ml) | AUClast (h*ng/ml) | AUCinf (h*ng/ml) | HL_(h) | MRTlast (h) | B/P value | Kpuu Value |
|---|---|---|---|---|---|---|---|---|
| T1 | 1.33 | 919.33 | 5253.44 | 5255.52 | 1.93 | 4.28 | ND | ND |
| T16 | 2.67 | 2840.00 | 17340.47 | 17356.14 | 2.07 | 4.48 | 1.41 | 0.518 |
| T22 | 2.00 | 594.00 | 3294.25 | 3501.92 | 2.64 | 3.97 | ND | ND |
| T27 | 3.33 | 1883.00 | 19396.00 | 19571.00 | 3.03 | 6.89 | ND | ND |
| T37 | 4.67 | 804.00 | 12250.75 | 17966.74 | 13.97 | 9.85 | ND | ND |
| T40 | 1.67 | 1646.67 | 17521.97 | 18021.23 | 4.40 | 6.75 | 1.88 | 0.286 |
| T58 | 2.00 | 1183 | 7535 | 7556 | 2.67 | 5.81 | 2.01 | 0.269 |
| T59 | 1.50 | 679.33 | 3905.96 | 3917.28 | 2.66 | 4.60 | 1.22 | 0.765 |
| T61 | 2.67 | 1963.33 | 10832.50 | 13196.24 | 2.95 | 3.49 | 0.56 | 0.038 |
| T92 | 2.67 | 901.33 | 4771.23 | 4834.51 | 1.59 | 3.80 | ND | ND |

All literatures mentioned in the present invention are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

**1.** A compound of formula I or a pharmaceutically acceptable salt, solvate or prodrug thereof:

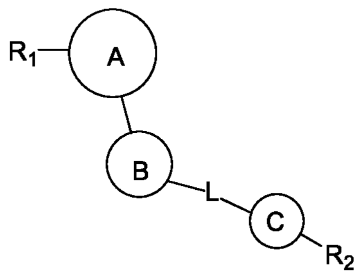

Formula I

wherein,

A is selected from the group consisting of

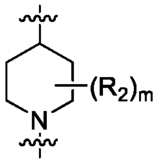, 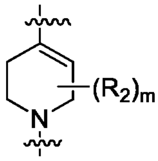, 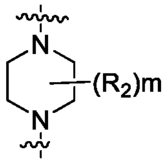,

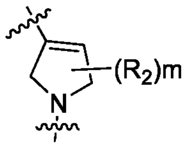, 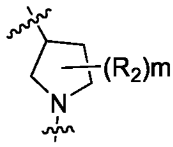, 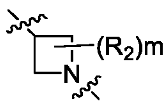, and 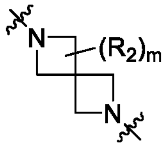;

B is selected from the group consisting of

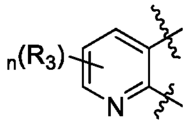, 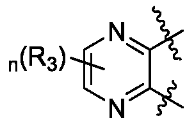,

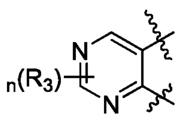, 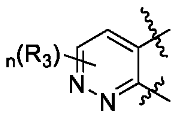, 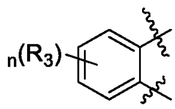, 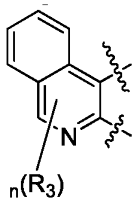, 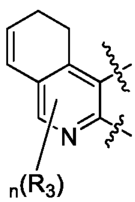, and 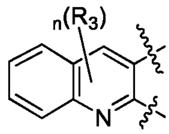;

C is selected from the group consisting of

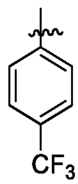, 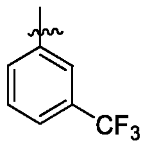, 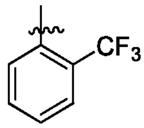, 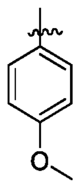,

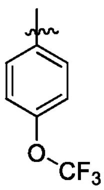, 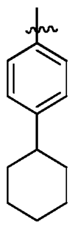, 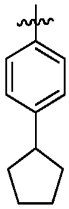, 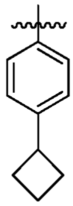, 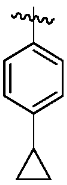, 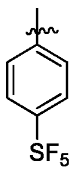, 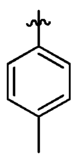, 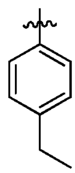, 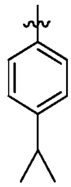, 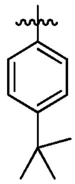, 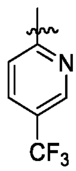,

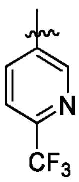, 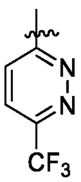, 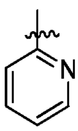, 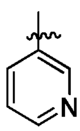, 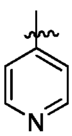, 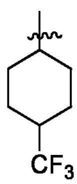, 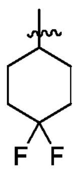, 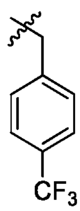, and 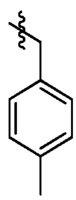;

L is selected from the group consisting of $NR_4$, O, S, Se, sulfoxide, sulfone, and $CR_2R_3$;
$R_1$ is selected from the group consisting of

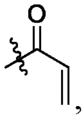, 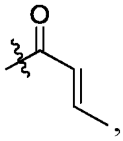, 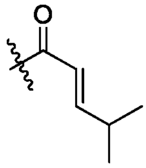, 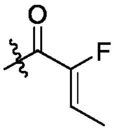,

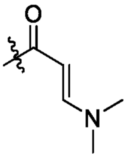, 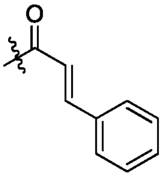, 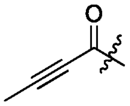, 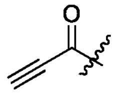, 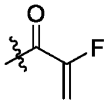, 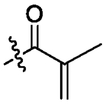, 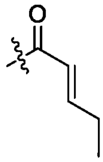, 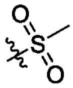,

and

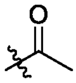,

which is connected to N on A;

$R_2$ is each independently selected from the group consisting of H, halogen, CN, $NH_2$, hydroxyl, ester group, ureido, urethane, amido, $C_{1-6}$ alkyl, $C_{1-6}$ alkoxy, $C_{3-6}$ cycloalkyl, $C_{3-6}$ cycloalkoxy, aryl, and heteroaryl, wherein the hydrogen in $NH_2$, ester, ureido, urethane, amido, $C_{1-6}$ alkyl, $C_{1-6}$ alkoxy, $C_{3-6}$ cycloalkyl, $C_{3-6}$ cycloalkoxy, aryl, and heteroaryl can be optionally substituted, and the substituents are H, halogen, CN, $NH_2$, hydroxyl, ester group, ureido, urethane, amido, $C_{1-6}$ alkyl, $C_{1-6}$ alkoxy, $C_{3-6}$ cycloalkyl, $C_{3-6}$ cycloalkoxy, aryl, and heteroaryl, or $R_2$ forms a 3-7-membered ring with the attached structure, or a 3-7-membered ring is formed between the different $R_2$;

$R_3$ is each independently selected from the group consisting of H, halogen, CN, $NH_2$, hydroxyl, ester group, ureido, urethane, amido, $C_{1-6}$ alkyl, $C_{1-6}$ alkoxy, $C_{3-6}$ cycloalkyl, $C_{3-6}$ cycloalkoxy, aryl, and heteroaryl, wherein the hydrogen in $NH_2$, ester group, ureido, urethane, amido, $C_{1-6}$ alkyl, $C_{1-6}$ alkoxy, $C_{3-6}$ cycloalkyl, $C_{3-6}$ cycloalkoxy, aryl, and heteroaryl can be optionally substituted, and the substituents are H, halogen, CN, $NH_2$, hydroxyl, ester group, ureido, urethane, amido, $C_{1-6}$ alkyl, $C_{1-6}$ alkoxy, $C_{3-6}$ cycloalkyl, $C_{3-6}$ cycloalkoxy, aryl, and heteroaryl, or a 5-7-membered ring is formed between the different $R_3$ and the attached B;

$R_4$ is selected from the group consisting of H, ester group, $-C(O)R_5$, ureido, $C_{1-6}$ alkyl, $C_{1-6}$ alkoxy, $C_{3-6}$ cycloalkyl, aryl, and heteroaryl, wherein the hydrogen in the ester group, ureido, $C_{1-6}$ alkyl, $C_{3-6}$ cycloalkyl, aryl, and heteroaryl can be optionally substituted, and the substituents are H, halogen, CN, $NH_2$, hydroxyl, ester group, ureido, urethane, amido, $C_{1-6}$ alkyl, $C_{1-6}$ alkoxy, $C_{3-6}$ cycloalkyl, $C_{3-6}$ cycloalkoxy, aryl, and heteroaryl;

$R_5$ is selected from the group consisting of H, $C_{1-6}$ alkyl, $C_{3-6}$ cycloalkyl, aryl, and heteroaryl;

m and n are each independently selected from the group consisting of 0, 1, 2, 3, 4, 5, and 6.

2. The compound or the pharmaceutically acceptable salt, solvate or prodrug thereof according to claim 1, wherein, m is selected from the group consisting of 0, 1, 2 and 3.

3. The compound or the pharmaceutically acceptable salt, solvate or prodrug thereof according to claim 1, wherein, n is selected from the group consisting of 0, 1, 2 and 3.

4. The compound or the pharmaceutically acceptable salt, solvate or prodrug thereof according to claim 1, wherein, A is selected from the group consisting of

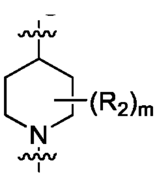,

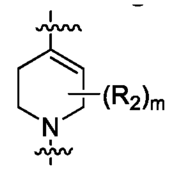

, and

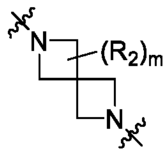

;

$R_2$ and m are as defined in claim 1.

**5.** The compound or the pharmaceutically acceptable salt, solvate or prodrug thereof according to claim 1, wherein B is selected from the group consisting of

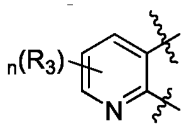

and

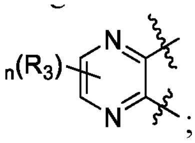

$R_3$ and n are as defined in claim 1.

**6.** The compound or the pharmaceutically acceptable salt, solvate or prodrug thereof according to claim 1, wherein, the compound is selected from the group consisting of

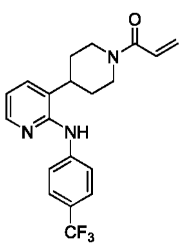

T1

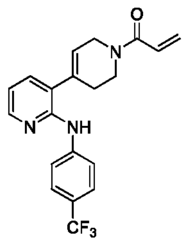

T2

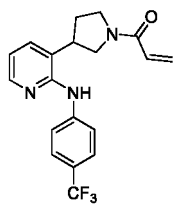

T3

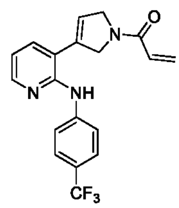

T4

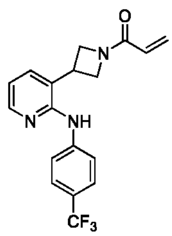

T5

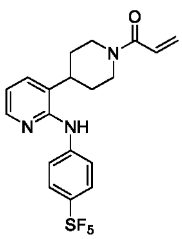

T6

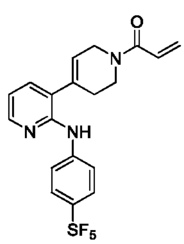

T7

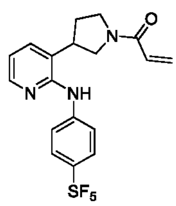

T8

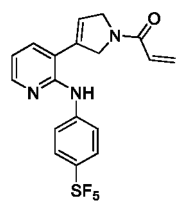

T9

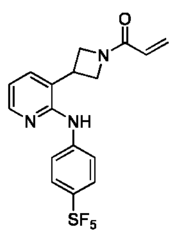

T10

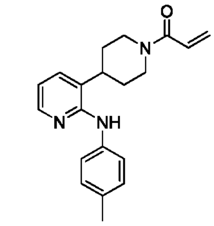 T11
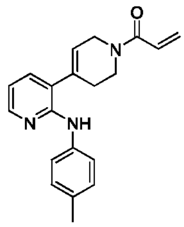 T12
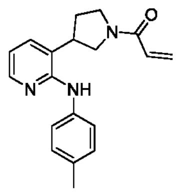 T13
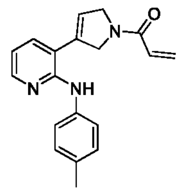 T14
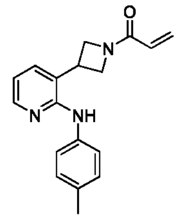 T15

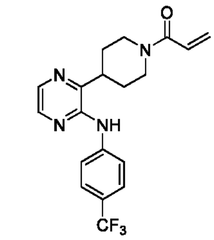 T16
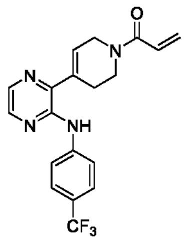 T17
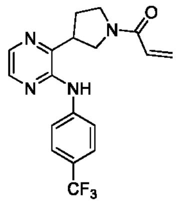 T18
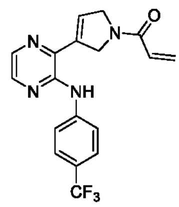 T19
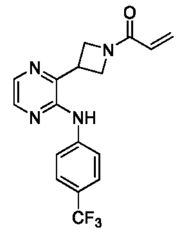 T20

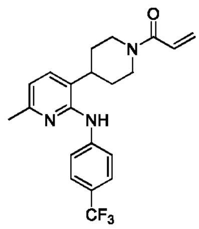 T21
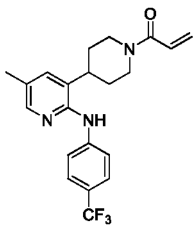 T22
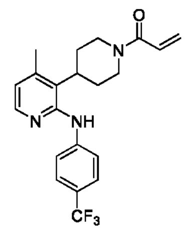 T23
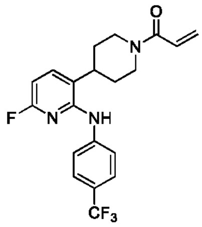 T24
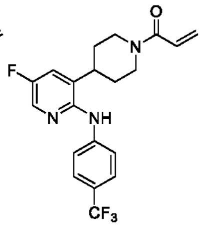 T25
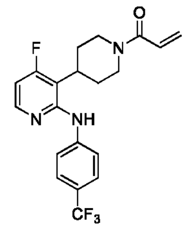 T26

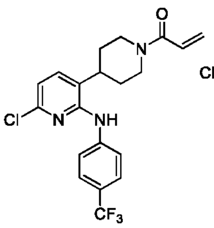 T27
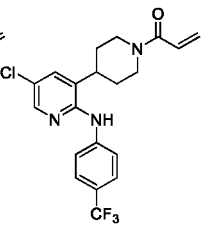 T28
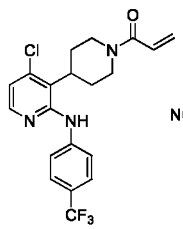 T29
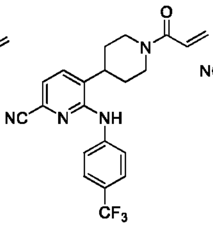 T30
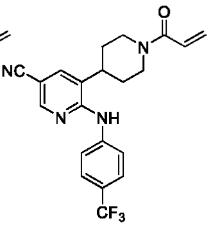 T31
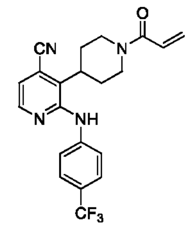 T32

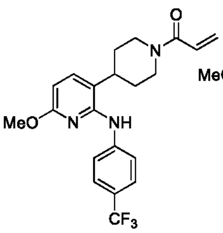 T33
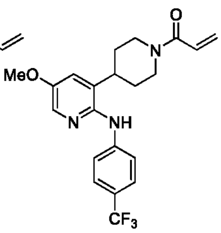 T34
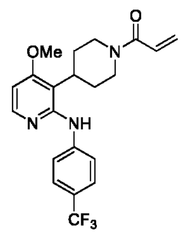 T35
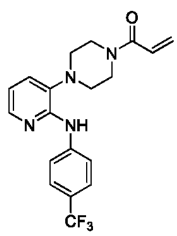 T36
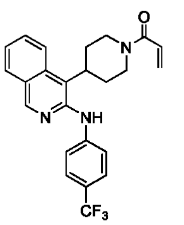 T37
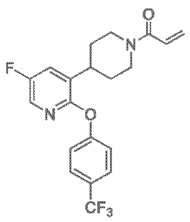 T38

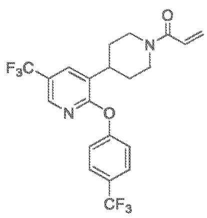
T39

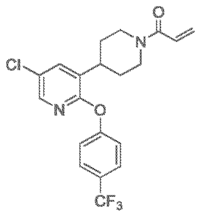
T40

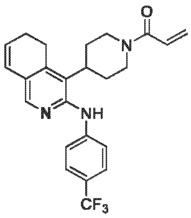
T41

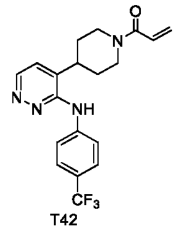
T42

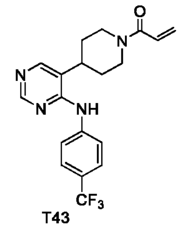
T43

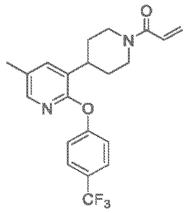
T44

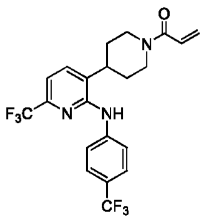
T45

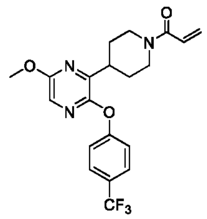
T46

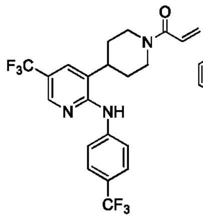
T47

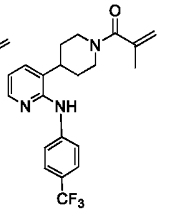
T48

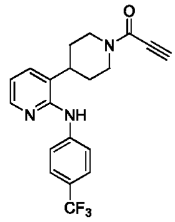
T49

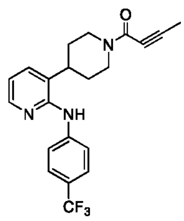
T50

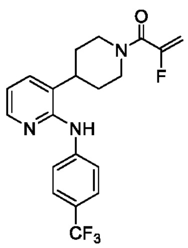
T51

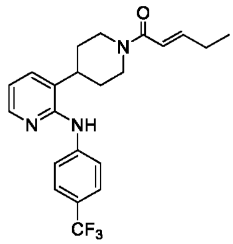
T52

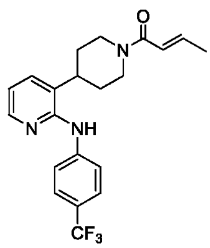
T53

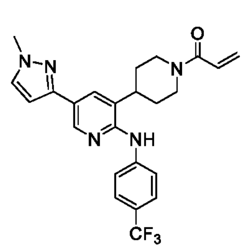
T54

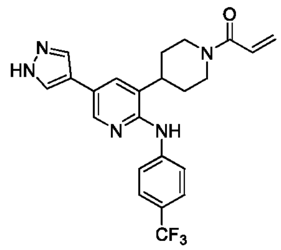
T55

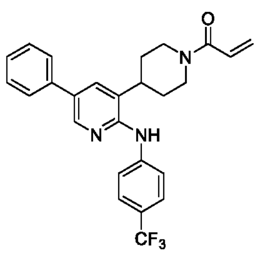
T56

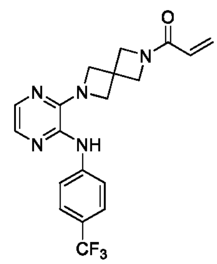
T57

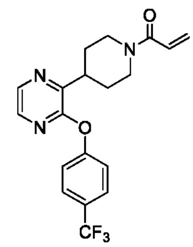
T58

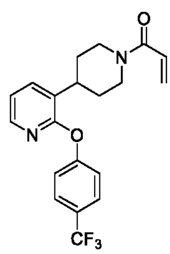
T59

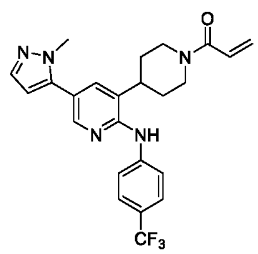
T60

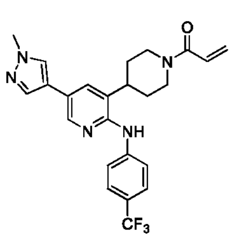
T61

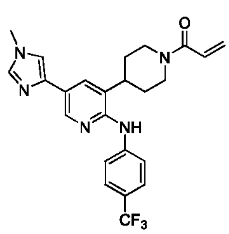
T62

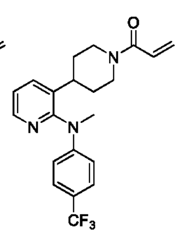
T63

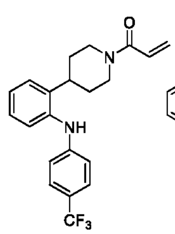
T64

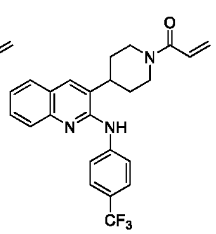
T65

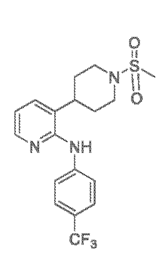
T66

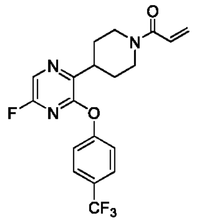
T67

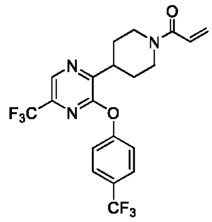
T68

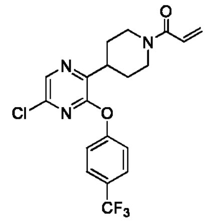
T69

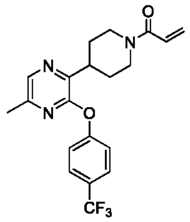
T70

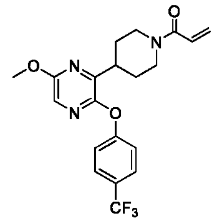
T71

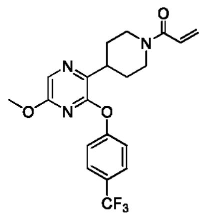
T72

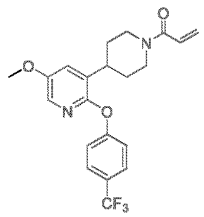
T73

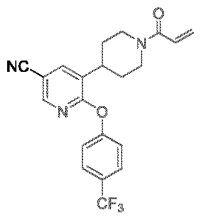
T74

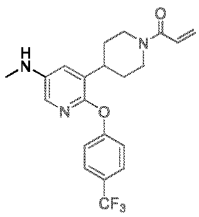
T75

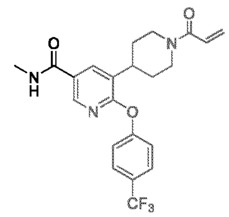
T76

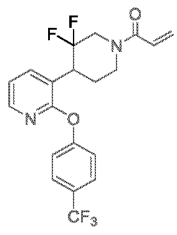
T77

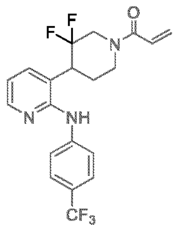
T78

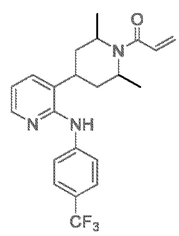
T79

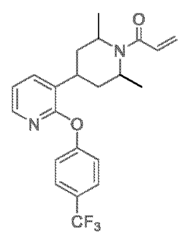
T80

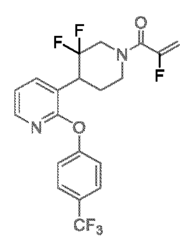
T81

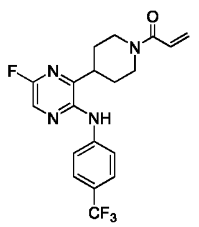
T82

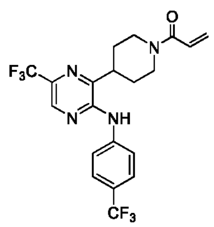
T83

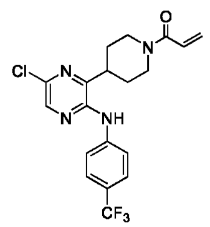
T84

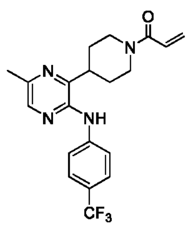
T85

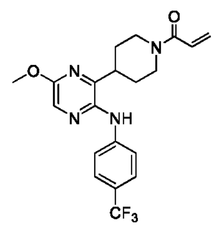
T86

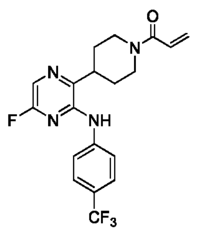
T87

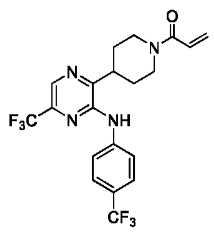
T88

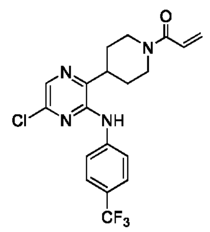
T89

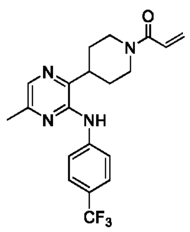
T90

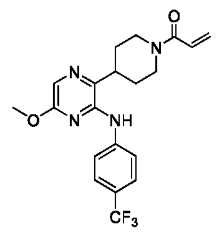
T91

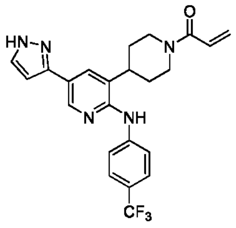
T92

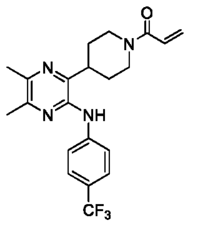
T93

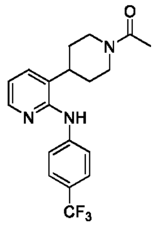
T94

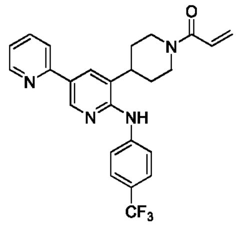
T95

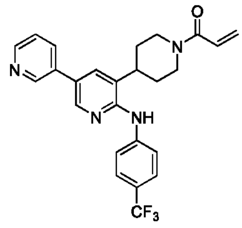
T96

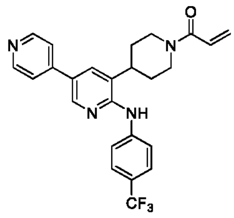
T97

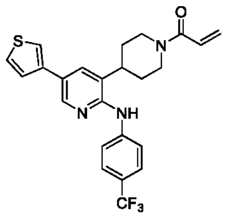
T98

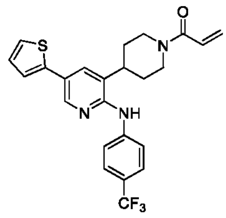
T99

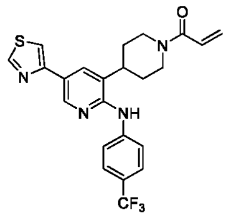
T100

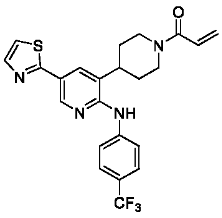
T101

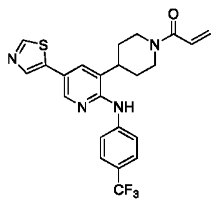
T102

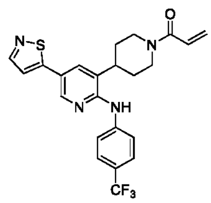
T103

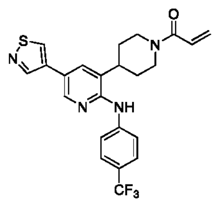
T104

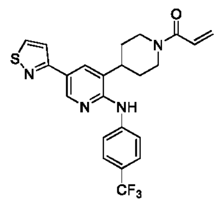
T105

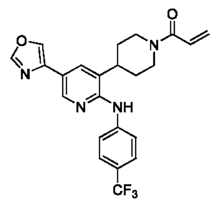
T106

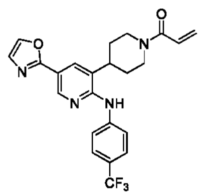
T107

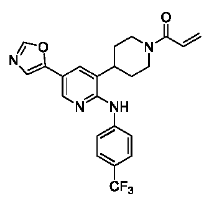
T108

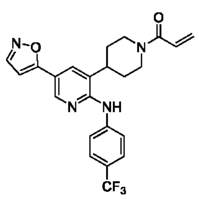
T109

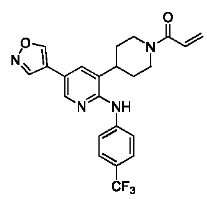
T110

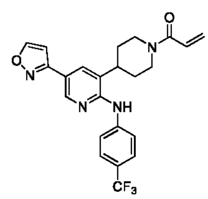
T111

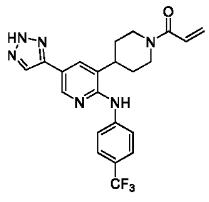
T112

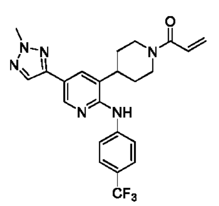
T113

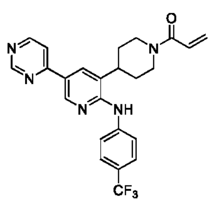
T114

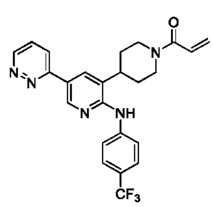
T115

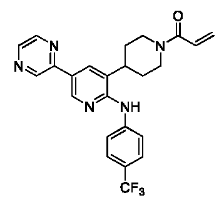
T116

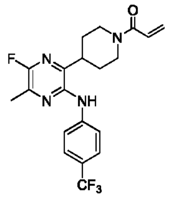
T117

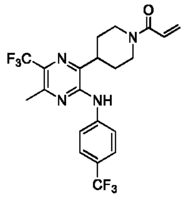
T118

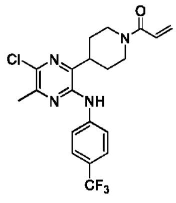
T119

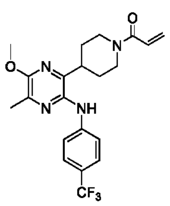
T120

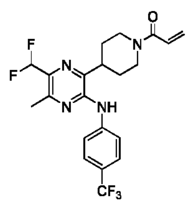
T121

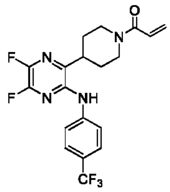

T122

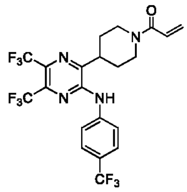

T123

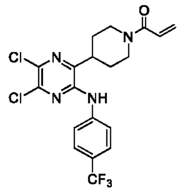

T124

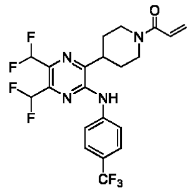

T125

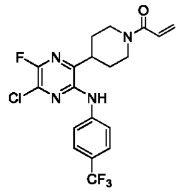

T126

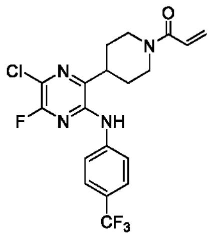

T127

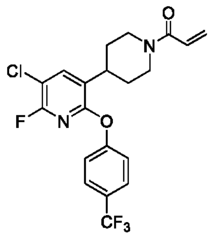

T128

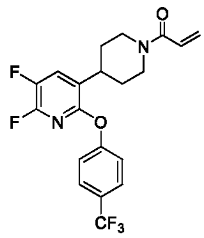

T129

**7.** A pharmaceutical composition comprises pharmaceutically acceptable carriers and one or more safe and effective amounts of the compound or the pharmaceutically acceptable salt, solvate or prodrug thereof of claim 1.

**8.** Use of the pharmaceutical composition of claim 7 for the preparation of a drug, the drug is used for the prevention and/or treatment of proliferative diseases.

**9.** Use of the pharmaceutical composition of claim 7 for the preparation of a drug, the drug is used for purposes selected from the group consisting of

1) prevention and/or treatment of diseases associated with abnormal activity of YAP/TEAD;
2) prevention and/or treatment of diseases associated with dysregulation of the Hippo pathway; and
3) preventing and/or treatment diseases associated with dysregulation of YAP or TAZ or YAP/TAZ or YAP/TEAD or YAP/TAZ/TEAD.

**10.** A combination of the compound or the pharmaceutically acceptable salt, solvate or prodrug thereof of claim 1 with a second drug, wherein the combination is used for the prevention and/or treatment of cancer;
the second drug is selected from the group consisting of ERK inhibitors, MEK inhibitors, KRAS inhibitors, BRAF inhibitors, EGFR inhibitors, Wnt inhibitors and PD-1 inhibitors.

**INTERNATIONAL SEARCH REPORT**

International application No.
**PCT/CN2023/093095**

**A. CLASSIFICATION OF SUBJECT MATTER**

C07D401/04(2006.01)i; C07D401/14(2006.01)i; A61P35/00(2006.01)i; A61K31/44(2006.01)i

According to International Patent Classification (IPC) or to both national classification and IPC

**B. FIELDS SEARCHED**

Minimum documentation searched (classification system followed by classification symbols)

IPC:C07D A61P A61K

Documentation searched other than minimum documentation to the extent that such documents are included in the fields searched

Electronic data base consulted during the international search (name of data base and, where practicable, search terms used)

CNKI; CNABS; CNTXT; DWPI; VCN; ENTXT; ENTXTC; WPABS; ISI-WEB OF SCIENCE; STN: 浙江同源康医药, 梁阿朋, 刘广斌, 陈少清, 李钧, 吴豫生, 尹洲, 董胜利, 牛成山, 李美华, 多环, 吡嗪, 哌啶, 哌嗪, 吡咯, 吡啶, 吡嗪, 嘧啶, 哒嗪, 癌症, 肿瘤, 增殖, Hippo, YAP, TAZ, TEAD, polycyclic, pyrazine, piperidine, piperazine, pyrrole, pyridine, pyrimidine, pyridazine, cancer, tumour, tumor, structural formula (I)

**C. DOCUMENTS CONSIDERED TO BE RELEVANT**

| Category* | Citation of document, with indication, where appropriate, of the relevant passages | Relevant to claim No. |
|---|---|---|
| X | TALMON Maria et al. "Design, Synthesis and Biological Evaluation of Vortioxetine Derivatives as New COX-1/2 Inhibitors in Human Monocytes" *Bioorganic & Medicinal Chemistry,* Vol. 28, No. 23, 11 September 2020 (2020-09-11), ISSN: 1464-3391, page 3, Fig.2, and page 5, Fig.3 | 1-3, 7 |
| X | WO 2018014880 A1 (LI QINGENG) 25 January 2018 (2018-01-25) description, page 13, table | 1-3 |
| X | CN 102300860 A (AMGEN INC.) 28 December 2011 (2011-12-28) description, paragraphs [0666], [0675], and [1375] | 1-5, 7-10 |
| X | DATEBASE REGISTRY [Online]. "RN 2127383-47-3, RN 2127382-82-3, et al." *CHEMICAL ABSTRACTS SERVICE,* 14 September 2017 (2017-09-14), pages 1-50 | 1-5 |
| X | CN 103814016 A (MERCK PATENT GMBH) 21 May 2014 (2014-05-21) claims 1-24 | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 July 2023** | **12 August 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/093095**

**C. DOCUMENTS CONSIDERED TO BE RELEVANT**

| Category* | Citation of document, with indication, where appropriate, of the relevant passages | Relevant to claim No. |
|---|---|---|
| A | WO 2009081259 A1 (PFIZER INC.) 02 July 2009 (2009-07-02)<br>entire document | 1-10 |
| A | CN 102250022 A (ZHEJIANG UNIVERSITY) 23 November 2011 (2011-11-23)<br>entire document | 1-10 |
| A | WO 2010077992 A1 (AMGEN INC.) 08 July 2010 (2010-07-08)<br>entire document | 1-10 |
| A | WO 2019222538 A1 (THE UNIVERSITY OF NORTH CAROLINA AT CHAPEL HILL) 21 November 2019 (2019-11-21)<br>entire document | 1-10 |
| A | WO 2019089626 A1 (THE UNITED STATES OF AMERICA, AS REPRESENTED BY THE SECRETARY, DEPARTMENT OF HEALTH AND HUMAN SERVICES et al.) 09 May 2019 (2019-05-09)<br>entire document | 1-10 |

Form PCT/ISA/210 (second sheet) (July 2022)

**INTERNATIONAL SEARCH REPORT**
**Information on patent family members**

International application No.
**PCT/CN2023/093095**

| Patent document cited in search report | Publication date (day/month/year) | Patent family member(s) | Publication date (day/month/year) |
|---|---|---|---|
| WO 2018014880 A1 | 25 January 2018 | CA 3031458 A1 | 25 January 2018 |
| | | AU 2017300463 A1 | 14 March 2019 |
| | | KR 20190032418 A | 27 March 2019 |
| | | IL 264403 A | 31 March 2019 |
| | | EP 3489225 A1 | 29 May 2019 |
| | | CN 109890799 A | 14 June 2019 |
| | | JP 2019523256 A | 22 August 2019 |
| | | EP 3489225 A4 | 18 December 2019 |
| | | US 2021284615 A1 | 16 September 2021 |
| CN 102300860 A | 28 December 2011 | AU 2009313768 A1 | 20 May 2010 |
| | | CA 2742993 A1 | 20 May 2010 |
| | | WO 2010057121 A1 | 20 May 2010 |
| | | US 2010137278 A1 | 03 June 2010 |
| | | TW 201030006 A | 16 August 2010 |
| | | MX 2011005100 A | 31 May 2011 |
| | | SG 171173 A1 | 29 June 2011 |
| | | US 2011160182 A1 | 30 June 2011 |
| | | US 2011160202 A1 | 30 June 2011 |
| | | KR 20110083746 A | 20 July 2011 |
| | | EP 2364308 A1 | 14 September 2011 |
| | | US 8053438 B2 | 08 November 2011 |
| | | VN 27991 A | 25 November 2011 |
| | | ID 201201086 A | 01 March 2012 |
| | | ZA 201104380 A | 28 March 2012 |
| | | JP 2012508776 A | 12 April 2012 |
| | | US 8247418 B2 | 21 August 2012 |
| | | HK 1161251 A0 | 24 August 2012 |
| | | EP 2364308 B1 | 24 October 2012 |
| | | US 2012277209 A1 | 01 November 2012 |
| | | US 8329700 B2 | 11 December 2012 |
| | | AU 2009313768 B2 | 10 January 2013 |
| | | IL 212687 A | 03 February 2013 |
| | | ES 2397218 T3 | 05 March 2013 |
| | | NZ 592508 A | 26 April 2013 |
| | | TW I396689 B | 21 May 2013 |
| | | HK 1161251 A1 | 31 May 2013 |
| | | MX 312636 B | 26 August 2013 |
| | | IN 201104425 P1 | 27 September 2013 |
| | | EA 019206 B1 | 30 January 2014 |
| | | CN 102300860 B | 27 August 2014 |
| | | CA 2742993 C | 20 January 2015 |
| | | JP 5746972 B2 | 08 July 2015 |
| | | BR PI0922095 A2 | 24 September 2019 |
| CN 103814016 A | 21 May 2014 | JP 2016185953 A | 27 October 2016 |
| | | JP 6298096 B2 | 20 March 2018 |
| | | MX 354412 B | 05 March 2018 |
| | | IL 229863 A | 29 June 2017 |
| | | KR 20150144817 A | 28 December 2015 |
| | | EA 031153 B1 | 30 November 2018 |
| | | WO 2012170976 A2 | 13 December 2012 |

Form PCT/ISA/210 (patent family annex) (July 2022)

**INTERNATIONAL SEARCH REPORT**
**Information on patent family members**

International application No.
**PCT/CN2023/093095**

| Patent document cited in search report | Publication date (day/month/year) | Patent family member(s) | | | Publication date (day/month/year) |
|---|---|---|---|---|---|
| | | WO | 2012170976 | A3 | 11 April 2013 |
| | | US | 2017136048 | A1 | 18 May 2017 |
| | | US | 10016448 | B2 | 10 July 2018 |
| | | KR | 20140007954 | A | 20 January 2014 |
| | | US | 2018243324 | A1 | 30 August 2018 |
| | | US | 10413562 | B2 | 17 September 2019 |
| | | ES | 2923760 | T3 | 30 September 2022 |
| | | AU | 2012267491 | A1 | 31 October 2013 |
| | | AU | 2012267491 | B2 | 06 July 2017 |
| | | EP | 2718270 | A2 | 16 April 2014 |
| | | EP | 2718270 | B1 | 27 April 2022 |
| | | AU | 2017235978 | A1 | 19 October 2017 |
| | | AU | 2017235978 | B2 | 04 April 2019 |
| | | SG | 194219 | A1 | 29 November 2013 |
| | | KR | 20170021367 | A | 27 February 2017 |
| | | KR | 101880966 | B1 | 23 July 2018 |
| | | EP | 4056562 | A1 | 14 September 2022 |
| | | JP | 2020073593 | A | 14 May 2020 |
| | | JP | 6849834 | B2 | 31 March 2021 |
| | | EA | 027584 | B1 | 31 August 2017 |
| | | JP | 6853204 | B2 | 31 March 2021 |
| | | JP | 2014517016 | A | 17 July 2014 |
| | | KR | 20190011343 | A | 01 February 2019 |
| | | US | 2014162983 | A1 | 12 June 2014 |
| | | US | 9073947 | B2 | 07 July 2015 |
| | | CA | 2833771 | A1 | 13 December 2012 |
| | | CA | 2833771 | C | 03 August 2021 |
| | | US | 2015259363 | A1 | 17 September 2015 |
| | | US | 9580449 | B2 | 28 February 2017 |
| | | KR | 20180084153 | A | 24 July 2018 |
| | | MX | 2013012983 | A1 | 31 December 2013 |
| | | HK | 1197234 | A0 | 09 January 2015 |
| | | IN | 201309111 | P1 | 24 June 2016 |
| | | SG | 194219 | B | 27 June 2016 |
| | | SG | 10201604682 | A1 | 28 July 2016 |
| | | CN | 106831732 | A | 13 June 2017 |
| | | ZA | 201306693 | A | 26 July 2017 |
| | | ZA | 201307632 | A | 26 July 2017 |
| | | HK | 1197234 | A1 | 15 February 2018 |
| | | SG | 10201604682 | B | 23 March 2018 |
| | | HK | 1237779 | A0 | 20 April 2018 |
| | | CN | 106831732 | B | 24 December 2019 |
| | | HK | 1237779 | A1 | 30 October 2020 |
| | | BR | 112013030442 | B1 | 09 November 2021 |
| | | CN | 103814016 | B | 08 March 2017 |
| | | SG | 10201801668 | A1 | 28 March 2018 |
| | | KR | 20180084153 | A | 24 July 2018 |
| | | JP | 2018115165 | A | 26 July 2018 |
| | | BR | 112013030442 | A2 | 27 September 2016 |
| | | SG | 10201801668 | B | 01 June 2021 |

Form PCT/ISA/210 (patent family annex) (July 2022)

**INTERNATIONAL SEARCH REPORT**
**Information on patent family members**

International application No.
**PCT/CN2023/093095**

| Patent document cited in search report | Publication date (day/month/year) | Patent family member(s) | Publication date (day/month/year) |
|---|---|---|---|
| | | IN 373365 B | 06 August 2021 |
| WO 2009081259 A1 | 02 July 2009 | None | |
| CN 102250022 A | 23 November 2011 | CN 102250022 B | 16 October 2013 |
| WO 2010077992 A1 | 08 July 2010 | US 2010160280 A1 | 24 June 2010 |
| | | CA 2746307 A1 | 08 July 2010 |
| | | TW 201035051 A | 01 October 2010 |
| | | AU 2009333214 A1 | 30 June 2011 |
| | | EP 2376455 A1 | 19 October 2011 |
| | | MX 2011006575 A | 31 October 2011 |
| | | JP 2012512255 A | 31 May 2012 |
| | | EP 2376455 B1 | 14 November 2012 |
| | | ES 2397934 T3 | 12 March 2013 |
| | | AU 2009333214 B2 | 26 September 2013 |
| | | CA 2746307 C | 19 November 2013 |
| | | US 8637500 B2 | 28 January 2014 |
| | | MX 318571 B | 19 March 2014 |
| WO 2019222538 A1 | 21 November 2019 | None | |
| WO 2019089626 A1 | 09 May 2019 | US 2021179630 A1 | 17 June 2021 |
| | | US 11505559 B2 | 22 November 2022 |
| | | US 2023079399 A1 | 16 March 2023 |
| | | EP 3704102 A1 | 09 September 2020 |
| | | EP 4155300 A1 | 29 March 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009081259 A1


### Non-patent literature cited in the description


- **SAMBROOK et al.** Molecular Cloning: Laboratory Manual. Cold Spring Harbor Laboratory Press, 1989
- *CHEMICAL ABSTRACTS*, 125572-95-4
- *CHEMICAL ABSTRACTS*, 7447-40-7